# EUROPEAN PATENT APPLICATION

(11) **EP 3 864 991 A1**
(43) Date of publication of application: **18.08.2021**
(21) Application number: 19870500.6
(22) Date of filing: 08.10.2019
(51) Int. Cl.: A43B 17/00, A43B 13/38, A43D 1/02, A61F 5/02, A61H 3/00

(54) **ORTHOTIC DEVICE, ORTHOTIC DEVICE PRODUCTION METHOD, INFORMATION PROCESSING DEVICE, INFORMATION PROCESSING METHOD, SYSTEM, AND PROGRAM**

(30) Priority: 10.10.2018 JP 2018191521; 10.10.2018 JP 2018191522; 10.10.2018 JP 2018191523
(71) Applicant: JSR Corporation, Tokyo 105-8640 (JP)
(72) Inventor: KOMATSU, Satoshi, Tokyo 105-8640 (JP); KOBE, Takashi, Tokyo 105-8640 (JP); KOSHIKARI, Yoshiki, Tokyo 105-8640 (JP); HAYASHIDA, Taizo, Tokyo 105-8640 (JP); KAWASE, Reiji, Tokyo 105-8640 (JP)
(74) Representative: TBK
(86) International application number: PCT/JP2019/039696
(87) International publication number: WO 2020/075722

(57) **Abstract**

An orthosis according to an embodiment has a first region and a second region. The first region is constituted with a first unit cell structure that is a unit cell structure in which a space of a polygonal prism shape is defined as one unit, and includes a plurality of structural columns connecting two points among a plurality of vertices forming the polygonal prism shape. The second region is constituted with a second unit cell structure different from the first unit cell structure. The first unit cell structure and the second unit cell structure include at least one structural column connecting a certain vertex among the plurality of vertices and another vertex different from a vertex on a side including the certain vertex.

## Description

### Field

Embodiments of the present invention relate to an orthosis, an orthosis manufacturing method, an information processing apparatus, an information processing method, a system, and a program.

### Background

Conventionally, in the medical field, when a physical function is impaired or lost due to an illness or injury, an orthosis worn on the body is used to support the function or protect the affected part. Not merely for medical purposes, an orthosis is used also for assisting and protecting physical functions in sports and daily life.

For example, a medical insole is known as an example of an orthosis. Medical insoles are manufactured by combining materials having various physical properties according to the position and symptoms of the affected part so as to be customized for individual patients.

### Citation List

### Patent Literature

Patent Literature 1: JP 2017-012751 A
Patent Literature 2: WO 2016/137818 A
Patent Literature 3 WO 2014/100462 A
Patent Literature 4: JP 2016-155042 A
Patent Literature 5: JP 2014-111196 A
Patent Literature 6: JP 5222191 B2

### Summary

### Technical Problem

However, in the conventional techniques, an orthosis having appropriate physical properties is not always provided in accordance with regions. For example, medical insoles are required to have different physical properties depending on the region, such as a region (part) corresponding to the toe and the region corresponding to the heel. However, insoles having appropriate physical properties are not always provided for individual regions.

An object of the present invention is to provide an orthosis having appropriate physical properties according to the region, an orthosis manufacturing method, an information processing apparatus capable of designing an orthosis, an information processing method, a system, and a program.

### Solution to Problem

In order to solve the problem described above and to achieve the goal, in the present invention, the present invention discloses an orthosis comprising:
a first region constituted with a first unit cell structure being a unit cell structure having a space of a polygonal prism shape as one unit and having a plurality of structural columns connecting two points out of a plurality of vertices forming the polygonal prism shape; and
a second region constituted with a second unit cell structure different from the first unit cell structure,
wherein the first unit cell structure and the second unit cell structure have at least one structural column connecting a certain vertex among the plurality of vertices to a vertex different from a vertex on a side including the certain vertex.

Further, the present invention discloses an information processing apparatus comprising:
a reception unit that receives input related to physical property information represented by a relationship between a change in load and a change in a displacement amount;
a generation unit that
determines shape parameters that define physical properties of an orthosis based on the physical property information received by the reception unit, and
generates model data for designing the orthosis including the determined shape parameters; and
an output control unit that outputs the model data generated by the generation unit.

Further, the present invention discloses an information processing apparatus comprising:
an acquisition unit that acquires first site information including outer shape data of a site of a first subject based on image data obtained by imaging the site of the first subject;
a generation unit that generates first model data for designing a first orthosis to be worn on the site of the first subject by inputting the first site information into a trained model that has been trained, for sites of a plurality of second subjects, by using second site information including outer shape data of the site of each of the second subjects, second model data for designing a second orthosis to be worn on the site of each of the second subjects, and evaluation information related to evaluation of the second orthosis; and
an output control unit that outputs the first model data.

Here, a subject shows a human (a test subject) or an animal. Further, a site of the subject shows a part of body of the subject such as a foot, a hand, elbow, knee, shoulder, or head.

Further, the present invention discloses a system equipped with at least an information processing apparatus and a modeling device,
the system comprising:
an acquisition unit that acquires first site information including outer shape data of a site of a first subject based on image data obtained by imaging the site of the first subject;
a generation unit that generates first model data for designing a first orthosis to be worn on the site of the first subject by inputting the first site information into a trained model that has been trained, for sites of a plurality of second subjects, by using second site information including outer shape data of the site of each of the second subjects, second model data for designing a second orthosis to be worn on the site of each of the second subjects, and evaluation information related to evaluation of the second orthosis; and
a modeling unit that models the first orthosis based on the first model data.

Further, the present invention discloses an orthosis manufacturing method comprising:
an acquisition step of acquiring first site information including outer shape data of a site of a first subject based on image data obtained by imaging the site of the first subject;
a generation step of generating first model data for designing a first orthosis to be worn on the site of the first subject by inputting the first site information into a trained model that has been trained, for sites of a plurality of second subjects, by using second site information including outer shape data of the site of each of the second subjects, second model data for designing a second orthosis to be worn on the site of each of the second subjects, and evaluation information related to evaluation of the second orthosis; and
a modeling step of modeling the first orthosis based on the first model data.

Further, the present invention discloses an information processing method comprising:
an acquisition step of acquiring first site information including outer shape data of a site of a first subject based on image data obtained by imaging the site of the first subject;
a generation step of generating first model data for designing a first orthosis to be worn on the site of the first subject by inputting the first site information into a trained model that has been trained, for sites of a plurality of second subjects, by using second site information including outer shape data of the site of each of the second subjects, second model data for designing a second orthosis to be worn on the site of each of the second subjects, and evaluation information related to evaluation of the second orthosis; and
an output control step of outputting the first model data.

Further, the present invention discloses a program for causing a computer to execute processes comprising:
acquiring first site information including outer shape data of a site of a first subject based on image data obtained by imaging the site of the first subject;
generating first model data for designing a first orthosis to be worn on the site of the first subject by inputting the first site information into a trained model that has been trained, for sites of a plurality of second subjects, by using second site information including outer shape data of the site of each of the second subjects, second model data for designing a second orthosis to be worn on the site of each of the second subjects, and evaluation information related to evaluation of the second orthosis; and
outputting the first model data.

Further, the present invention discloses an information processing apparatus comprising:
a learning unit that generates a trained model, for sites of a plurality of subjects, by performing machine learning using site information including outer shape data of the site of each of the subjects, model data for designing an orthosis to be worn on the site of each of the subjects, and evaluation information regarding evaluation of the orthosis; and
an output control unit that outputs the trained model.

Further, the present invention discloses an information processing method comprising:
a learning step of generating a trained model, for sites of a plurality of subjects, by performing machine learning using site information including outer shape data of the site of each of the subjects, model data for designing an orthosis to be worn on the site of each of the subjects, and evaluation information regarding evaluation of the orthosis; and
an output control step of outputting the trained model.

Further, the present invention discloses a program for causing a computer to execute processes comprising:
generating a trained model, for sites of a plurality of subjects, by performing machine learning using site information including outer shape data of the site of each of the subjects, model data for designing an orthosis to be worn on the site of each of the subjects, and evaluation information regarding evaluation of the orthosis; and
outputting the trained model.

### Advantageous Effects of Invention

According to the present invention, it is possible to provide an orthosis having appropriate physical properties in accordance with the region, an orthosis manufacturing method, an information processing apparatus capable of designing an orthosis, an information processing method, a system, and a program.

### Brief Description of Drawings

FIG. 1 is a diagram illustrating an example of a structure of an insole of a first embodiment.
FIG. 2 is a diagram illustrating a unit cell structure of the first embodiment.
FIG. 3 is a diagram illustrating the unit cell structure of the first embodiment.
FIG. 4 is a diagram illustrating shape parameters defined in each of regions of the insole of the first embodiment.
FIG. 5 is a diagram illustrating a relationship between the shape parameters and the physical properties of the first embodiment.
FIG. 6 is a diagram illustrating a relationship between the shape parameters and the physical properties of the first embodiment.
FIG. 7 is a diagram illustrating a relationship between the shape parameters and the physical properties of the first embodiment.
FIG. 8 is a diagram illustrating a relationship between the shape parameters and the physical properties of the first embodiment.
FIG. 9 is a diagram illustrating a relationship between the shape parameters and the physical properties of the first embodiment.
FIG. 10 is a flowchart illustrating an example of a manufacturing method of the first embodiment.
FIG. 11 is a diagram illustrating an example of a schematic configuration of a system of a second embodiment.
FIG. 12 is a diagram illustrating an example of a hardware configuration of an information processing apparatus of the second embodiment.
FIG. 13 is a diagram illustrating an example of a function of the information processing apparatus of the second embodiment.
FIG. 14 is a diagram illustrating an example of outer shape data of an insole of the second embodiment.
FIG. 15 is a diagram illustrating an example of an input screen for texture information of the second embodiment.
FIG. 16 is a diagram illustrating an example of related information referred to in a generation unit of the second embodiment.
FIG. 17 is a diagram illustrating the shape of a unit cell structure and the length of each side of the second embodiment.
FIG. 18 is a diagram illustrating a unit cell model of the second embodiment.
FIG. 19 is a diagram illustrating a relationship between the type of unit cell model and the load-displacement characteristic of the second embodiment.
FIG. 20 is a diagram illustrating a relationship between L/S and the load-displacement characteristic of the second embodiment.
FIG. 21 is a diagram illustrating a relationship between the volume of an intersection and the load-displacement characteristic of the second embodiment.
FIG. 22 is a diagram illustrating a relationship between the volume of the intersection and the load-displacement characteristic of the second embodiment.
FIG. 23 is a diagram illustrating a difference in shape recovery speed depending on the volume of the intersection of the second embodiment.
FIG. 24 is a diagram illustrating an example of a display screen provided by an output control unit of the second embodiment.
FIG. 25 is a flowchart illustrating an operation example of the information processing apparatus of the second embodiment.
FIG. 26 is a diagram illustrating an example of an input screen of a first modification of the second embodiment.
FIG. 27 is a diagram illustrating an example of a display screen of a second modification of the second embodiment.
FIG. 28 is a flowchart illustrating an operation example of an information processing apparatus according to a third embodiment.
FIG. 29 is a diagram illustrating processing of a generation unit of the third embodiment.
FIG. 30 is a diagram illustrating the processing of the generation unit of the third embodiment.
FIG. 31 is a diagram illustrating the processing of the generation unit of the third embodiment.
FIG. 32 is a flowchart illustrating an operation example of an information processing apparatus according to a fourth embodiment.
FIG. 33 is a diagram illustrating processing of a generation unit of the fourth embodiment.
FIG. 34 is a diagram illustrating an example of a schematic configuration of a system of a fifth embodiment.
FIG. 35 is a diagram illustrating an example of a function of an information processing apparatus of the fifth embodiment.
FIG. 36 is a diagram illustrating a correction process of model data according to the fifth embodiment.
FIG. 37 is a flowchart illustrating an example of a manufacturing method of an embodiment.
FIG. 38 is a diagram illustrating an example of a schematic configuration of a system of a sixth embodiment.
FIG. 39 is a diagram illustrating an example of a hardware configuration of an information processing apparatus of the sixth embodiment.
FIG. 40 is a diagram illustrating an example of a function of the information processing apparatus of the sixth embodiment.
FIG. 41 is a diagram illustrating processing during learning and application performed by the information processing apparatus of the sixth embodiment.
FIG. 42 is a diagram illustrating an example of foot information of the sixth embodiment.
FIG. 43 is a diagram illustrating an example of outer shape data of an insole of the sixth embodiment.
FIG. 44 is a diagram illustrating an example of shape parameters of the insole of the sixth embodiment.
FIG. 45 is a diagram illustrating the shape of a unit cell structure and the length of each side of the sixth embodiment.
FIG. 46 is a diagram illustrating a unit cell model of the sixth embodiment.
FIG. 47 is a diagram illustrating a relationship between the type of unit cell model and the load-displacement characteristic of the sixth embodiment.
FIG. 48 is a diagram illustrating a relationship between L/S and the load-displacement characteristic of the sixth embodiment.
FIG. 49 is a diagram illustrating a relationship between the volume of an intersection and the load-displacement characteristic of the sixth embodiment.
FIG. 50 is a diagram illustrating a relationship between the volume of the intersection and the load-displacement characteristic of the sixth embodiment.
FIG. 51 is a diagram illustrating a difference in shape recovery speed depending on the volume of the intersection of the sixth embodiment.
FIG. 52 is a diagram illustrating a correction process of model data according to the sixth embodiment.
FIG. 53 is a flowchart illustrating an operation example of the information processing apparatus of the sixth embodiment.
FIG. 54 is a flowchart illustrating an operation example of the information processing apparatus according to the sixth embodiment.
FIG. 55 is a flowchart illustrating an example of a manufacturing method of the sixth embodiment.
FIG. 56 is a diagram illustrating a lattice cube designed by OpenSCAD.
FIG. 57 is a diagram illustrating a load-displacement curve of a lattice cube with a different unit cell.
FIG. 58 is a diagram illustrating a behavior during cube compression.
FIG. 59 is a diagram illustrating a filling pattern by a slicer when modeling a cube by an FDM method.
FIG. 60 is a diagram illustrating a load-displacement curve of a cube in which a filling rate is varied with the filling pattern as a rectilinear shape.
FIG. 61 is a diagram illustrating a load-displacement curve of a cube in which the filling rate is fixed at 20% and the filling pattern is changed.
FIG. 62 is a diagram illustrating a compression behavior of an FDM modeling sample.
FIG. 63 is a diagram illustrating a load-displacement curve of a lattice cube when a unit cell is fixed and a column thickness (L/S) and a cell dimensional ratio are changed.
FIG. 64 is a diagram illustrating a load-displacement curve of an existing medical insole material.

### Description of Embodiments

Hereinafter, embodiments of an orthosis, an orthosis manufacturing method, an information processing apparatus, an information processing method, a system, and a program according to the present invention will be described in detail with reference to the accompanying drawings.

### (First embodiment)

In the following embodiment, a medical insole will be described as an example of the orthosis. However, the embodiment is not limited to the insole but is widely applicable to orthoses to be applied to individual sites of a human body, such as a hand, elbow, knee, shoulder, or head. Furthermore, the embodiment is not limited to medical orthoses, and is widely applicable to an orthosis used for assisting or protecting physical functions in sports and daily life, for example. The insole is also referred to as an internal fitting, an inner sole, or the like.

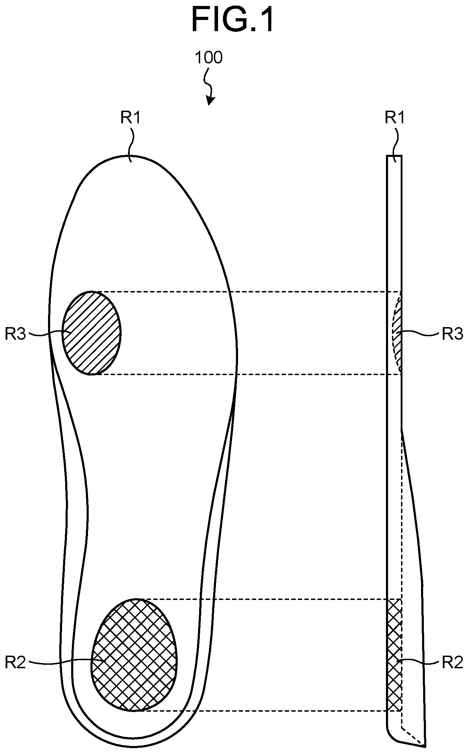

FIG. 1 is a diagram illustrating an example of a structure of an insole 100 of a first embodiment. The left figure of FIG. 1 illustrates a plan view of the insole 100 for the right foot, as viewed from the contact surface side in direct or indirect contact with each of sites of a human body. The right figure in FIG. 1 illustrates a side view of the left figure. The toe side of the insole 100 is illustrated on the upper side of FIG. 1, while the heel side of the insole 100 is illustrated on the lower side of FIG. 1.

As illustrated in FIG. 1, the insole 100 of the first embodiment has a region R1, a region R2, and a region R3. The region R1 is a region that constitutes the overall outer shape of the insole 100. The region R2 is a region where the site near the calcaneus (heel bone) touches. The region R3 is a region coming in contact with the site near the metatarsal joint of the big toe.

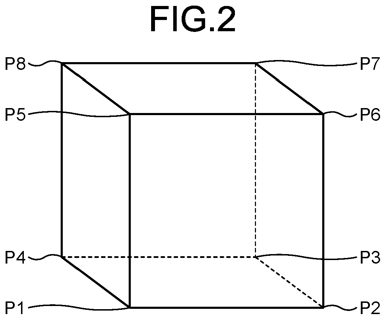

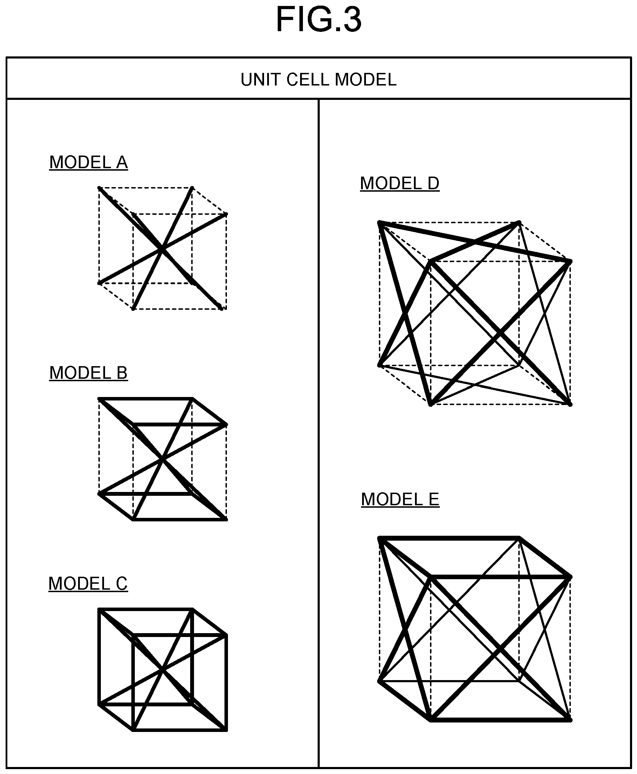

The region R1, region R2, and region R3 according to the first embodiment are constituted with unit cell structures different from each other. The structure of the insole 100 illustrated in FIG. 1 is just an example. For example, the number, position, size, shape, and thickness of each of regions constituting the insole 100 can be flexibly changed depending on the purpose and symptoms of the wearer. That is, the insole 100 has at least two regions constituted with unit cell structures different from each other. Hereinafter, the unit cell structure will be described with reference to FIGS. 2 and 3. FIGS. 2 and 3 are diagrams illustrating the unit cell structure of the first embodiment.

FIG. 2 illustrates an exemplary spatial shape defined as one unit of the unit cell structure. As illustrated in FIG. 2, the unit cell structure has a cubic space constituted with six square faces, as one unit. The unit cell structure has eight points P1 to P8 corresponding to vertices.

Here, the notation method of lines and faces in the unit cell structure will be described. In the first embodiment, when a line or a face is described, the vertices constituting the line or the face are described in parentheses. For example, the notation "line (P1, P2)" represents a line connecting the point P1 and the point P2. The notation "line (P1, P7)" represents a line (diagonal) connecting the point P1 and the point P7. Furthermore, the notation "face (P1, P2, P3, P4)" represents a face (bottom face) having points P1, P2, P3, and P4 as vertices. The structural columns are described similarly to the line notation method.

The unit cell structure according to the first embodiment includes a plurality of structural columns connecting two points out of the plurality of points P1 to P8. That is, the spatial shape of FIG. 2 corresponds to the shape of the unit cell structure. The number and direction (arrangement direction) of the structural columns are defined in advance by a unit cell model, for example.

FIG. 3 illustrates a unit cell model representing a basic skeleton shape of a unit cell structure. In FIG. 3, the dashed lines indicate a cubic space (space in FIG. 2) corresponding to one unit. The solid lines indicate the existence of structural columns. Preferably, the unit cell model illustrated in FIG. 3 is arranged in each of regions of the insole 100 so that its arrangement direction matches the direction of load applied to the orthosis in the vertical downward direction of FIG. 3. The direction of load applied to the orthosis is a direction in which the load received from each of sites of the human body is applied.

As illustrated in FIG. 3, for example, a unit cell model includes model A, model B, model C, model D, and model E. Here, model A, model B, and model C each have an intersection of structural columns at a center of a cubic space. Furthermore, model D and model E each have an intersection of structural columns at the center of at least one of a plurality of faces constituting the cubic shape.
Model A is a unit cell model with four structural columns arranged along the diagonals of the cubic shape. Specifically, model A has a structural column (P1, P7), a structural column (P2, P8), a structural column (P3, P5), and a structural column (P4, P6).
Model B is a unit cell model including, in addition to the four structural columns similar to model A, eight structural columns arranged along a plurality of sides surrounding the bottom face and the top face constituting the cubic shape. Specifically, model B includes four structural columns included in model A. In addition, model B includes four structural columns that surround the bottom face (P1, P2, P3, P4), namely, a structural column (P1, P2), a structural column (P2, P3), a structural column (P3, P4), and a structural column (P4, P1). In addition, model B has four structural columns that surround the top face (P5, P6, P7, P8), namely, a structural column (P5, P6), a structural column (P6, P7), a structural column (P7, P8), and a structural column (P8, P5) .
Model C is a unit cell model further including four structural columns arranged in the load direction in addition to 12 structural columns similar to model B. Specifically, model C has 12 structural columns included in model B. Model C further includes four structural columns, namely, a structural column (P1, P5), a structural column (P2, P6), a structural column (P3, P7), and a structural column (P4, P8) arranged in the direction of the load applied to the insole.
   That is, the difference between model B and model C is whether the model includes the four structural columns, namely, a structural column (P1, P5), a structural column (P2, P6), a structural column (P3, P7), and a structural column (P4, P8) arranged in the direction of the load.
Model D is a unit cell model with 12 structural columns arranged along the diagonals of each of the six faces. Specifically, model D has two structural columns, namely, a structural column (P1, P3) and a structural column (P2, P4) along the diagonals of the bottom face (P1, P2, P3, P4). Specifically, model D has two structural columns, namely, a structural column (P5, P7) and a structural column (P6, P8) along the diagonals of the top face (P5, P6, P7, P8). Specifically, model D has two structural columns, namely, a structural column (P1, P6) and a structural column (P2, P5) along the diagonals of the side face (P1, P2, P5, P6). Moreover, model D has two structural columns, namely, a structural column (P2, P7) and a structural column (P3, P6) along the diagonals of the side face (P2, P3, P6, P7). In addition, model D has two structural columns, namely, a structural column (P3, P8) and a structural column (P4, P7) along the diagonals of the side face (P3, P4, P7, P8). In addition, model D has two structural columns, namely, a structural column (P1, P8) and a structural column (P4, P5) along the diagonals of the side face (P1, P4, P5, P8) .

As compared with model D, model E is a unit cell model further including eight structural columns arranged along a plurality of sides surrounding the bottom face and the top face, without including the structural columns along the diagonals of the bottom face or the top face. Specifically, model E has two structural columns, namely, a structural column (P1, P6) and a structural column (P2, P5) along the diagonals of the side face (P1, P2, P5, P6). In addition, model E has two structural columns, namely, a structural column (P2, P7) and a structural column (P3, P6) along the diagonals of the side face (P2, P3, P6, P7). In addition, model E has two structural columns, namely, a structural column (P3, P8) and a structural column (P4, P7) along the diagonals of the side face (P3, P4, P7, P8). In addition, model E has two structural columns, namely, a structural column (P1, P8) and a structural column (P4, P5) along the diagonals of the side face (P1, P4, P5, P8). In addition, model E has four structural columns surrounding the bottom face (P1, P2, P3, P4), namely, a structural column (P1, P2), a structural column (P2, P3), a structural column (P3, P4), and a structural column (P4, P1). In addition, model E has four structural columns surrounding the top face (P5, P6, P7, P8), namely, a structural column (P5, P6), a structural column (P6, P7), a structural column (P7, P8), and a structural column (P8, P5).

That is, the difference between model D and model E is the presence or absence of structural columns arranged on the bottom face and top face. Specifically, model D has four structural columns, namely, a structural column (P1, P3), a structural column (P2, P4), a structural column (P5, P7), and a structural column (P6, P8), along the diagonals of the bottom face and the top face. In addition, model E has eight structural columns surrounding the bottom face and the top face, namely, a structural column (P1, P2), a structural column (P2, P3), a structural column (P3, P4), a structural column (P4, P1), a structural column (P5, P6), a structural column (P6, P7), a structural column (P7, P8), and a structural column (P8, P5).

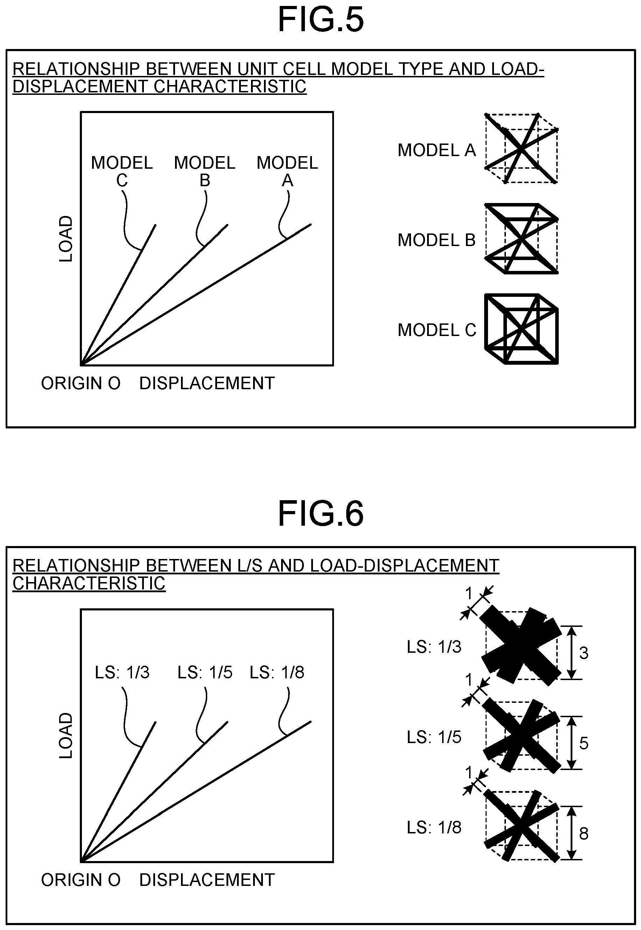

In FIG. 6, the structural columns of model D and model E are illustrated by solid lines of two types of thickness. However, this is intended to clarify the illustration, rather than indicating the actual thickness of the structural columns. In other words, the thicker solid line indicates the structural column included in the "faces visible on the front side of the box" when the cubic space is regarded as a box. Furthermore, the thinner solid line indicates a structural column included only in the "faces visible on the back side of the box" when the cubic space is regarded as a box. The "faces visible on the front side of the box" correspond to the top face (P5, P6, P7, P8), the side face (P1, P2, P5, P6), and the side face (P1, P4, P5, P8). The "faces visible on the back side of the box" correspond to the bottom face (P1, P2, P3, P4), the side face (P2, P3, P6, P7), and the side face (P3, P4, P7, P8) .

In this manner, the region R1, region R2, and region R3 are constituted with unit cell structures different from each other. That is, each of regions of the insole 100 has a structure having a plurality of unit cell structures repeatedly and continuously arranged. Specifically, each of regions of the insole 100 has at least one layer including a plurality of unit cell structures arranged on an identical plane, with these layers being stacked to form the region. The cross-sectional shape of the structural column may be any shape such as a polygon including a quadrangle, a pentagon or a hexagon, a circle or an ellipse, or the like.

The contents illustrated in FIGS. 2 and 3 are merely an example, and the present invention is not limited to the contents in the drawings. For example, the cubic space illustrated in FIG. 2 is merely an example, and the spatial shape defined as one unit of the unit cell structure may be a polygonal prism shape. In this case, it is preferable that the unit cell structure has a space having any one of a triangular prism shape, a quadrangular prism shape, or a hexagonal prism shape, as one unit. As the triangular prism shape, a regular triangular prism shape is preferable. Furthermore, as the quadrangular prism shape, a rectangular parallelepiped shape is preferable in addition to the above cubic shape. Furthermore, as the hexagonal prism shape, a regular hexagonal prism shape is preferable. Note that it is preferable that the unit cell structure of each of the regions R1 to R3 constituting the orthosis has an identical spatial shape. Furthermore, the structural column is a column such as a polygonal prism shape or a cylindrical shape. Furthermore, the lateral direction of the structural column is a direction orthogonal to an axial direction of the structural column.

Furthermore, the model illustrated in FIG. 3 is merely an example, and the unit cell model may have structural columns at any positions. Still, it is preferable that the unit cell structure includes a plurality of structural columns so that any of the structural columns passes through all the vertices constituting the space of the polygonal prism shape.

Furthermore, the unit cell structure preferably has a structural column arranged in a direction (diagonal direction) intersecting the load direction. For example, model A, model B, and model C have structural columns along the diagonal of the cubic shape, and model D and model E have structural columns arranged along the diagonals of the faces constituting the cubic shape. That is, the unit cell structure includes at least one structural column connecting a certain vertex among the plurality of vertices and another vertex different from a vertex on a side including the certain vertex.

Furthermore, the unit cell structure is not to limited to a structure having an intersection of structural columns at the center of a cubic space, or a structure having an intersection of structural columns at the center of at least one of a plurality of faces constituting the cubic shape. Still, it would be preferable that the unit cell structure has an intersection where at least two structural columns intersect at different position than the plurality of vertices.

Furthermore, in the case of a structure having an intersection of structural columns in the center of a cubic space, it is preferable that the unit cell structure includes at least two structural columns arranged along the diagonals of the polygonal prism shape and intersecting each other at the intersection. Furthermore, in the case of a quadrangular prism shape, it is preferable that the unit cell structure has four structural columns arranged along the diagonals of the quadrangular prism shape and intersecting each other at the intersection.

Furthermore, in the case of a structure having an intersection of structural columns in the center of at least one face out of the plurality of faces constituting the cubic shape, it is preferable that the unit cell structure includes at least two structural columns arranged along the diagonals of the at least one face out of the bottom face, top face, or side face constituting the polygonal prism shape and intersecting each other at the intersection.

Furthermore, it is preferable that the unit cell structure has a plurality of structural columns arranged along a plurality of sides surrounding at least one face out of the bottom face and the top face constituting the polygonal prism shape. Furthermore, the unit cell structure is preferably arranged in the direction of the load applied to the insole (orthosis).

Back to the description of FIG. 1. In the insole 100, the unit cell structure of the region R1, the unit cell structure of the region R2, and the unit cell structure of the region R3 have mutual different shape parameters that define the physical properties. The shape parameters include at least one of the shape of the unit cell structure, the length of each side of the unit cell structure, the number of structural columns, the direction of the structural columns, the thickness of the structural columns, or the volume of the intersection, for example.

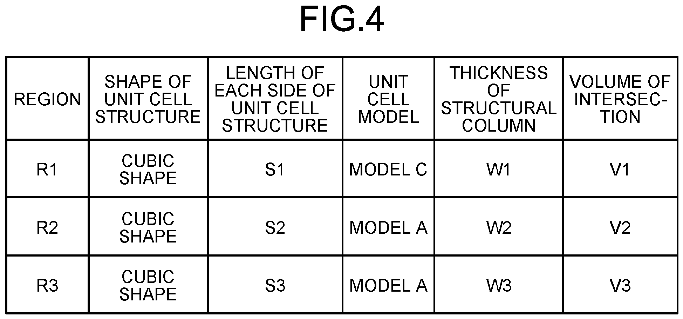

FIG. 4 is a diagram illustrating shape parameters defined in each of regions of the insole 100 of the first embodiment. Note that FIG. 4 illustrates a case where the number and direction of structural columns are defined by the type of unit cell model.

As illustrated in FIG. 4, for example, the parameters defined in the region R1 are the shape of the unit cell structure: "cubic shape", the length of each side of the unit cell structure: "S1", the unit cell model: "model C", the thickness of the structural columns: "W1", and the volume of the intersection: "V1". This indicates that the region R1 is constituted with the unit cell model of model C, the thickness of its structural columns is "W1", and the volume of its intersection is "V1".

Moreover, the parameters defined in the region R2 are the shape of the unit cell structure: "cubic shape", the length of each side of the unit cell structure: "S2", the unit cell model: "model A", the thickness of the structural columns: "W2", and the volume of the intersection: "V2". This indicates that the region R2 is constituted with the unit cell model of model A, the thickness of its structural columns is "W2", and the volume of its intersection is "V2".

Moreover, the parameters defined in the region R3 are the shape of the unit cell structure: "cubic shape", the length of each side of the unit cell structure: "S3", the unit cell model: "model A", the thickness of the structural columns: "W3", and the volume of the intersection: "V3". This indicates that the region R3 is constituted with the unit cell model of model A, the thickness of its structural columns is "W3", and the volume of its intersection is "V3".

In this manner, each of the regions R1 to R3 has a difference in at least one shape parameter, out of the shape of the unit cell structure, the length of each side of the unit cell structure, the number of structural columns, the direction of the structural columns, the thickness of the structural columns, or the volume of the intersection. This gives each of the regions R1 to R3 mutually different physical properties. Furthermore, it is preferable that the unit cell structure of the region R1, the unit cell structure of the region R2, and the unit cell structure of the region R3 define a space having the identical shape and the identical size (here, the length of each side), as one unit. Next, the relationship between shape parameters and the physical properties will be described with reference to FIGS. 5 to 9.

FIGS. 5, 6, 7, 8, and 9 are diagrams illustrating a relationship between the shape parameters and the physical property of the first embodiment. FIGS. 5 to 9 use the load-displacement characteristic as the physical property. The load-displacement characteristic expresses a relationship between a load [N] applied to an object and a displacement [mm] of the object due to the load, and is utilized as a physical index of the hardness or adhesion of the object. In the present embodiment, the load-displacement characteristic is measured by applying a load to an object having a unit cell structure. In FIGS. 5 to 8, the vertical axis of the graph corresponds to the load [N], and the horizontal axis of the graph corresponds to the displacement [mm]. That is, in FIGS. 5 to 8, the harder the object, the steeper the graph, and the softer the object, the gentler the graph. Normally, although the graph is often obtained as a curve, FIGS. 5 to 8 use a straight line for simplification.

FIG. 5 illustrates a relationship between the types of unit cell models and the load-displacement characteristic. In FIG. 5, the load-displacement characteristic of the object constituted with the unit cell models of the models A, B, and C illustrated in FIG. 3 will be described. In FIG. 5, the thickness of the structural columns of the models A, B, and C and the volume of the intersection are constant.

From the load-displacement characteristic illustrated in FIG. 5, it is observed that the larger the number of structural columns included in each of the models, the harder the object tends to be. Furthermore, based on the load-displacement characteristic illustrated in FIG. 5, it is observed that the closer the direction of the structural column is to the load direction, the harder the object tends to be. Therefore, constituting the individual regions with different types of unit cell models would make it possible to provide a plurality of regions having different physical properties.

FIG. 6 illustrates a relationship between the thickness (L) of the structural column/length (S) of one side of the unit space and the load-displacement characteristic. FIG. 6 uses model A of FIG. 3 to explain the load-displacement characteristic of objects having structural columns of different thicknesses. In FIG. 6, the volume of the intersection of each of the objects is constant.

Furthermore, FIG. 6 illustrates an example of the thickness of the structural column, in which the ratio of the thickness (L) of the structural column and the length (S) of one side of the unit space is different. In the example of FIG. 6, "L/S: 1/3" corresponds to the case where the structural column is the thickest, with "L/S: 1/5" and "L/S: 1/8" corresponding to the case where the structural column is thinner and thinnest, respectively. The thickness of the structural column can be, for example, the length of the longest straight line among the straight lines passing through the center of gravity in the "cross section in the lateral direction" of the structural column.

From the load-displacement characteristic illustrated in FIG. 6, it is observed that the larger the L/S (thicker the column), the harder the object tends to be. In addition, the smaller the L/S (thinner the column), the softer the object tends to be. Therefore, constituting a plurality of regions with structural columns with different thicknesses would make it possible to provide a plurality of regions having different physical properties.

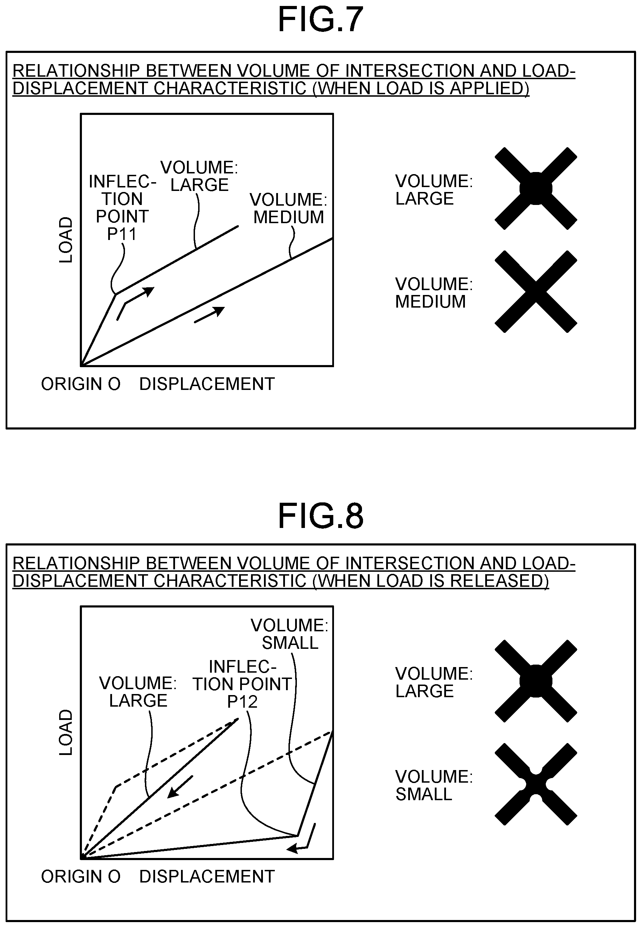

FIG. 7 illustrates a relationship between the volume of the intersection and the load-displacement characteristic when a load is applied. FIG. 7 uses model A of FIG. 3 to explain the load-displacement characteristic of objects having intersections with different volumes. In FIG. 7, the thickness (L) of the structural column of each of objects is constant. In the load-displacement characteristic when a load is applied as illustrated in FIG. 7, a point (position) on the graph indicating the displacement corresponding to the load moves in the direction of the arrow in the graph.

In the load-displacement characteristic illustrated in FIG. 7, an inflection point P11 is observed in the graph in a case where the volume of the intersection is large. In other words, an object with a large volume at the intersection behaves like a hard substance between an origin O and the inflection point P11 together with application with a load, and behaves like a soft substance after the inflection point P11. The change in physical properties (change in the slope of the graph) in this load-displacement characteristic is felt as adhesion when a person using the orthosis applies a load to the insole. Moreover, an object with a medium volume at the intersection behaves similarly to a soft object. Therefore, constituting a plurality of regions with intersections with different volumes would make it possible to provide a plurality of regions having different physical properties.

FIG. 8 illustrates a relationship between the volume of the intersection and the load-displacement characteristic when a load is released. FIG. 8 uses model A of FIG. 3 to explain the load-displacement characteristic of objects having intersections with different volumes. In FIG. 8, the thickness (L) of the structural column of each of objects is constant. In the load-displacement characteristic when a load is released as illustrated in FIG. 8, a point (position) on the graph indicating the displacement corresponding to the load moves in the direction of the arrow in the graph. The dashed graph illustrated in FIG. 8 corresponds to the graph when a load is applied.

In the load-displacement characteristic illustrated in FIG. 8, when the volume of the intersection is large, the shape recovery speed is high and a slope relatively close to linear can be obtained. That is, for an object having a large volume at the intersection, the displacement can make a quick recovery when the load is released. This physical property is felt as adhesion when the person using the orthosis releases the load from the insole. By contrast, when the volume of the intersection is small in the load-displacement characteristic illustrated in FIG. 8, the shape recovery speed is slow with observation of an inflection point P12. In other words, regarding an object with a small volume at the intersection, the amount of change in the displacement is small up to the inflection point P12 together with the release of the load, and the shape recovers to the original shape between the inflection point P12 and the origin O. Therefore, constituting a plurality of regions with intersections with different volumes would make it possible to provide a plurality of regions having different physical properties.

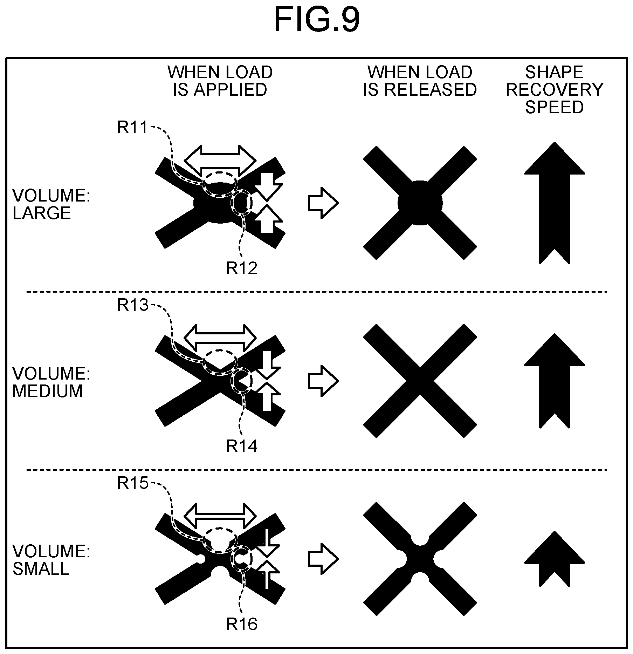

FIG. 9 describes the difference in shape recovery speed according to the volume of the intersection when a load is applied from the top face to the bottom face of the unit cell structure in model A of FIG. 3. FIG. 9 illustrates the change in the shape of the intersection at transition from application of the load to the release of the load, and the shape recovery speed in the change. The upper row of FIG. 9 illustrates a case where the intersection has a large volume, the middle row illustrates a case where the intersection has a medium volume, and the lower row illustrates a case where the intersection has a small volume. The length of the arrow indicating the shape recovery speed corresponds to the degree of the shape recovery speed.

With FIG. 9, the shape of the intersection will be described. When the volume is not adjusted in particular, the intersection has a shape as illustrated in the middle row of FIG. 9 (when the load is released). In the present embodiment, this shape will be described as a state in which the intersection has a medium volume.

When the intersection has a large volume, the intersection has a spherical shape or a polyhedral shape with the intersection as a substantially center, as illustrated in the upper row of FIG. 9 (when the load is released). That is, the intersection has a positively raised shape as compared with a state in which a plurality of structural columns is simply intersected. In this case, the intersection has a cross section larger than the cross section of the structural column in the lateral direction. Specifically, the area of the cross section of the intersection is preferably 1.1 times or more and 20 times or less as compared with the cross section of the structural column in the lateral direction, more preferably be 1.2 times or more and 10 times or less, and still more preferable when the area is 1.5 times or more and 5 times or less, in particular. Here, the area of the cross section of the intersection is based on the surface having the largest area among the surfaces passing through the center of gravity of the intersection.

In contrast, when the intersection has a small volume, the intersection has a shape in which the structural columns in the vicinity of the intersection are thinned, as illustrated in the lower row of FIG. 9 (when the load is released). In this case, the intersection has a cross section smaller than the cross section of the structural column in the lateral direction. Specifically, the area of the cross section of the intersection is preferably 0.05 times or more and 0.9 times or less as compared with the cross section of the structural column in the lateral direction, more preferably be 0.1 times or more and 0.8 times or less, and particularly preferable when the area is 0.2 times or more and 0.7 times or less. Here, the area of the cross section of the intersection is based on the surface having the largest area among the surfaces passing through the center of gravity of the intersection.

Here, as illustrated in FIG. 9, energy for shape recovery is considered to be accumulated at the intersection when a load is applied. For example, when a load is applied from the upper side to the lower side in FIG. 9, the two structural columns on both sides of a region R13 at the intersection are expanded, resulting in accumulation of the restoring force for the recovery of the columns in the region R13. In addition, the two structural columns on both sides of a region R14 at the intersection are narrowed, resulting in accumulation of a repulsive force for their recovery in the region R14.

When the intersection has a large volume, the restoring force and the repulsive force are accumulated as a large amount of energy. Therefore, the restoring force of a region R11 is greater than the restoring force of the region R13, and the repulsive force of a region R12 is greater than the repulsive force of the region R14. Consequently, it is considered that the greater the volume of the intersection, the higher the shape recovery speed.

When the intersection has a small volume, the restoring force and the repulsive force are accumulated as a small amount of energy. Therefore, the restoring force of a region R15 is less than the restoring force of the region R13, and the repulsive force of a region R16 is less than the repulsive force of the region R14. Consequently, it is considered that the smaller the volume of the intersection, the lower the shape recovery speed.

In this manner, having a plurality of regions by a unit cell structure having mutually different shape parameters makes it possible to provide the insole 100 with a plurality of regions having different physical properties.

The contents illustrated in FIGS. 4 to 9 are merely an example, and the present invention is not limited to the contents in the drawings. For example, in FIG. 4, since the exemplary spatial shape is a cubic shape, the description relates to the case where the length of each side is set by a uniform value (S1, S2, S3, or the like). However, the present invention is not limited to this, and the length of each side may be set individually. Furthermore, although FIG. 9 uses three levels for illustrating the volume of the intersection, the present invention is not limited to the three levels of volumes and can manufacture the intersection so as to have a desired volume.

As described above, the insole 100 of the first embodiment includes a first region constituted with a first unit cell structure being a unit cell structure having a space of a polygonal prism shape as one unit and having a plurality of structural columns connecting two points out of a plurality of vertices forming the polygonal prism shape. Furthermore, the insole 100 of the first embodiment includes a second region constituted with a second unit cell structure different from the first unit cell structure. This gives the insole 100 of the first embodiment appropriate physical properties corresponding to the regions. For example, the manufacturer of the insole 100 can provide an insole that is partially adjusted to certain physical properties in accordance with a state of the load being applied and the condition of the affected part.

### (Other embodiments related to first embodiment)

In addition to the above-described embodiments, various types of different embodiments may be implemented.

### (Orthosis manufacturing method)

In the above embodiment, the insole 100 according to the embodiment has been described. Alternatively, however, a person (manufacturer) who manufactures the insole 100 can manufacture the insole 100 by the following manufacturing method.

Here, being configured by a unit cell structure having different shape parameters, each of regions of the insole 100 has different physical properties. This makes it possible to give each of the regions of the insole 100 different physical properties even when manufactured from a uniform material. As a result, the manufacturer can integrally model (mold) the insole 100 having a plurality of regions by using various additive manufacturing technologies (three-dimensional modeling technology) such as a material extrusion method, a vat photopolymerization method, and a selective laser sintering method.

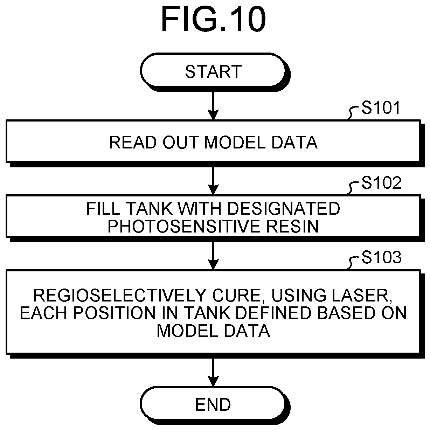

FIG. 10 is a flowchart illustrating an example of the manufacturing method of the first embodiment. The manufacturing method illustrated in FIG. 10 is executed by a modeling device capable of executing a three-dimensional modeling technology by a vat photopolymerization method. The modeling device includes a vat (tank) for filling the photosensitive resin, which is the material of the insole 100, and a laser for regioselectively curing the photosensitive resin in the vat by a photopolymerization reaction. Note that known modeling devices can be flexibly applied as the modeling device.

As illustrated in FIG. 10, a modeling device reads out model data (step S101). This model data is information that forms the basis for modeling with a modeling device. The model data is created in advance by a prosthetist, for example, and preliminarily stored in a storage device included in the modeling device. The storage device corresponds to, for example, a hard disc drive (HDD), a solid state drive (SSD), or the like.

Subsequently, the modeling device fills the tank with designated photosensitive resin (step S102). Here, the photosensitive resin may be designated in advance by the model data, or may be designated by an operator each time the modeling method is executed.

Subsequently, by using a laser, the modeling device regioselectively cures individual positions in the tank defined based on the model data (step S103). For example, the modeling device sequentially emits laser toward the positions in the tank corresponding to the positions where the structural columns exist in the model data. That is, the modeling device sequentially cures the positions in the tank corresponding to the unit cell structure of each of the region R1, the region R2, and the region R3. With this operation, the modeling device can model an insole containing a plurality of regions having mutually different physical properties using a single photosensitive resin.

The material of the insole 100 can be any material as long as it is a material applicable to modeling technology. For example, any photosensitive resin that can be modeled by additive manufacturing technology can be appropriately selected.

### (Load direction)

Although the above embodiment is a case where the load direction corresponds to the vertical downward direction in the drawing as illustrated in FIG. 9, for example, the embodiment is not limited to this. For example, the direction of the unit cell structure in the orthosis can be appropriately set by the manufacturer. For example, in the case of a hand-worn orthosis, the normal direction at the contact point between the hand and the orthosis may be defined as the load direction. In this case, the unit cell structure of FIG. 3 is arranged in the orthosis so that the vertical downward direction in the drawing corresponds to the normal direction at the contact point with the human body.

According to the embodiment described above, it is possible to provide an orthosis having appropriate physical properties corresponding to the region and a method of manufacturing the orthosis.

### (Second embodiment)

The conventional technique cannot ensure the designing of an orthosis having appropriate physical properties. For example, in conventional orthosis design, what types of physical properties are given in the material used for manufacturing the insole is decided on the basis of the experience of the prosthetist who designs the orthosis. For this reason, when creating orthoses for a subject with a certain symptom by different prosthetists, materials with different physical properties are often used for individual orthoses, and thus, designing of orthoses with appropriate physical properties is not to be ensured.

Second to fifth embodiments aim to provide an information processing apparatus, a system, an orthosis manufacturing method, an information processing method, and a program, capable of designing an orthosis having appropriate physical properties.

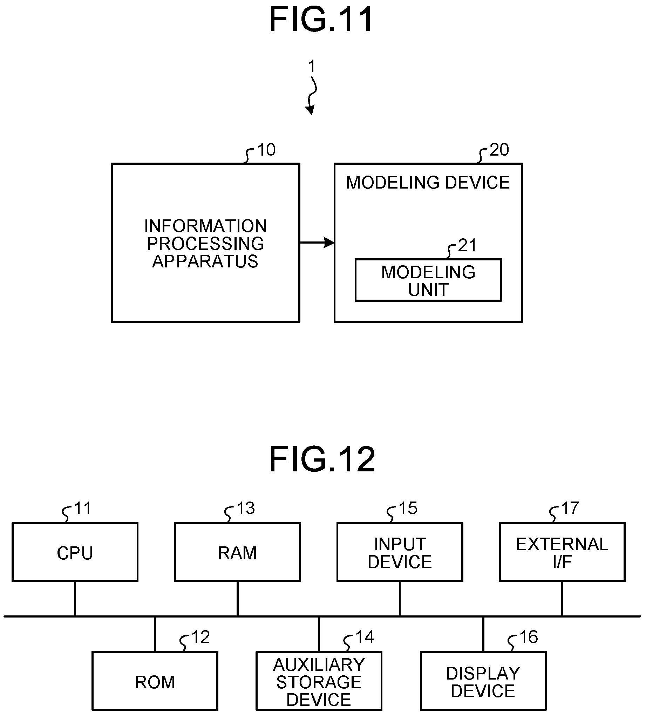

FIG. 11 is a diagram illustrating an example of a schematic configuration of a system 1 of the second embodiment. As illustrated in FIG. 11, the system 1 of the embodiment includes an information processing apparatus 10, and a modeling device 20. The devices illustrated in FIG. 11 are directly or indirectly communicable with each other by a network such as a local area network (LAN) or a wide area network (WAN).

The information processing apparatus 10 generates model data for designing an orthosis. The model data is information that is the basis for modeling an orthosis worn by a subject (orthosis wearer) by using the modeling device 20. The processing details for generating model data will be described below. The information processing apparatus 10 transmits the generated model data to the modeling device 20.

In the following embodiment, a medical insole will be described as an example of the orthosis. However, the embodiment is not limited to the insole but is widely applicable to orthoses to be applied to individual sites of a human body, such as a hand, elbow, knee, shoulder, or head. Furthermore, the embodiment is not limited to medical orthoses, and is widely applicable to an orthosis used for assisting or protecting physical functions in sports and daily life, for example. The insole is also referred to as an internal fitting, an inner sole, or the like. In addition, the insoles according to the following embodiments correspond to the insoles 100 described in the first embodiment.

The modeling device 20 includes a modeling unit 21 that models an orthosis to be worn by the subject based on the model data received from the information processing apparatus 10. In the present embodiment, the modeling unit 21 is constituted with a group of hardware elements that provide the functions of a three-dimensional printer.

For example, the modeling unit 21 includes: a liquid layer (tank) for filling the photosensitive resin that is the material of the insole; and a laser for curing the photosensitive resin in the liquid layer by a photopolymerization reaction. By using a laser, the modeling unit 21 regioselectively cures the position in the liquid layer defined based on the model data and thereby models a three-dimensional structure corresponding to the model data.

Next, the information processing apparatus 10 of the present embodiment will be described. FIG. 12 is a diagram illustrating an example of a hardware configuration of the information processing apparatus 10 of the second embodiment. As illustrated in FIG. 12, the information processing apparatus 10 includes a central processing unit (CPU) 11, read only memory (ROM) 12, random access memory (RAM) 13, an auxiliary storage device 14, an input device 15, a display device 16, and an external interface (I/F) 17.

The CPU 11 is a processor (processing circuit) that comprehensively controls the operation of the information processing apparatus 10 by executing a program and implements various functions of the information processing apparatus 10. Various functions of the information processing apparatus 10 will be described below.

The ROM 12 is non-volatile memory and stores various types of data (information written at the manufacturing stage of the information processing apparatus 10) including a program for starting the information processing apparatus 10. The RAM 13 is volatile memory having a working region of the CPU 11. The auxiliary storage device 14 stores various data such as a program executed by the CPU 11. The auxiliary storage device 14 is constituted with a hard disc drive (HDD), a solid state drive (SSD), or the like.

The input device 15 is a device for an operator of the information processing apparatus 10 to perform various operations. Examples of the input device 15 are a mouse, a keyboard, a touch panel, or hardware keys. In addition, the operator corresponds to, for example, a prosthetist who creates an orthosis, a medical person such as a doctor or a physiotherapist, and a subject who wears the orthosis.

The display device 16 displays various types of information. For example, the display device 16 displays image data, model data, a graphical user interface (GUI) for receiving various operations from an operator, a medical image, or the like. Examples of the display device 16 are a liquid crystal display, an organic electro luminescence (EL) display, or a cathode ray tube display. The input device 15 and the display device 16 may be integrated in the form of a touch panel, for example.

The external I/F 17 is an interface for connecting (communicating) with an external device such as the modeling device 20. Although not illustrated, the external I/F17 may be connected to a diagnostic medical imaging device such as an X-ray computed tomography (CT) device or a magnetic resonance imaging (MRI) device.

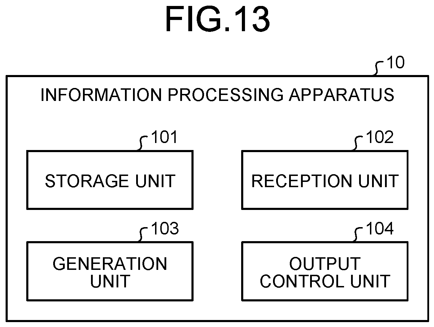

FIG. 13 is a diagram illustrating an example of a function of the information processing apparatus 10 of the second embodiment. In the example of FIG. 13, only the functions related to the present embodiment are illustrated, but the functions of the information processing apparatus 10 are not limited to these. As illustrated in FIG. 13, the information processing apparatus 10 includes a storage unit 101, a reception unit 102, a generation unit 103, and an output control unit 104. The storage unit 101 is implemented by, for example, the auxiliary storage device 14 (for example, an HDD). The reception unit 102, the generation unit 103, and the output control unit 104 are implemented by the CPU 11, for example.

The storage unit 101 stores preset information of model data, a database referred to when generating model data, or the like. The storage unit 101 can also store image data (DICOM data) captured by a diagnostic medical imaging device.

The reception unit 102 has a function of receiving input from the operator. For example, the reception unit 102 receives an input operation performed by the operator via the input device 15, converts the received input operation into an electric signal, and transmits the signal to individual units in the information processing apparatus 10.

For example, the operator operates the input device 15 to set the outer shape data of an insole. The outer shape data of the insole includes information such as the shape, size, thickness, position of each region, and positional relationship of individual regions for a plurality of regions having mutually different physical properties.

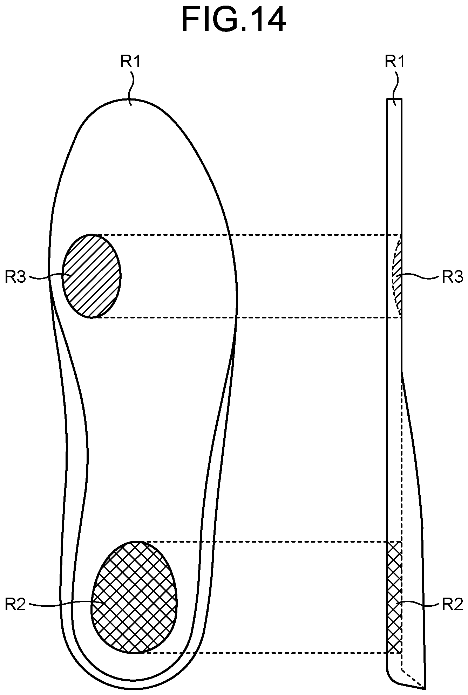

FIG. 14 is a diagram illustrating an example of outer shape data of the insole of the second embodiment. The left figure of FIG. 14 illustrates a plan view of an insole for the right foot as viewed from a contact surface side in direct or indirect contact with individual sites of a human body. The right figure in FIG. 14 illustrates a side view of the left figure. The toe side of the insole is illustrated on the upper side of FIG. 14, while the heel side of the insole is illustrated on the lower side of FIG. 14.

As illustrated in FIG. 14, for example, the outer shape data of the insole includes information regarding a region R1, a region R2, and a region R3. Of these, the region R1 is a region that constitutes the overall outer shape of the insole. The region R2 is a region where the site near the calcaneus (heel bone) touches. The region R3 is a region coming in contact with the site near the metatarsal joint of the big toe.

Here, the outer shape data of the insole includes information regarding the shape of the region, the size of the region, the thickness of the region, and the position of the region for each of the regions R1, R2, and R3. Furthermore, the outer shape data of the insole includes information indicating the position of the region R2 with respect to the region R1 and information indicating the position of the region R3 with respect to the region R1.

Specifically, preset information of the outer shape data of the insole is stored in advance in the storage unit 101. In accordance with the operation from an operator, the reception unit 102 reads out the preset information of the outer shape data of the insole from the storage unit 101. Subsequently, the operator performs an operation of setting (adjusting) information such as the number, size, shape, thickness, and position of the regions set in the preset information of the outer shape data of the insole, in accordance with the subject. Having received this operation, the reception unit 102 sets the outer shape data of the insole in accordance with the received operation.

The reception unit 102 then receives input of identification information for identification of sensations. Here, the identification information is information for identifying the sensation given to the user by using an object, such as "sensation A" and "sensation B". However, it is preferable to use expressions in sensuous terms such as "hardness" and "fittability" rather than using simple identification information because such expressions are capable of intuitively transmitting information to the operator. Hereinafter, the information expressed in such sensuous terms will be referred to as "texture information". The texture information is an example of identification information that identifies the sensation.

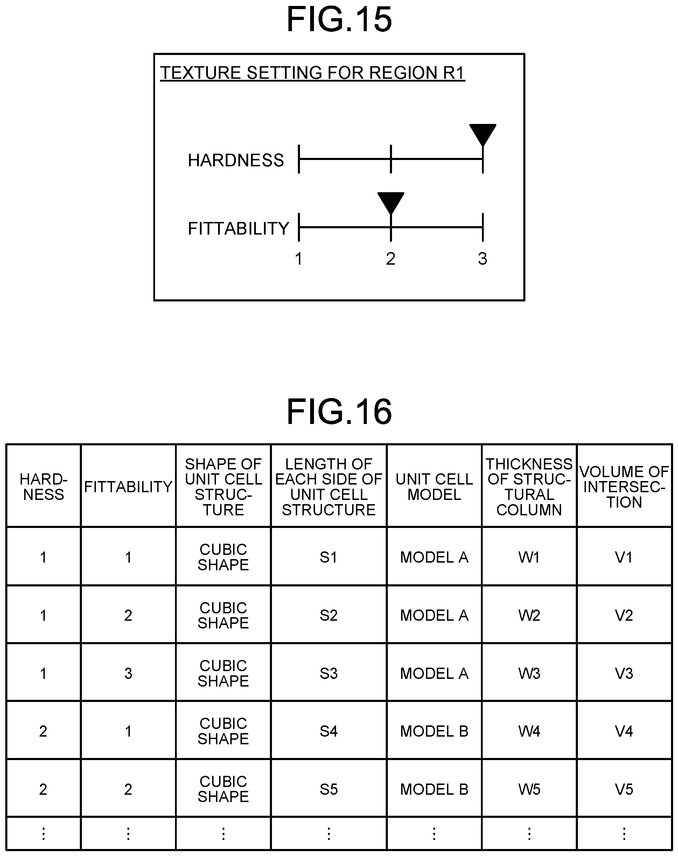

FIG. 15 is a diagram illustrating an example of an input screen for texture information of the second embodiment. As illustrated in FIG. 15, for example, the reception unit 102 displays a screen for inputting texture information for each of regions of the insole. This screen displays scales by which "hardness" and "fittability" can be designated in three levels. Here, "hardness" is texture information representing the hardness of an object (each of regions of the orthosis). Furthermore, the "fittability" is texture information indicating adhesion between the person wearing the orthosis and the object, for example.

For example, the operator inputs texture information and degree information by changing the position of a cursor (inverted triangle in the figure) at the top of each of scales for "hardness" and "fittability" individually. The example in FIG. 15 illustrates an exemplary case where "3" is designated as the degree information of "hardness" and "2" is designated as the degree information of "fittability". In the following description, texture information and degree information are combined in description. For example, the hardness "3" indicates designation of the texture information "hardness" and the degree information "3".

After the operator has input the texture information and the degree information, the reception unit 102 receives the input texture information and the degree information. Specifically, the reception unit 102 receives input of texture information for each of a plurality of regions of the orthosis. The reception unit 102 then transmits the received texture information and degree information to the generation unit 103. In the example illustrated in FIG. 15, the reception unit 102 transmits the hardness "3" and the fittability "2" to the generation unit 103.

The contents illustrated in FIGS. 14 and 15 are merely an example, and the present invention is not limited to the contents in the drawings. For example, the number, position, size, shape, and thickness of each of regions included in the outer shape data of the insole illustrated in FIG. 14 can be flexibly changed according to the purpose of use and the symptom of the wearer. Furthermore, the texture information illustrated in FIG. 15 is not limited to "hardness" and "fittability", and any texture information can be set. In addition, the degree information is not limited to three levels, and can be set in any number of levels.

The generation unit 103 generates model data for designing the orthosis based on the texture information received by the reception unit 102. For example, with reference to the related information (table) in which the texture information, the degree information, and the shape parameter that defines the physical properties of the orthosis are associated with each other, the generation unit 103 determines the shape parameter corresponding to the combination of the texture information and the degree information received by the reception unit 102.

Here, individual regions of the insole according to the present embodiment are constituted with a unit cell structure different from each other. The unit cell structure is a unit cell structure in which a space of a polygonal prism shape is defined as one unit, and includes a plurality of structural columns connecting two points among a plurality of vertices forming the polygonal prism shape. The unit cell structure of each of regions of the insole is defined by a parameter including at least one of the number of a plurality of structural columns constituting the unit cell structure with a polygonal prism shaped space defined as one unit, a direction of the structural columns, a thickness of the structural columns, a volume of intersection of the structural columns, or size of the unit cell structure.

That is, the generation unit 103 determines, as the shape parameter, a parameter including at least one of the number of a plurality of structural columns constituting the unit cell structure with a polygonal prism shaped space as one unit, the direction of the structural columns, the thickness of the structural columns, the volume of intersection of the structural columns, the shape of the unit cell structure, or the size of the unit cell structure. In addition, the generation unit 103 determines the number of structural columns and the direction of the structural columns by determining a unit cell model representing an arrangement pattern of a plurality of structural columns constituting the unit cell structure. The generation unit 103 then generates model data including the determined shape parameter. Hereinafter, the processes and shape parameters of the generation unit 103 will be described with reference to FIGS. 16 to 23.

FIG. 16 is a diagram illustrating an example of related information referred to in the generation unit 103 of the second embodiment. This related information (table) is information in which a combination of texture information and degree information is associated with various shape parameters. This related information is stored in advance in the storage unit 101, for example.

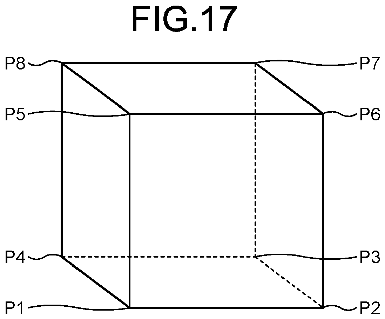

As illustrated in FIG. 16, the related information is information in which items of "hardness", "fittability", "shape of unit cell structure", "length of each side of unit cell structure", "unit cell model", "thickness of structural column", and "volume of intersection" are associated with each other. Here, "shape of unit cell structure", "length of each side of unit cell structure", "unit cell model", "thickness of structural column", and "volume of intersection" are an example of shape parameters. Various shape parameters will be described below with reference to FIGS. 17 to 23.

For example, a first record of related information in FIG. 16 has hardness: "1", fittability: "1", shape of unit cell structure: "cubic shape", length of each side: "S1", unit cell model: "model A", the thickness of the structural column: "W1", and the volume of the intersection: "V1" associated with each other. This means that, when a combination of hardness: "1" and fittability: "1" has been input, the shape parameters of the shape of the unit cell structure: "cubic shape", the length of each side: "S1", the model: "model A", the thickness of the structural column: "W1", and the volume of intersection: "V1" will be selected. Furthermore, FIG. 16 indicates that various shape parameters are similarly stored in association with other combinations of hardness and fittability.

In this manner, in the related information illustrated in FIG. 16, various shape parameters are associated and stored for individual combinations of the hardness of "1" to "3" and the fittability of "1" to "3".

The contents illustrated in FIG. 16 are merely an example, and the present invention is not limited to the contents in the drawing. For example, although FIG. 16 is an exemplary case where the hardness and the fittability are individually input (selected) in three levels, the levels can be set to any number. Furthermore, it is preferable that the unit cell structure in each of the regions in the insole has one unit of a space having the identical shape and the identical size (here, the length of each side). Furthermore, the related information of FIG. 16 may be stored in an external storage device communicable with the information processing apparatus 10, not limited to the storage unit 101. For example, when the related information is stored in an external storage device on a network, the generation unit 103 can refer to the related information stored in the external storage device on the network.

Hereinafter, various shape parameters, namely, the shape of the unit cell structure, the length of each side of the unit cell structure, the model, the thickness of the structural columns, and the volume of the intersection, will be described with reference to FIGS. 17 to 23.

First, the "shape of the unit cell structure" and the "length of each side of the unit cell structure" in FIG. 16 will be described. The "shape of the unit cell structure" is the shape of the space defined as one unit of the unit cell structure. The "length of each side of the unit cell structure" corresponds to the size of the space defined as one unit of the unit cell structure.

FIG. 17 is a diagram illustrating the shape of the unit cell structure and the length of each side of the second embodiment. As illustrated in FIG. 17, the "shape of the unit cell structure" corresponds to a cubic shape constituted with six square faces. This cubic space has eight points P1 to P8 corresponding to the vertices. Furthermore, in the example of FIG. 17, since this space has a cubic shape, the lengths of all sides are equal. The length of each side of the space in FIG. 17 is expressed by a distance between the points P1 and P2, for example.

Here, the notation method of lines and faces in the unit cell structure will be described. In the present embodiment, when a line or a face is described, the vertices constituting the line or the face are described in parentheses. For example, the notation "line (P1, P2)" represents a line connecting the point P1 and the point P2. The notation "line (P1, P7)" represents a line (diagonal) connecting the point P1 and the point P7. Furthermore, the notation "face (P1, P2, P3, P4)" represents a face (bottom face) having points P1, P2, P3, and P4 as vertices. The structural columns are described similarly to the line notation method.

The unit cell structure according to the embodiment includes a plurality of structural columns connecting two of the plurality of points P1 to P8. That is, the spatial shape of FIG. 17 corresponds to the shape of the unit cell structure. The number and direction (arrangement direction) of the structural columns are defined in advance by a unit cell model, for example.

The contents illustrated in FIG. 17 are merely an example, and the present invention is not limited to the contents in the drawing. For example, the cubic space illustrated in FIG. 17 is merely an example, and the spatial shape defined as one unit of the unit cell structure may be a polygonal prism shape. In this case, it is preferable that the unit cell structure has a space having any one of a triangular prism shape, a quadrangular prism shape, or a hexagonal prism shape, as one unit. As the triangular prism shape, a regular triangular prism shape is preferable. Furthermore, as the quadrangular prism shape, a rectangular parallelepiped shape is preferable in addition to the above cubic shape. Furthermore, as the hexagonal prism shape, a regular hexagonal prism shape is preferable. Note that it is preferable that the unit cell structure of each of the regions R1 to R3 constituting the orthosis has an identical spatial shape. Furthermore, the structural column is a column such as a polygonal prism shape or a cylindrical shape. Furthermore, the lateral direction of the structural column is a direction orthogonal to an axial direction of the structural column.

Next, the "unit cell model" of FIG. 16 will be described. The unit cell model represents the basic skeleton shape of the unit cell structure.

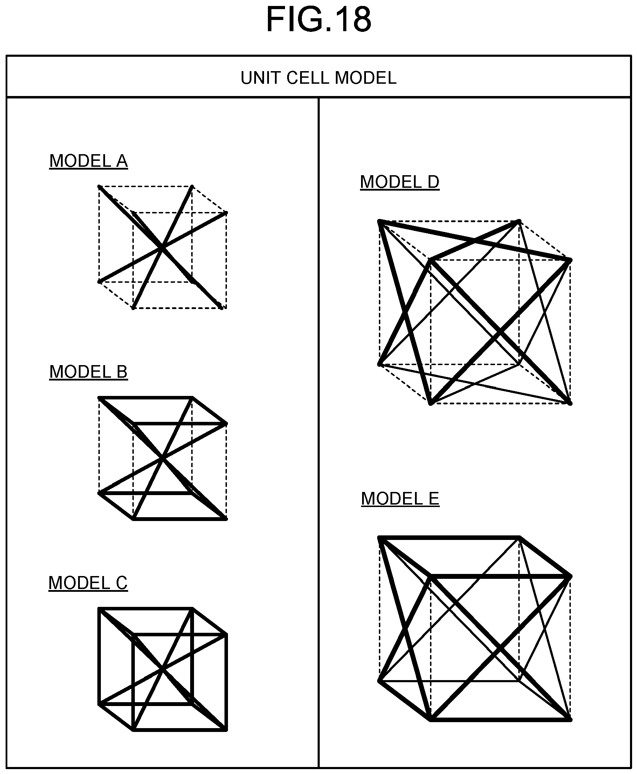

FIG. 18 is a diagram illustrating the unit cell model of the second embodiment. In FIG. 18, the dashed lines indicate a cubic space (space in FIG. 17) corresponding to one unit. The solid lines indicate the existence of structural columns. Preferably, the unit cell model illustrated in FIG. 18 is arranged in each of regions of the insole so that its arrangement direction matches the direction of a load applied to the orthosis in the vertical downward direction of FIG. 18. The direction of load applied to the orthosis is a direction in which the load received from each of sites of the human body is applied.

As illustrated in FIG. 18, for example, a unit cell model includes model A, model B, model C, model D, and model E. Here, model A, model B, and model C each have an intersection of structural columns at a center of a cubic space. Furthermore, model D and model E each have an intersection of structural columns at the center of at least one of a plurality of faces constituting the cubic shape.
Model A is a unit cell model with four structural columns arranged along the diagonals of the cubic shape. Specifically, model A has a structural column (P1, P7), a structural column (P2, P8), a structural column (P3, P5), and a structural column (P4, P6).
Model B is a unit cell model including, in addition to the four structural columns similar to model A, eight structural columns arranged along a plurality of sides surrounding the bottom face and the top face constituting the cubic shape. Specifically, model B includes four structural columns included in model A. In addition, model B includes four structural columns that surround the bottom face (P1, P2, P3, P4), namely, a structural column (P1, P2), a structural column (P2, P3), a structural column (P3, P4), and a structural column (P4, P1). In addition, model B has four structural columns that surround the top face (P5, P6, P7, P8), namely, a structural column (P5, P6), a structural column (P6, P7), a structural column (P7, P8), and a structural column (P8, P5) .
Model C is a unit cell model further including four structural columns arranged in the load direction in addition to 12 structural columns similar to model B. Specifically, model C has 12 structural columns included in model B. Model C further includes four structural columns, namely, a structural column (P1, P5), a structural column (P2, P6), a structural column (P3, P7), and a structural column (P4, P8) arranged in the direction of the load applied to the insole.
   That is, the difference between model B and model C is whether the model includes the four structural columns, namely, a structural column (P1, P5), a structural column (P2, P6), a structural column (P3, P7), and a structural column (P4, P8) arranged in the direction of the load.
Model D is a unit cell model with 12 structural columns arranged along the diagonals of each of the six faces. Specifically, model D has two structural columns, namely, a structural column (P1, P3) and a structural column (P2, P4) along the diagonals of the bottom face (P1, P2, P3, P4). Specifically, model D has two structural columns, namely, a structural column (P5, P7) and a structural column (P6, P8) along the diagonals of the top face (P5, P6, P7, P8) . Specifically, model D has two structural columns, namely, a structural column (P1, P6) and a structural column (P2, P5) along the diagonals of the side face (P1, P2, P5, P6). Moreover, model D has two structural columns, namely, a structural column (P2, P7) and a structural column (P3, P6) along the diagonals of the side face (P2, P3, P6, P7). In addition, model D has two structural columns, namely, a structural column (P3, P8) and a structural column (P4, P7) along the diagonals of the side face (P3, P4, P7, P8). In addition, model D has two structural columns, namely, a structural column (P1, P8) and a structural column (P4, P5) along the diagonals of the side face (P1, P4, P5, P8) .

As compared with model D, model E is a unit cell model further including eight structural columns arranged along a plurality of sides surrounding the bottom face and the top face, without including the structural columns along the diagonals of the bottom face or the top face. Specifically, model E has two structural columns, namely, a structural column (P1, P6) and a structural column (P2, P5) along the diagonals of the side face (P1, P2, P5, P6). In addition, model E has two structural columns, namely, a structural column (P2, P7) and a structural column (P3, P6) along the diagonals of the side face (P2, P3, P6, P7). In addition, model E has two structural columns, namely, a structural column (P3, P8) and a structural column (P4, P7) along the diagonals of the side face (P3, P4, P7, P8). In addition, model E has two structural columns, namely, a structural column (P1, P8) and a structural column (P4, P5) along the diagonals of the side face (P1, P4, P5, P8). In addition, model E has four structural columns surrounding the bottom face (P1, P2, P3, P4), namely, a structural column (P1, P2), a structural column (P2, P3), a structural column (P3, P4), and a structural column (P4, P1). In addition, model E has four structural columns surrounding the top face (P5, P6, P7, P8), namely, a structural column (P5, P6), a structural column (P6, P7), a structural column (P7, P8), and a structural column (P8, P5).

That is, the difference between model D and model E is the presence or absence of structural columns arranged on the bottom face and top face. Specifically, model D has four structural columns, namely, a structural column (P1, P3), a structural column (P2, P4), a structural column (P5, P7), and a structural column (P6, P8), along the diagonals of the bottom face and the top face. In addition, model E has eight structural columns surrounding the bottom face and the top face, namely, a structural column (P1, P2), a structural column (P2, P3), a structural column (P3, P4), a structural column (P4, P1), a structural column (P5, P6), a structural column (P6, P7), a structural column (P7, P8), and a structural column (P8, P5).

In FIG. 6, the structural columns of model D and model E are illustrated by solid lines of two types of thickness. However, this is intended to clarify the illustration, rather than indicating the actual thickness of the structural columns. In other words, the thicker solid line indicates the structural column included in the "faces visible on the front side of the box" when the cubic space is regarded as a box. Furthermore, the thinner solid line indicates a structural column included only in the "faces visible on the back side of the box" when the cubic space is regarded as a box. The "faces visible on the front side of the box" correspond to the top face (P5, P6, P7, P8), the side face (P1, P2, P5, P6), and the side face (P1, P4, P5, P8). The "faces visible on the back side of the box" correspond to the bottom face (P1, P2, P3, P4), the side face (P2, P3, P6, P7), and the side face (P3, P4, P7, P8) .

That is, each of regions of the insole of the present embodiment has a structure containing a plurality of unit cell models repeatedly and continuously arranged. Specifically, each of regions of the insole has at least one layer including a plurality of unit cell structures arranged on an identical plane, with these layers being stacked to form the region. The cross-sectional shape of the structural column may be any shape such as a polygon including a quadrangle, a pentagon or a hexagon, a circle or an ellipse, or the like.

The contents illustrated in FIG. 18 are merely an example, and the present invention is not limited to the contents in the drawing. For example, the model illustrated in FIG. 18 is merely an example, and the unit cell model may have structural columns at any positions. Still, it is preferable that the unit cell structure includes a plurality of structural columns so that any of the structural columns passes through all the vertices constituting the space of the polygonal prism shape.

Furthermore, the unit cell structure is not to limited to a structure having an intersection of structural columns at the center of a cubic space, or a structure having an intersection of structural columns at the center of at least one of a plurality of faces constituting the cubic shape. Still, it would be preferable that the unit cell structure has an intersection where at least two structural columns intersect at different position than the plurality of vertices.

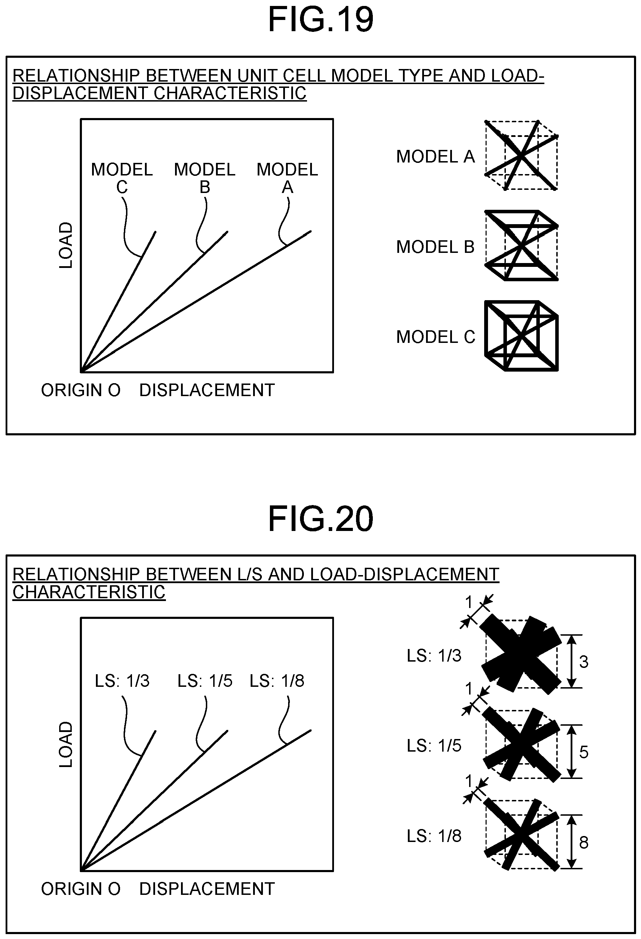

FIG. 19 is a diagram illustrating a relationship between the type of unit cell model and the load-displacement characteristic of the second embodiment. Here, the load-displacement characteristic represents a relationship between a load [N] applied to an object and a displacement [mm] of the object due to the load, and is utilized as a physical index of the hardness or adhesion of the object. In the present embodiment, the load-displacement characteristic is measured by applying a load to an object having a unit cell structure. In the graph of load-displacement characteristic, the vertical axis of the graph corresponds to the load [N], and the horizontal axis of the graph corresponds to the displacement [mm]. That is, in the graph of load-displacement characteristic, the harder the object, the steeper the graph, and the softer the object, the gentler the graph. Normally, although the graph is often obtained as a curve, the following figures use a straight line for simplification.

In FIG. 19, the load-displacement characteristic of the object constituted with the unit cell models of the models A, B, and C illustrated in FIG. 18 will be described. In FIG. 19, the thickness of the structural columns of the models A, B, and C and the volume of the intersection are constant.

From the load-displacement characteristic illustrated in FIG. 19, it is observed that the larger the number of structural columns included in each of the models, the harder the object tends to be. Furthermore, based on the load-displacement characteristic illustrated in FIG. 19, it is observed that the closer the direction of the structural column is to the load direction, the harder the object tends to be. Therefore, constituting the individual regions with different types of unit cell models would make it possible to provide a plurality of regions having different physical properties.

Next, the "thickness of structural columns" in FIG. 16 will be described. The thickness of the structural columns is the thickness of each of the structural columns in the unit cell model, and is represented by the ratio of the thickness (L) of the structural columns to the length (S) of one side of the unit space.

FIG. 20 is a diagram illustrating a relationship between L/S and the load-displacement characteristic of the second embodiment. FIG. 20 uses model A of FIG. 18 to explain the load-displacement characteristic of objects having structural columns of different thicknesses. In FIG. 20, the volume of the intersection of each of the objects is constant.

Furthermore, FIG. 20 illustrates as an example of the thickness of the structural column, in which the ratio of the thickness (L) of the structural column and the length (S) of one side of the unit space is different. In the example of FIG. 20, "L/S: 1/3" corresponds to the case where the structural column is the thickest, with "L/S: 1/5" and "L/S: 1/8" corresponding to the case where the structural column is thinner and thinnest, respectively. The thickness of the structural column can be, for example, the length of the longest straight line among the straight lines passing through the center of gravity in the "cross section in the lateral direction" of the structural column.

From the load-displacement characteristic illustrated in FIG. 20, it is observed that the larger the L/S (thicker the column), the harder the object tends to be. In addition, the smaller the L/S (thinner the column), the softer the object tends to be. Therefore, constituting a plurality of regions with structural columns with different thicknesses would make it possible to provide a plurality of regions having different physical properties.

Next, the "volume of the intersection" in FIG. 16 will be described. The volume of the intersection is the volume of the site (intersection) where at least two structural columns intersect in the unit space.

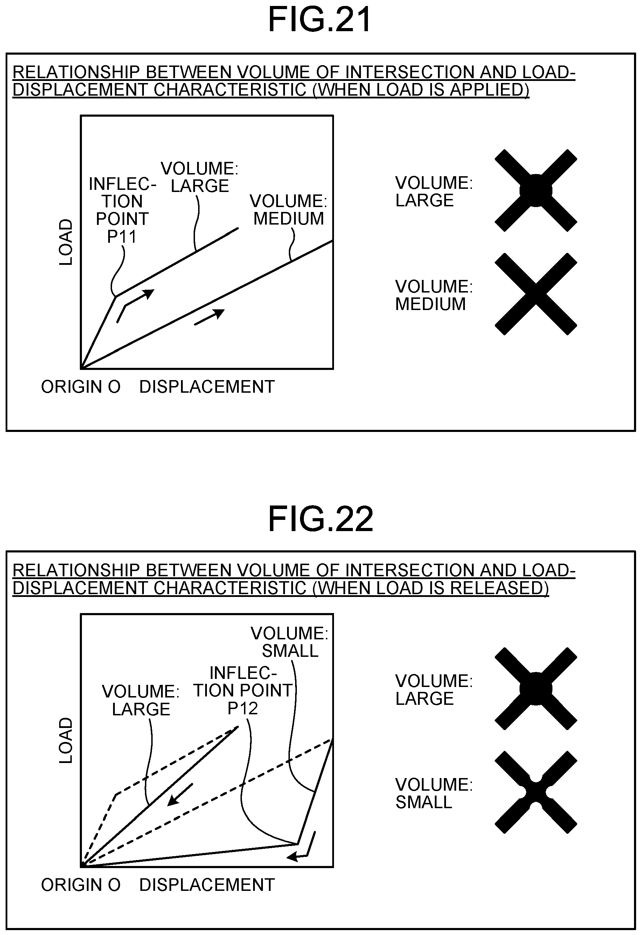

FIG. 21 is a diagram illustrating a relationship between the volume of an intersection and the load-displacement characteristic of the structural columns in the unit cell model generated in the second embodiment. FIG. 21 illustrates a relationship between the volume of the intersection and the load-displacement characteristic when a load is applied. FIG. 21 uses model A of FIG. 18 to explain the load-displacement characteristic of objects having intersections with different volumes. In FIG. 21, the thickness (L) of the structural column of each of objects is constant. In the load-displacement characteristic when a load is applied as illustrated in FIG. 21, a point (position) on the graph indicating the displacement corresponding to the load moves in the direction of the arrow in the graph.

In the load-displacement characteristic illustrated in FIG. 21, an inflection point P11 is observed in the graph in a case where the volume of the intersection is large. In other words, an object with a large volume at the intersection behaves like a hard substance between an origin O and the inflection point P11 together with application with a load, and behaves like a soft substance after the inflection point P11. The change in physical properties (change in the slope of the graph) in this load-displacement characteristic is felt as adhesion (adhesion at application of load) when a person using the orthosis applies a load to the insole. Moreover, an object with a medium volume at the intersection behaves similarly to a soft object. Therefore, constituting a plurality of regions with intersections with different volumes would make it possible to provide a plurality of regions having different physical properties.

FIG. 22 is a diagram illustrating a relationship between the volume of the intersection and the load-displacement characteristic of the second embodiment. FIG. 22 illustrates a relationship between the volume of the intersection and the load-displacement characteristic when a load is released. FIG. 22 uses model A of FIG. 18 to explain the load-displacement characteristic of objects having intersections with different volumes. In FIG. 22, the thickness (L) of the structural column of each of objects is constant. In the load-displacement characteristic when a load is released as illustrated in FIG. 22, a point (position) on the graph indicating the displacement corresponding to the load moves in the direction of the arrow in the graph. The dashed graph illustrated in FIG. 22 corresponds to the graph when a load is applied.

In the load-displacement characteristic illustrated in FIG. 22, when the volume of the intersection is large, the shape recovery speed is high and a slope relatively close to linear can be obtained. That is, for an object having a large volume at the intersection, the displacement can make a quick recovery when the load is released. This physical property is felt as adhesion (adhesion at release of load) when the person using the orthosis releases the load from the insole. By contrast, when the volume of the intersection is small in the load-displacement characteristic illustrated in FIG. 22, the shape recovery speed is slow with observation of an inflection point P12. In other words, regarding an object with a small volume at the intersection, the amount of change in the displacement is small up to the inflection point P12 together with the release of the load, and the shape recovers to the original shape between the inflection point P12 and the origin O. Therefore, constituting a plurality of regions with intersections with different volumes would make it possible to provide a plurality of regions having different physical properties.

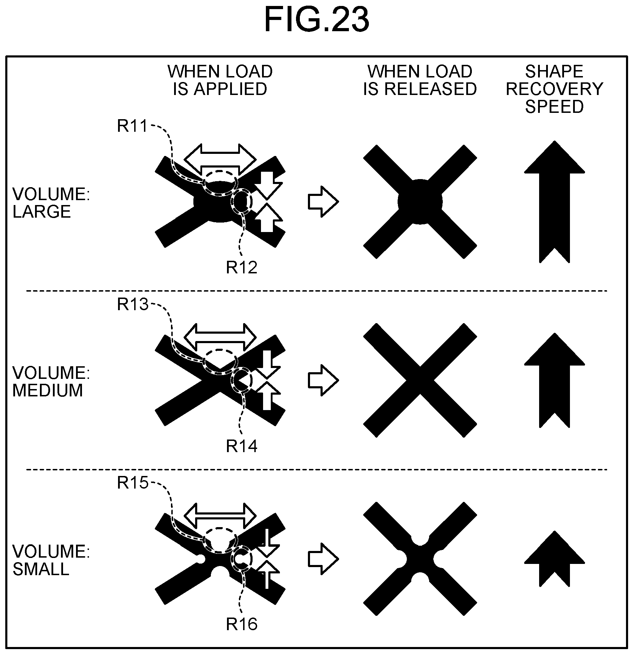

FIG. 23 is a diagram illustrating a difference in shape recovery speed depending on the volume of the intersection of the second embodiment. FIG. 23 describes the difference in shape recovery speed according to the volume of the intersection when a load is applied from the top face to the bottom face of the unit cell structure in model A of FIG. 18. FIG. 23 illustrates the change in the shape of the intersection at transition from application of the load to the release of the load, and the shape recovery speed in the change. The upper row of FIG. 23 illustrates a case where the intersection has a large volume, the middle row illustrates a case where the intersection has a medium volume, and the lower row illustrates a case where the intersection has a small volume. The length of the arrow indicating the shape recovery speed corresponds to the degree of the shape recovery speed.

With FIG. 23, the shape of the intersection will be described. When the volume is not adjusted in particular, the intersection has a shape as illustrated in the middle row of FIG. 23 (when the load is released). In the present embodiment, this shape will be described as a state in which the intersection has a medium volume.

When the intersection has a large volume, the intersection has a spherical shape or a polyhedral shape with the intersection as a substantially center, as illustrated in the upper row of FIG. 23 (when the load is released). That is, the intersection has a positively raised shape as compared with a state in which a plurality of structural columns is simply intersected. In this case, the intersection has a cross section larger than the cross section of the structural column in the lateral direction. Specifically, the area of the cross section of the intersection is preferably 1.1 times or more and 20 times or less as compared with the cross section of the structural column in the lateral direction, more preferably be 1.2 times or more and 10 times or less, and still more preferable when the area is 1.5 times or more and 5 times or less, in particular. Here, the area of the cross section of the intersection is based on the surface having the largest area among the surfaces passing through the center of gravity of the intersection.

In contrast, when the intersection has a small volume, the intersection has a shape in which the structural columns in the vicinity of the intersection are thinned, as illustrated in the lower row of FIG. 23 (when the load is released). In this case, the intersection has a cross section smaller than the cross section of the structural column in the lateral direction. Specifically, the area of the cross section of the intersection is preferably 0.05 times or more and 0.9 times or less as compared with the cross section of the structural column in the lateral direction, more preferably be 0.1 times or more and 0.8 times or less, and particularly preferable when the area is 0.2 times or more and 0.7 times or less. Here, the area of the cross section of the intersection is based on the surface having the largest area among the surfaces passing through the center of gravity of the intersection.

Here, as illustrated in FIG. 23, energy for shape recovery is considered to be accumulated at the intersection when a load is applied. For example, when a load is applied from the upper side to the lower side in FIG. 23, the two structural columns on both sides of a region R13 at the intersection are expanded, resulting in accumulation of the restoring force for the recovery of the columns in the region R13. In addition, the two structural columns on both sides of a region R14 at the intersection are narrowed, resulting in accumulation of a repulsive force for their recovery in the region R14.

When the intersection has a large volume, the restoring force and the repulsive force are accumulated as a large amount of energy. Therefore, the restoring force of a region R11 is greater than the restoring force of the region R13, and the repulsive force of a region R12 is greater than the repulsive force of the region R14. Consequently, it is considered that the greater the volume of the intersection, the higher the shape recovery speed.

When the intersection has a small volume, the restoring force and the repulsive force are accumulated as a small amount of energy. Therefore, the restoring force of a region R15 is less than the restoring force of the region R13, and the repulsive force of a region R16 is less than the repulsive force of the region R14. Consequently, it is considered that the smaller the volume of the intersection, the lower the shape recovery speed.

In this manner, having a plurality of regions by a unit cell structure having mutually different shape parameters makes it possible to provide the insole with a plurality of regions having different physical properties.

The contents illustrated in FIGS. 17 to 23 are merely an example, and the present invention is not limited to the contents in the drawings. Furthermore, although FIG. 23 uses three levels for illustrating the volume of the intersection, the present invention is not limited to the three levels of volumes and can manufacture the intersection so as to have a desired volume.

In this manner, the related information in FIG. 16 is preliminarily set based on the relationship between the various shape parameters and the load-displacement characteristic described in FIGS. 19 to 23. In addition, with reference to the related information in FIG. 16, the generation unit 103 determines the shape parameter based on the texture information and the degree information received by the reception unit 102. Specifically, the generation unit 103 determines the shape parameters that define the physical properties of the orthosis for each of the plurality of regions. Subsequently, the generation unit 103 generates model data including the outer shape data of the insole and the determined shape parameter.

The output control unit 104 has a function of outputting various types of information. For example, the output control unit 104 controls the display device 16 to display the image data designated by the operator. Furthermore, the output control unit 104 transmits the information designated by the operator to an external device via the external I/F17.

For example, the output control unit 104 outputs the model data generated by the generation unit 103. Specifically, the output control unit 104 stores the model data in the storage unit 101. In addition, the output control unit 104 controls the display device 16 to display the model data. Furthermore, the output control unit 104 outputs the model data to the modeling device 20.

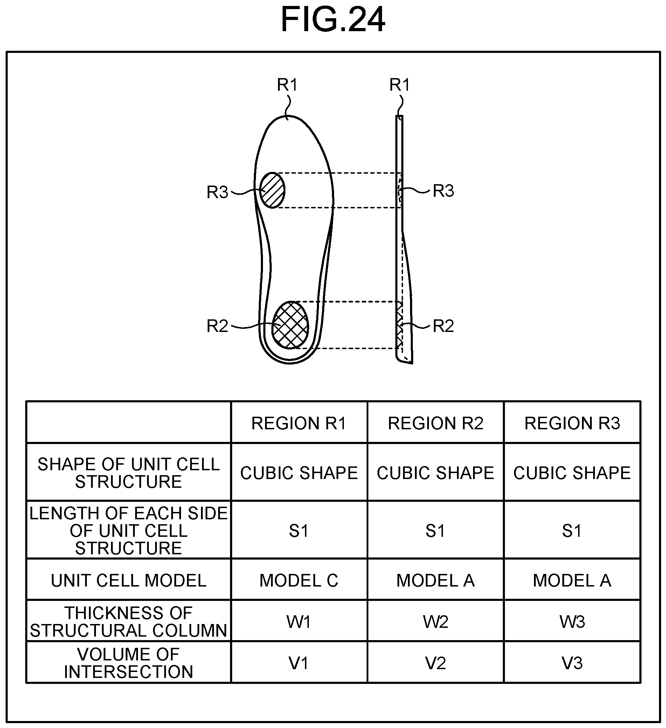

FIG. 24 is a diagram illustrating an example of a display screen provided by the output control unit 104 of the second embodiment. As illustrated on the upper side of FIG. 24, the output control unit 104 controls the display device 16 to display the outer shape data of the insole as image data. This image data includes information such as the position, size, shape, and thickness of each of the regions R1 to R3 of the insole.

Furthermore, as illustrated on the lower side of FIG. 24, the output control unit 104 controls the display device 16 to display a list of shape parameters of individual regions. In this list, the shape parameters determined for the individual regions R1 to R3 are described as text data. In the example illustrated in FIG. 24, the shape parameters of the unit cell structure of region R1 are the shape of the unit cell structure: "cubic shape", the length of each side of the unit cell structure: "S1", the unit cell model: "model C", the thickness of the structural columns: "W1", and the volume of the intersection: "V1". The shape parameters of the unit cell structure of region R2 are the shape of the unit cell structure: "cubic shape", the length of each side of the unit cell structure: "S1", the unit cell model: "model A", the thickness of the structural columns: "W2", and the volume of the intersection: "V2". The shape parameters of the unit cell structure of region R3 are the shape of the unit cell structure: "cubic shape", the length of each side of the unit cell structure: "S1", the unit cell model: "model A", the thickness of the structural columns: "W3", and the volume of the intersection: "V3".

The contents illustrated in FIG. 24 are merely an example, and the present invention is not limited to the contents in the drawing. For example, as the content of the model data displayed by the output control unit 104, various information can be displayed in accordance with the outer shape data of the insole set by the operator and the shape parameters determined based on the texture information and the degree information.

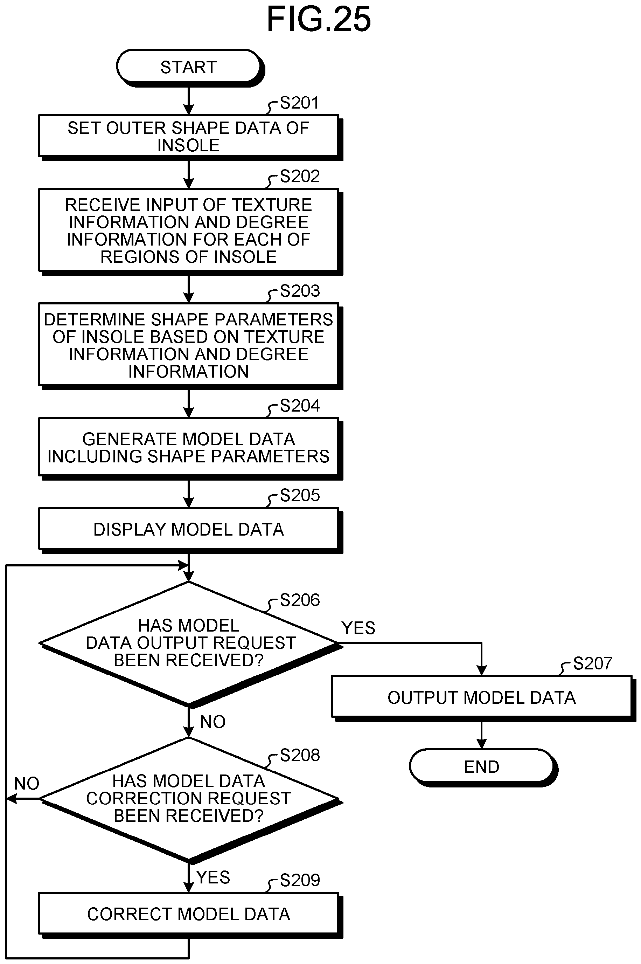

FIG. 25 is a flowchart illustrating an operation example of the information processing apparatus 10 of the second embodiment. Since the specific process of each of steps is as described above, detailed description thereof will be omitted as appropriate.

As illustrated in FIG. 25, the reception unit 102 sets the outer shape data of an insole (step S201). In addition, the reception unit 102 receives input of texture information and degree information for each of regions of the insole (step S202).

The generation unit 103 determines the shape parameters of the insole based on the texture information and the degree information (step S203). Subsequently, the generation unit 103 generates model data including the shape parameters (step S204). The output control unit 104 then displays the model data (step S205).

Next, the reception unit 102 determines whether the model data output request has been received (step S206). When the model data output request has been received (step S206, Yes), the output control unit 104 outputs the model data to the modeling device 20 (step S207), and ends the process.

In contrast, when the model data output request has not received (step S206, No), the reception unit 102 determines whether a model data correction request has been received (step S208). When the model data correction request has been received (step S208, Yes), the generation unit 103 corrects the model data in response to the model data correction request (step S209).

In contrast, when the model data correction request has not been received (step S208, No), or after step S209, the reception unit 102 returns to step S206 and determines whether the model data output request has been received. When the model data output request has been received in step S206 after step S209 (step S206, Yes), an output unit 205 outputs corrected model data to the modeling device 20 (step S207), and ends the process.

The processing procedure illustrated in FIG. 25 is merely an example, and is not limited to the contents illustrated in the drawing. For example, other processing procedures can be added (inserted) or the order of individual processes can be exchanged as long as there is no contradiction in the processing content. Furthermore, each of processing procedures does not necessarily have to be executed. For example, the processes of steps S208 and S209 do not necessarily have to be executed.

As described above, the information processing apparatus 10 of the present embodiment receives input of the identification information for identification of the sensation given to the subject by wearing the orthosis. Furthermore, the information processing apparatus 10 generates model data for designing the orthosis based on the received identification information. Furthermore, the information processing apparatus 10 outputs the generated model data. According to this procedure, the information processing apparatus 10 of the present embodiment can design an orthosis using a material having appropriate physical properties.

For example, according to the information processing apparatus 10 of the present embodiment, the physical properties defined by various shape parameters are expressed in sensuous terms that can be intuitively grasped easily by the operator. This allows the information processing apparatus 10 to input the physical properties of the orthosis by using the term that the operator intuitively recalls, making it possible to easily designate the material having appropriate physical properties.

### (First modification of second embodiment)

The second embodiment has described the case where the texture information is input using the cursor on the scale. However, the embodiment is not limited to this. For example, the reception unit 102 can receive input of texture information on a radar chart.

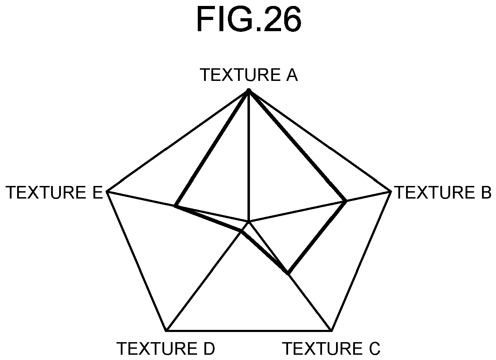

FIG. 26 is a diagram illustrating an example of an input screen of a first modification of the second embodiment. As illustrated in FIG. 26, for example, the reception unit 102 controls the display device 16 to display a radar chart defined by texture A, texture B, texture C, texture D, and texture E for each of regions of the insole. In addition, the reception unit 102 receives input of degree information regarding each of texture A, texture B, texture C, texture D, and texture E on the radar chart. The textures A to E correspond to hardness or fittability, for example.

In this manner, the reception unit 102 can also receive texture information and degree information on the radar chart illustrated in FIG. 26.

### (Second modification of second embodiment)

For example, the output control unit 104 can display a graph illustrating the load-displacement characteristic.

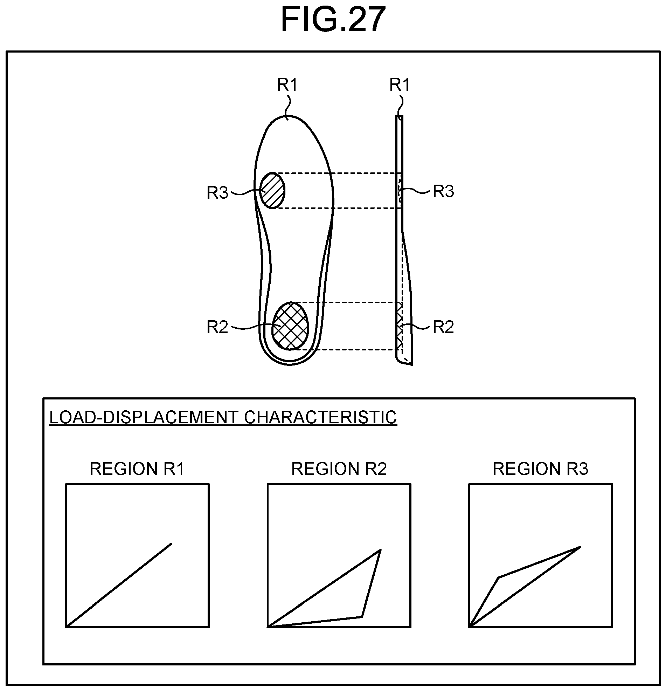

FIG. 27 is a diagram illustrating an example of a display screen of a second modification of the second embodiment. Since the outer shape data of the upper insole in FIG. 27 is similar to the outer shape data of the upper insole in FIG. 24, the description thereof will be omitted.

As illustrated on the lower side of FIG. 27, the output control unit 104 controls the display device 16 to display a graph of load-displacement characteristic for each of regions R1 to R3. This graph of load-displacement characteristic illustrates the physical properties defined by the shape parameters of each of regions. Note that the graph of the load-displacement characteristic illustrated in FIG. 27 may be displayed together with the model data of FIG. 24.

In this manner, the output control unit 104 can display a graph of physical property information represented by the relationship between the change in load and the change in displacement amount for the model data. With this configuration, the operator can easily grasp the physical properties represented by the shape parameters determined based on the texture information and the degree information input by the operator.

### (Third embodiment)

The second embodiment has described the case where the generation unit 103 determines the shape parameter corresponding to the texture information and the degree information with reference to the related information. However, the embodiment is not limited to this. For example, the generation unit 103 can specify the physical property information based on the texture information and the degree information and can determine the shape parameter based on the specified physical property information.

That is, in the information processing apparatus 10 of the third embodiment, the reception unit 102 receives input of the texture information and the input of the degree information indicating the degree of the sensation identified by the identification information. Subsequently, based on the identification information and the degree information, the generation unit 103 specifies the physical property information represented by the relationship between the change in the load and the change in the displacement amount. Based on the specified physical property information, the generation unit 103 then determines the shape parameter that defines the physical properties of the orthosis. Next, the output control unit 104 generates model data including the determined shape parameter.

The information processing apparatus 10 of the third embodiment has a configuration similar to the information processing apparatus 10 illustrated in FIG. 13, with a difference in a part of the processes. Therefore, in the third embodiment, the points different from those in the second embodiment will be mainly described, and the points having functions similar to the configurations described in the second embodiment will be omitted.

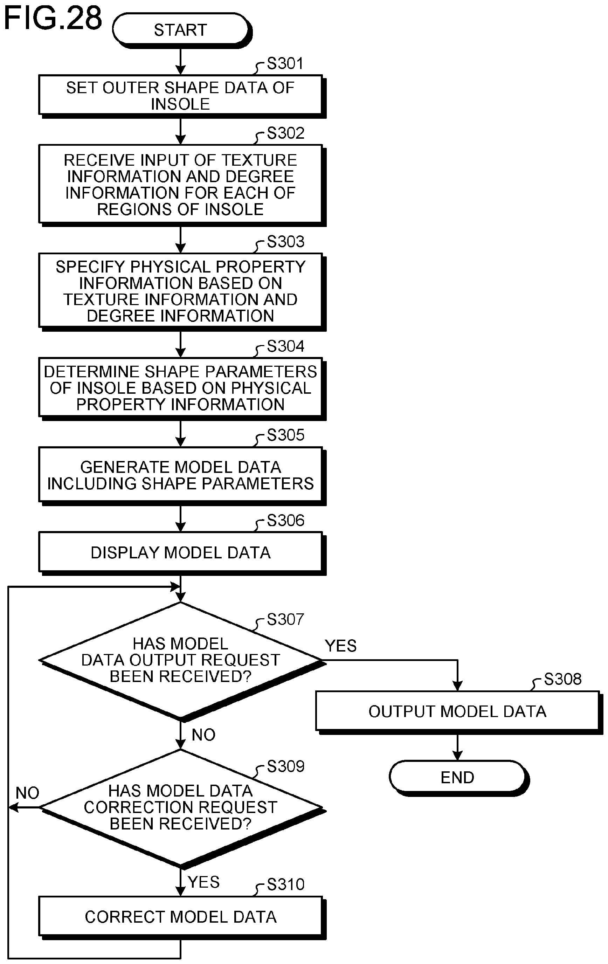

FIG. 28 is a flowchart illustrating an operation example of the information processing apparatus 10 of the third embodiment. Of the processing procedures illustrated in FIG. 28, the processes of step S301 and step S302 are similar to the processes of step S201 and step S202 illustrated in FIG. 25, and thus description thereof will be omitted. Since the processes of step S305 to step S310 are respectively similar to the processes of step S204 to step S209 illustrated in FIG. 25, the description thereof will be omitted.

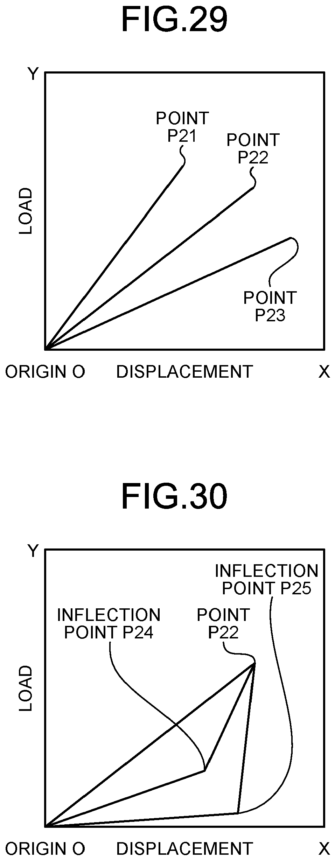

Furthermore, the following description will be given with reference to FIGS. 29 to 31. FIGS. 29 to 31 are diagrams illustrating processes of the generation unit 103 of the third embodiment. In the graphs of load-displacement characteristic of FIGS. 29 to 31, the displacement corresponds to the X direction and the load corresponds to the Y direction.

As illustrated in FIG. 28, the generation unit 103 specifies the physical property information based on the texture information and the degree information (step S303). Here, the physical property information is information indicating the physical properties of an object (orthosis), for example, information indicating a load-displacement characteristic (for example, information indicating a curve of a graph).

First, as illustrated in FIG. 29, the generation unit 103 specifies the "slope" in the load-displacement characteristic in accordance with the "hardness". Here, the storage unit 101 preliminarily stores information on the degree information of hardness and changes in the slope of the load-displacement characteristic in association with each other. For example, the hardness "3" is associated with the slope of the line connecting the origin O and a point P21. The hardness "2" is associated with the slope of the line connecting the origin O and a point P22. The hardness "1" is associated with the slope of the line connecting the origin O and a point P23.

For example, when the hardness "2" has been input by the operator, the generation unit 103 specifies the slope of the line connecting the origin O and the point P22.

Next, as illustrated in FIG. 30, the generation unit 103 specifies a "difference of integrated values" in the load-displacement characteristic in accordance with the "adhesion at the time of adhesion load release at the time of load release". Here, the "difference of integrated values" is a difference between an integrated value of the straight line connecting the displacement at the minimum load and the displacement at the maximum load in the load-change characteristic (area of a region enclosed by a straight line connecting the origin O and the point P22, a straight line representing X = (X component of the inflection point), and a straight line of Y = 0), and an integrated value of the line connecting the origin O, the inflection point, and the point P22 (area of a triangle obtained by connecting the origin O, the point P22, and the inflection point). Here, the storage unit 101 has preliminarily stored the degree information of adhesion at the time of adhesion load release at the time of load release, and the change in the difference of the integrated value of the load-displacement characteristic, in association with each other. For example, the adhesion "3" at the time of adhesion load release at time of load release is associated with the difference "0 (zero)" of the integrated values. This means that the load-displacement characteristic graph has no inflection points. Furthermore, the adhesion "2" at the time of adhesion load release at time of load release is associated with the difference "X1" of the integrated values. The difference "X1" of the integrated values corresponds to the area of the triangle connecting the origin O, the point P22, and an inflection point P24. That is, the difference "X1" of the integrated values means that the graph of the load-displacement characteristic has the inflection point P24. Furthermore, the adhesion "1" at the time of adhesion load release at time of the load release is associated with the difference "X2" of the integrated values (note that X2 satisfies: X2 > X1) . The difference "X2" of the integrated values corresponds to the area of the triangle connecting the origin O, the point P22, and an inflection point P25. That is, the difference "X2" of the integrated values means that the graph of the load-displacement characteristic has the inflection point P25.

For example, when the operator has input the adhesion "2" at the time of adhesion load release at the time of load release, the generation unit 103 specifies the difference "X1" of the integrated values.

That is, when the hardness "2" and the adhesion "2" at the time of the adhesion load release at the time of load release have been input, the generation unit 103 specifies the physical property information that the point (position) on the graph moves from the origin O to the point P22 when the load is applied and the point moves from the point P22 to the origin via the inflection point P24.

The generation unit 103 then determines the shape parameters of the insole based on the physical property information (step S304).

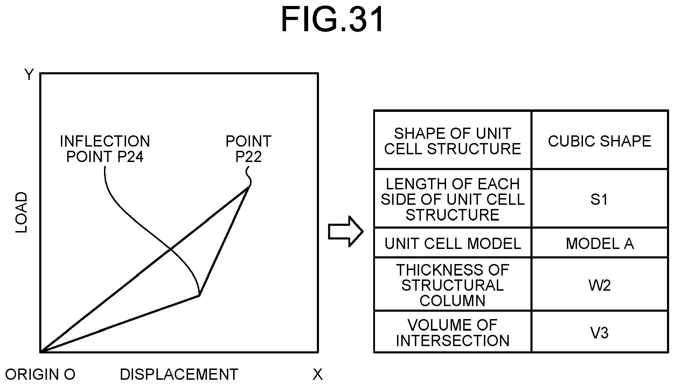

FIG. 31 exemplifies, on the left side of the figure, the physical property information specified in FIGS. 29 and 30. That is, the physical property information in FIG. 31 includes the slope in FIG. 29 and the difference between the integrated values in FIG. 30. Based on this physical property information, the generation unit 103 specifies the shape parameter as illustrated on the right side of FIG. 31.

For example, based on the outline of the graph of the load-displacement characteristic, the generation unit 103 determines the shape of the unit cell structure: "cubic shape", the length of each side of the unit cell structure: "S1", and the type of the unit cell model: "model A". In addition, the generation unit 103 determines the thickness: "W2" of the structural column based on the "slope" in the load-displacement characteristic. Subsequently, the generation unit 103 determines the volume of the intersection: "V3" based on the "difference of integrated values" in the load-displacement characteristic. In this manner, the generation unit 103 determines the shape parameters of the insole based on the physical property information.

In FIG. 31, the relationship between the outline of the load-displacement characteristic graph, and the combination of the shape of the unit cell structure, the length of each side of the unit cell structure, and the types of the unit cell model, is preferably defined in advance and stored in the storage unit 101. Furthermore, the relationship between the slope in the load-displacement characteristic and the thickness of the structural column is preferably defined in advance and stored in the storage unit 101. Furthermore, the relationship between the difference between the integrated values in the load-displacement characteristic and the volume of the intersection is preferably defined in advance and stored in the storage unit 101. Note that the method of determining the shape parameter based on the physical property information described in FIG. 31 is merely an example, and the determination can be made by any method.

As described above, the generation unit 103 according to the third embodiment can specify the physical property information based on the texture information and the degree information and can determine the shape parameter based on the specified physical property information. This enables detailed setting of the correspondence between the texture information and the shape parameter, leading to expectation that the orthosis will be designed using a material having more appropriate physical properties.

### (Fourth embodiment)

Furthermore, the information processing apparatus 10 can input identification information using a graph of load-displacement characteristic, for example.

That is, in the information processing apparatus 10 of the fourth embodiment, the reception unit 102 receives input related to the physical property information represented by the relationship between the change in the load and the change in the displacement amount as the identification information. Based on the physical property information received by the reception unit 102, the generation unit 103 determines the shape parameters that define the physical properties of an orthosis. The generation unit 103 generates model data including the determined shape parameters.

The information processing apparatus 10 of the fourth embodiment has a configuration similar to the information processing apparatus 10 illustrated in FIG. 13, with a difference in a part of the processes. Therefore, in the fourth embodiment, the points different from those in the second embodiment will be mainly described, and the points having functions similar to the configurations described in the second embodiment will be omitted.

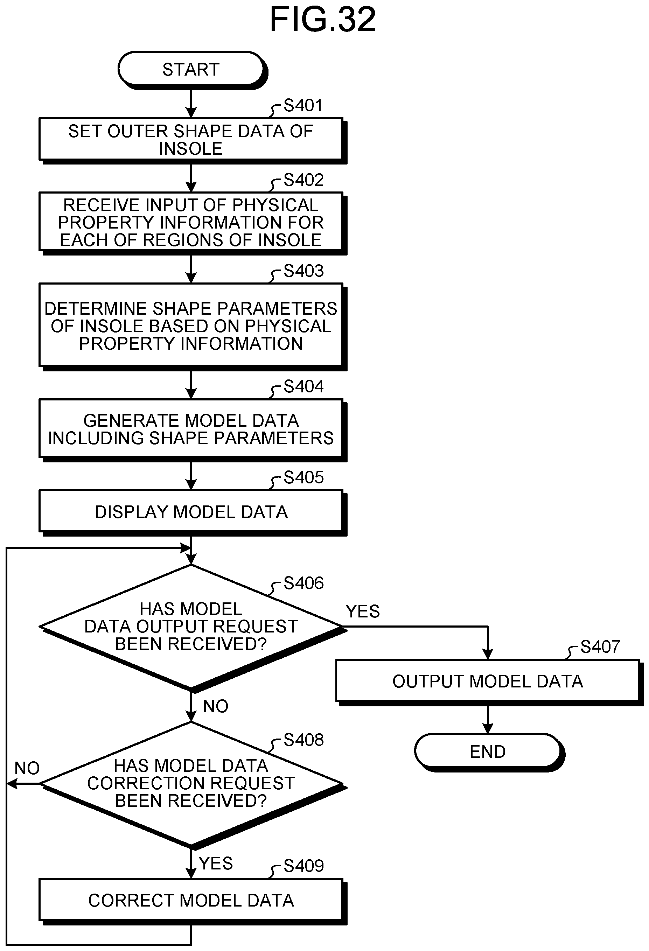

FIG. 32 is a flowchart illustrating an operation example of the information processing apparatus 10 according to the fourth embodiment. Of the processing procedures illustrated in FIG. 32, the process in step S401 is similar to the process in step S201 illustrated in FIG. 25, and thus the description thereof will be omitted. Since the processes of step S404 to step S409 are respectively similar to the processes of step S204 to step S209 illustrated in FIG. 25, the description thereof will be omitted.

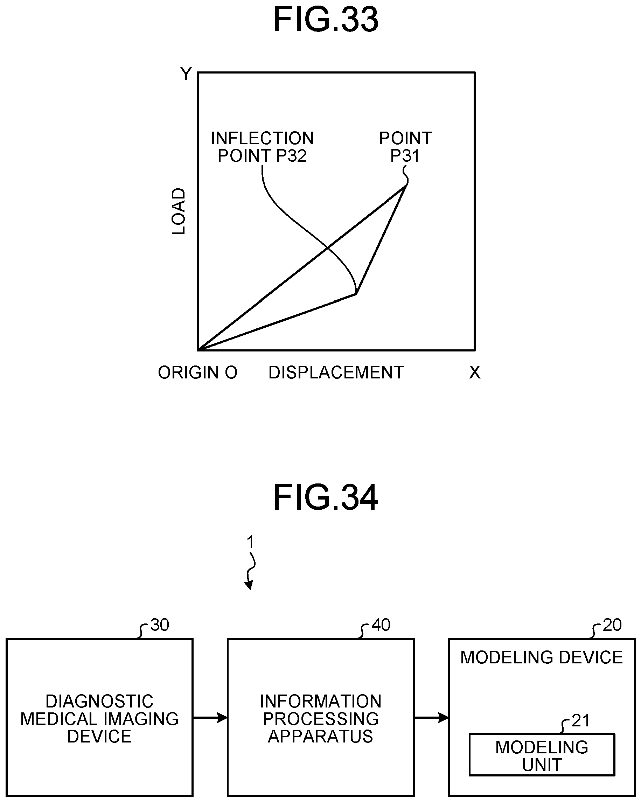

Furthermore, in the following description, the description will be given with reference to FIG. 33. FIG. 33 is a diagram illustrating processes of the generation unit 103 of the fourth embodiment. In the graph of load-displacement characteristic of FIG. 33, the displacement corresponds to the X direction and the load corresponds to the Y direction.

As illustrated in FIG. 32, the reception unit 102 receives input of physical property information for each of regions of the insole (step S402).

As illustrated in FIG. 33, the reception unit 102 receives inputs of "slope" and "difference of integrated values" on the graph of load-displacement characteristic. For example, the operator designates the "slope" in the load-displacement characteristic by moving the position of a point P31 to a certain position on the graph of the load-displacement characteristic. For example, in order to set a hard region, the operator moves the position of the point P31 upward (or to the left). In contrast, in order to set a soft region, the operator moves the position of the point P31 downward (or to the right).

Furthermore, for example, the operator designates the "difference of integrated values" in the load-displacement characteristic by moving the position of an inflection point P32 to a certain position on the graph of the load-displacement characteristic. For example, in order to set a region with a high fittability, the operator moves the position of the inflection point P32 upward (or to the left). By contrast, in order to set a region with a low fittability, the operator moves the position of the point P31 downward (or to the right).

In this manner, the reception unit 102 receives the "slope" of the line connecting the origin O and the point P31. Furthermore, the reception unit 102 receives the area of the triangle connecting the origin O, the point P31, and the inflection point P32, as the "difference of integrated values".

Then, the generation unit 103 determines the shape parameter of the insole based on the physical property information (step S403). Since the process of step S403 is similar to the process of step S304 illustrated in FIG. 32, the description thereof will be omitted.

As described above, the reception unit 102 of the fourth embodiment can receive the input of the identification information by using the graph of the load-displacement characteristic. This enables the operator to intuitively input the physical property information, leading to expectation that the orthosis will be designed using the material having more appropriate physical properties.

### (Fifth embodiment)

Furthermore, for example, when the image data of a part of the body of the subject (the site where the orthosis is worn) can be captured by a diagnostic medical imaging device, the information obtained from the image data can be reflected onto the model data.

FIG. 34 is a diagram illustrating an example of a schematic configuration of a system 1 of a fifth embodiment. As illustrated in FIG. 34, the system 1 of the fifth embodiment includes a diagnostic medical imaging device 30, an information processing apparatus 40, and a modeling device 20. The devices illustrated in FIG. 34 are directly or indirectly communicable with each other by a network such as a local area network (LAN) or a wide area network (WAN). Since the configuration of the modeling device 20 illustrated in FIG. 34 is similar to the configuration of the modeling device 20 illustrated in FIG. 11, the description thereof will be omitted.

The diagnostic medical imaging device 30 is a device that atraumatically generates image data that images a portion that is normally invisible. Specifically, the diagnostic medical imaging device 30 illustrated in FIG. 34 is a device capable of generating three-dimensional image data (volume data) in which at least the contour of the body surface at an imaging site of the subject and the bone existing within the imaging site are depicted. For example, the diagnostic medical imaging device 30 is an X-ray computed tomography (CT) device, a magnetic resonance imaging (MRI) device, or the like. Hereinafter, imaging performed by an X-ray CT device, which is an example of the diagnostic medical imaging device 10, will be briefly described.

The X-ray CT device performs imaging using a gantry device having a rotating frame that is rotatable while supporting an X-ray tube that emits X-rays and an X-ray detector that detects X-rays that have been transmitted through the subject at positions to face each other. By rotating the rotating frame while emitting X-rays from the X-ray tube, the X-ray CT device collects projection data and reconstructs X-ray CT image data from the projection data. For example, the X-ray CT image data is a tomographic image (two-dimensional X-ray CT image data) on a rotating surface (axial surface) of the X-ray tube and the X-ray detector.

The X-ray detector includes a plurality of rows of detection elements, which are X-ray detection elements arranged in the channel direction, being arranged in a rotation axis direction of the rotating frame. For example, an X-ray CT device having an X-ray detector including 16 rows of detection elements reconstructs a plurality of (for example, 16) tomographic images in a body axis direction of a subject from the projection data collected by one rotation of a rotating frame. In addition, the X-ray CT device uses a helical scan that moves the subject or gantry device while rotating the rotating frame so as to enable reconstruction of, for example, 500 tomographic images covering the entire heart as three-dimensional X-ray CT image data.

Here, the X-ray CT device as the diagnostic medical imaging device 30 illustrated in FIG. 34 is a device capable of imaging a subject in a standing, sitting, or lying postures. Such an X-ray CT device, for example, images a subject sitting on a chair formed of a material having high X-ray transparency to generate three-dimensional X-ray CT image data.

Incidentally, an MRI device can reconstruct an MRI image of any one cross section or MRI images (volume data) of a plurality of certain cross sections from MR signals collected by changing the phase-encoding gradient magnetic field, the slice selection gradient magnetic field, and the frequency- encoding gradient magnetic field.

In the fifth embodiment, the diagnostic medical imaging device 30 generates three-dimensional X-ray CT image data in which the region including the foot of the subject is photographed as image data. The diagnostic medical imaging device 30 then transmits the generated image data to the information processing apparatus 40.

Specifically, the diagnostic medical imaging device 30 converts the image data into digital imaging and communications in medicine (DICOM) data in a format conforming to the DICOM standard, and transmits the converted data to the information processing apparatus 40. The diagnostic medical imaging device 30 creates DICOM data in which incidental information has been added to the image data. The incidental information includes patient ID that enables unique identification of the subject, patient information (name, gender, age, etc.), the type of examination in which the image was taken, the examination site (imaging site), the patient's posture at the time of imaging, information regarding image size, and the like. The information regarding the image size is used as information for converting the length in the image space into the length in the real space. The fifth embodiment is also applicable in cases where the diagnostic medical imaging device 30 transmits the three-dimensional X-ray CT image data obtained by imaging including the foot or feet of the subject in the standing, sitting, or lying posture, as image data, to the information processing apparatus 20.

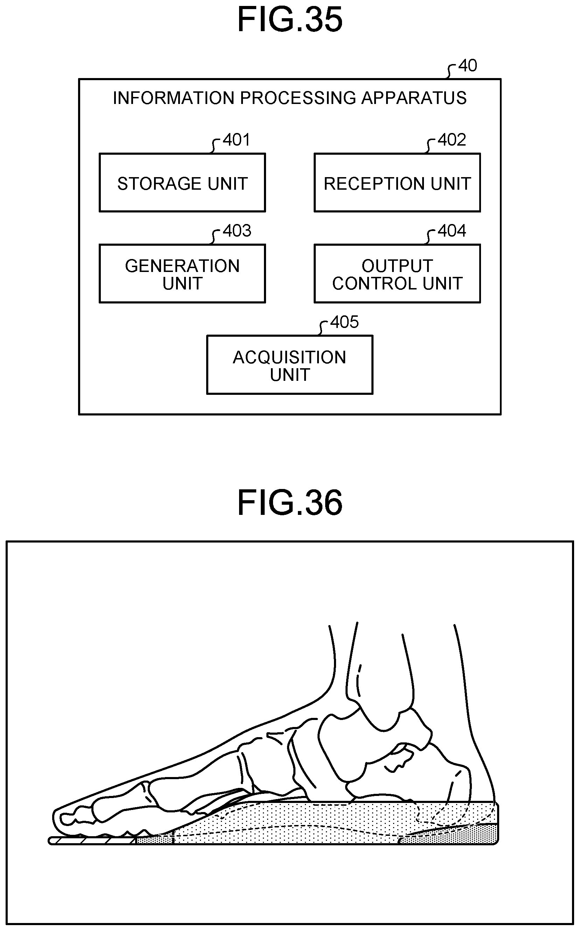

FIG. 35 is a diagram illustrating an example of a function of the information processing apparatus 40 of the fifth embodiment. As illustrated in FIG. 35, the information processing apparatus 40 includes a storage unit 401, a reception unit 402, a generation unit 403, an output control unit 404, and an acquisition unit 405. The information processing apparatus 40 has a hardware configuration similar to that of the information processing apparatus 10 illustrated in FIG. 12. The storage unit 401, the reception unit 402, the generation unit 403, and the output control unit 404 illustrated in FIG. 35 respectively have functions similar to the storage unit 101, the reception unit 102, the generation unit 103, and the output control unit 104 illustrated in FIG. 11, and thus, the description thereof will be omitted.

The acquisition unit 405 acquires site information including outer shape data of at least a part of the body of the subject based on image data. For example, the acquisition unit 405 transmits a transmission request for image data corresponding to the patient ID to the diagnostic medical imaging device 30 via the external I/F 17. The external I/F 17 stores the image data received from the diagnostic medical imaging device 30 in the storage unit 401. The acquisition unit 405 acquires the image data from the storage unit 401.

Then, the acquisition unit 405 acquires the site information including the outer shape data of the foot of the subject based on the image data. For example, the acquisition unit 405 performs known image processing such as edge detection processing on the three-dimensional X-ray CT image data to extract the contour (body surface) of the foot. With this operation, the acquisition unit 203 acquires, from the image data, outer shape data representing the surface shape of the foot of the subject in three dimensions. The acquisition unit 405 transmits the acquired site information to the generation unit 403.

The generation unit 403 generates model data based on the site information. For example, the generation unit 403 generates model data based on the site information acquired by the acquisition unit 405. For example, the generation unit 403 adjusts the outer shape data of the insole according to the outer shape data of the foot. The generation unit 403 generates model data including the outer shape data of the adjusted insole.

Furthermore, the output control unit 404 controls to simultaneously display the model data and the site information. The reception unit 402 receives an operation of correcting the model data on an image in which the model data and the site information are simultaneously displayed. Subsequently, the generation unit 403 corrects the model data in accordance with the operation received by the reception unit 402.

FIG. 36 is a diagram illustrating a correction process of model data according to the fifth embodiment.

As illustrated in FIG. 36, the output control unit 404 controls the display device 16 to display the outer shape data of the insole included in the model data together with the outer shape data of the foot included in the site information. Here, the output control unit 404 displays the position of the outer shape data of the insole and the position of the outer shape data of the foot in association with each other.

The reception unit 402 receives an operation of correcting the model data on the display screen of FIG. 36. Subsequently, the generation unit 403 corrects the model data in accordance with the operation received by the reception unit 402. With this procedure, the operator can correct the outer shape data of the insole while confirming the outer shape data of the actual foot on the screen.

(Other embodiments related to second to fifth embodiments)

In addition to the above-described embodiments, various types of different embodiments may be implemented.

### (Orthosis manufacturing method)

The modeling device 20 of the embodiment can manufacture an orthosis by the following manufacturing method.

For example, the modeling device 20 can model (mold) an orthosis by using various additive manufacturing technologies (three-dimensional modeling technology) such as a material extrusion method, a vat photopolymerization method, and a selective laser sintering method.

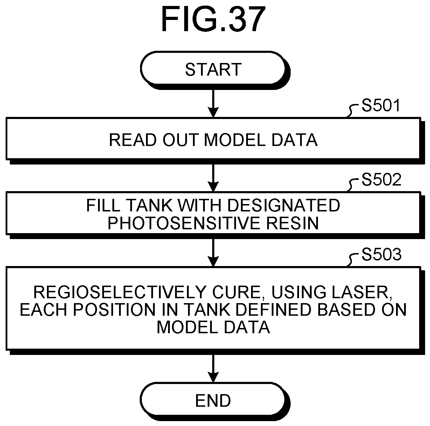

FIG. 37 is a flowchart illustrating an example of a manufacturing method of an embodiment. The manufacturing method illustrated in FIG. 37 is executed by the modeling device 20 capable of executing a three-dimensional modeling technology by a vat photopolymerization method. The modeling device 20 (modeling unit 21) includes a vat (tank) for filling the photosensitive resin, which is the material of the orthosis, and a laser for regioselectively curing the photosensitive resin in the vat by a photopolymerization reaction. Note that known modeling devices can be flexibly applied as the modeling device 20.

As illustrated in FIG. 37, the modeling unit 21 reads out model data (step S501). This model data is information that forms the basis for modeling with the modeling device 20. The model data is created in advance by a manufacturer, for example, and preliminarily stored in a storage device included in the modeling device. The storage device corresponds to, for example, a hard disc drive (HDD), a solid state drive (SSD), or the like.

Subsequently, the modeling unit 21 fills the tank with designated photosensitive resin (step S502). Here, the photosensitive resin may be designated in advance by the model data, or may be designated by an operator each time the modeling method is executed.

Subsequently, by using a laser, the modeling unit 21 regioselectively cures individual positions in the tank defined based on the model data (step S503). For example, the modeling unit 21 sequentially emits laser toward the positions in the tank corresponding to the positions where the structural columns exist in the model data. That is, the modeling unit 21 sequentially cures the positions in the tank corresponding to the unit cell structure of each of regions of the orthosis. With this procedure, the modeling unit 21 can manufacture an orthosis having appropriate physical properties.

The material of the orthosis can be any material as long as it is a material that can be used for modeling technology. For example, any photosensitive resin that can be modeled by additive manufacturing technology can be appropriately selected.

### (Orthosis manufacturing method using system 1)

As described above, the system 1 of the embodiment includes the information processing apparatus 10 and the modeling device 20. That is, the system 1 can manufacture the orthosis by the functions of the information processing apparatus 10 and the modeling device 20.

That is, in the system 1, the information processing apparatus 10 receives input of the identification information that identifies the sensation given to the user of the orthosis. The information processing apparatus 10 generates model data for designing the orthosis based on the received identification information. In the system 1, the modeling device 20 models the orthosis based on the model data.

For example, in the system 1, the information processing apparatus 10 generates model data by the processes of steps S201 to S209 illustrated in FIG. 25.

The information processing apparatus 10 transmits the model data to the modeling device 20. For example, the output control unit 104 of the information processing apparatus 10 converts the model data into a data format that can be used by the modeling device 20, and transmits the converted model data to the modeling device 20.

Subsequently, in the system 1, the modeling device 20 models the orthosis based on the model data received from the information processing apparatus 10 by using the processes of steps S501 to S503 illustrated in FIG. 37.

According to this procedure, the system 1 can manufacture an orthosis having appropriate physical properties. The individual processing units included in the information processing apparatus 10 and the modeling device 20 may be provided in any of the devices. For example, when the modeling device 20 includes the generation unit 103, the modeling device 20 may generate model data based on the identification information. In this case, the output control unit 104 of the information processing apparatus 10 will transmit the identification information received by the reception unit 102 to the modeling device 20.

### (Load direction)

Although the above embodiment is a case where the load direction corresponds to the vertical downward direction in the drawing as illustrated in FIG. 23, for example, the embodiment is not limited to this. For example, the direction of the unit cell structure in the orthosis can be appropriately set by the manufacturer. For example, in the case of a hand-worn orthosis, the normal direction at the contact point between the hand and the orthosis may be defined as the load direction. In this case, the unit cell structure of FIG. 18 is arranged in the orthosis so that the vertical downward direction in the drawing corresponds to the normal direction at the contact point with the human body.

According to the embodiment described above, it is possible to provide an information processing apparatus, a system, an information processing method, and a program capable of designing an orthosis using a material having appropriate physical properties.

The above-described embodiments can be combined with the above modifications in any manner, and the above modifications may be combined in any manner.

The programs executed by the information processing apparatuses 10 or 40 of the above-described embodiments may be recorded as files in an installable format or an executable format in computer readable recording medium such as a CD-ROM, flexible disk (FD), CD-R, DVD, or universal serial bus (USB) and provided. Alternatively, the programs may be provided or distributed via a network such as the Internet. Furthermore, various programs may be provided by being incorporated in advance in a non-volatile storage medium such as ROM.

### (Sixth embodiment)

The conventional techniques have had problems in design of orthosis which takes time and cost, and in varying quality of orthosis. For example, the conventional insole manufacturing involves many operation steps manually performed by a prosthetist, resulting in increased time and cost to design the orthosis and varying quality among the prosthetists.

A sixth embodiment aims to provide an information processing apparatus, a system, an orthosis manufacturing method, an information processing method, and a program capable of achieving facilitated designing and stable quality of an orthosis.

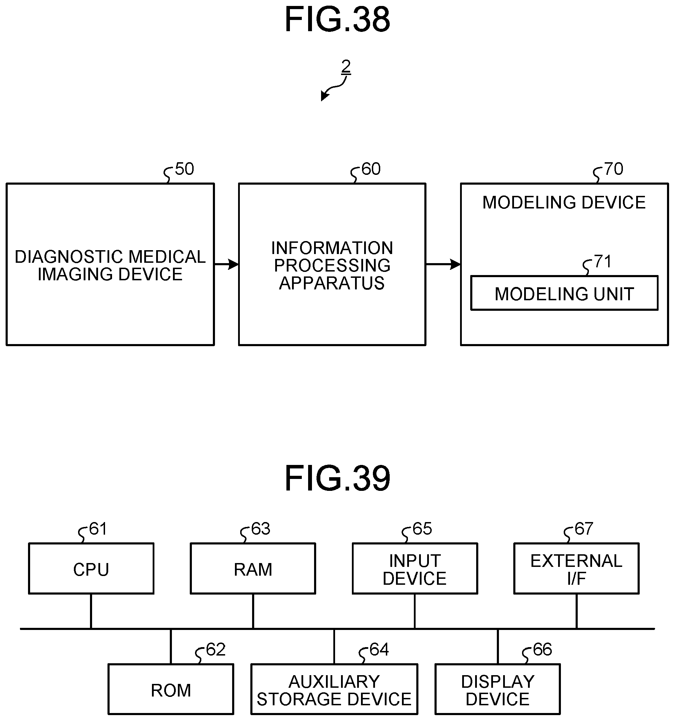

FIG. 38 is a diagram illustrating an example of a schematic configuration of a system 2 of the sixth embodiment. As illustrated in FIG. 38, the system 2 of the sixth embodiment includes a diagnostic medical imaging device 50, an information processing apparatus 60, and a modeling device 70. The devices illustrated in FIG. 38 are directly or indirectly communicable with each other by a network such as a local area network (LAN) or a wide area network (WAN).

The diagnostic medical imaging device 50 is a device that atraumatically generates image data that images a portion that is normally invisible. Specifically, the diagnostic medical imaging device 50 illustrated in FIG. 38 is a device capable of generating three-dimensional image data (volume data) in which at least the contour of the body surface at an imaging site of the subject and the bone existing within the imaging site are depicted. For example, the diagnostic medical imaging device 50 is an X-ray computed tomography (CT) device, a magnetic resonance imaging (MRI) device, or the like. Hereinafter, imaging performed by an X-ray CT device, which is an example of the diagnostic medical imaging device 50, will be briefly described.

The X-ray CT device performs imaging using a gantry device having a rotating frame that is rotatable while supporting an X-ray tube that emits X-rays and an X-ray detector that detects X-rays that have been transmitted through the subject at positions to face each other. By rotating the rotating frame while emitting X-rays from the X-ray tube, the X-ray CT device collects projection data and reconstructs X-ray CT image data from the projection data. For example, the X-ray CT image data is a tomographic image (two-dimensional X-ray CT image data) on a rotating surface (axial surface) of the X-ray tube and the X-ray detector.

The X-ray detector includes a plurality of rows of detection elements, which are X-ray detection elements arranged in the channel direction, being arranged in a rotation axis direction of the rotating frame. For example, an X-ray CT device having an X-ray detector including 16 rows of detection elements reconstructs a plurality of (for example, 16) tomographic images in a body axis direction of a subject from the projection data collected by one rotation of a rotating frame. In addition, the X-ray CT device uses a helical scan that moves the subject or gantry device while rotating the rotating frame so as to enable reconstruction of, for example, 500 tomographic images covering the entire heart as three-dimensional X-ray CT image data.

Here, the X-ray CT device as the diagnostic medical imaging device 50 illustrated in FIG. 38 is a device capable of imaging a subject in a standing, sitting, or lying postures. Such an X-ray CT device, for example, images a subject sitting on a chair formed of a material having high X-ray transparency to generate three-dimensional X-ray CT image data.

Incidentally, an MRI device can reconstruct an MRI image of any one cross section or MRI images (volume data) of a plurality of certain cross sections from MR signals collected by changing the phase-encoding gradient magnetic field, the slice selection gradient magnetic field, and the frequency- encoding gradient magnetic field.

In the present embodiment, the diagnostic medical imaging device 50 generates three-dimensional X-ray CT image data in which the region including the foot of the subject is photographed as image data. The diagnostic medical imaging device 50 then transmits the generated image data to the information processing apparatus 60.

Specifically, the diagnostic medical imaging device 50 converts the image data into digital imaging and communications in medicine (DICOM) data in a format conforming to the DICOM standard, and transmits the DICOM data to the information processing apparatus 60. The diagnostic medical imaging device 50 creates DICOM data in which incidental information has been added to the image data. The incidental information includes patient ID that enables unique identification of the subject, patient information (name, gender, age, etc.), the type of examination in which the image was taken, the examination site (imaging site), the patient's posture at the time of imaging, information regarding image size, and the like. The information regarding the image size is used as information for converting the length in the image space into the length in the real space. The present embodiment is also applicable in cases where the diagnostic medical imaging device 50 transmits the three-dimensional X-ray CT image data obtained by imaging including the foot or feet of the subject in the standing, sitting, or lying posture, as image data, to the information processing apparatus 60.

The information processing apparatus 60 acquires image data of a subject (wearer) who wears the orthosis from the diagnostic medical imaging device 50. For example, an operator of the information processing apparatus 60 (a prosthetist, or the like) performs a search using the patient ID of the subject. With this operation, the information processing apparatus 60 acquires three-dimensional X-ray CT image data including the foot of the subject from the diagnostic medical imaging device 50.

The information processing apparatus 60 uses various types of information acquired from the image data of the subject to generate model data for designing the orthosis to be worn by the subject. The model data is information that is the basis for modeling an orthosis worn by a subject (orthosis wearer) with the modeling device 70. The processing details for generating model data will be described below. The information processing apparatus 60 transmits the generated model data to the modeling device 70.

In the following embodiment, a medical insole will be described as an example of the orthosis. However, the embodiment is not limited to the insole but is widely applicable to orthoses to be applied to individual sites of a human body, such as a hand, elbow, knee, shoulder, or head. Furthermore, the embodiment is not limited to medical orthoses, and is widely applicable to an orthosis used for assisting or protecting physical functions in sports and daily life, for example. The insole is also referred to as an internal fitting, an inner sole, or the like. In addition, the insoles according to the following embodiments correspond to the insoles 100 described in the first embodiment.

The modeling device 70 includes a modeling unit 71 that models an orthosis to be worn by the subject based on model data received from the information processing apparatus 60. In the present embodiment, the modeling unit 71 is constituted with a group of hardware elements that provide the functions of a three-dimensional printer.

For example, the modeling unit 71 includes: a liquid layer (tank) for filling the photosensitive resin that is the material of the insole; and a laser for curing the photosensitive resin in the liquid layer by a photopolymerization reaction. By using a laser, the modeling unit 71 regioselectively cures the position in the liquid layer defined based on the model data and thereby models a three-dimensional structure corresponding to the model data.

Next, the information processing apparatus 60 of the present embodiment will be described. FIG. 39 is a diagram illustrating an example of a hardware configuration of the information processing apparatus 60 of the sixth embodiment. As illustrated in FIG. 39, the information processing apparatus 60 includes a central processing unit (CPU) 61, read only memory (ROM) 62, random access memory (RAM) 63, an auxiliary storage device 64, an input device 65, a display device 66, and an external interface (I/F) 67.

The CPU 61 is a processor (processing circuit) that comprehensively controls the operation of the information processing apparatus 60 by executing a program and implements various functions of the information processing apparatus 60. Various functions of the information processing apparatus 60 will be described below.

The ROM 62 is non-volatile memory and stores various data (information written at the manufacturing stage of the information processing apparatus 60) including a program for starting the information processing apparatus 60. The RAM 63 is volatile memory having a working region of CPU 61. The auxiliary storage device 64 stores various data such as a program executed by the CPU 61. The auxiliary storage device 64 is constituted with a hard disc drive (HDD), a solid state drive (SSD), or the like.

The input device 65 is a device for an operator who uses the information processing apparatus 60 to perform various operations. Examples of the input device 65 are a mouse, a keyboard, a touch panel, or hardware keys. In addition, the operator corresponds to, for example, a prosthetist who creates an orthosis, a medical person such as a doctor or a physiotherapist, and a subject who wears the orthosis.

The display device 66 displays various types of information. For example, the display device 66 displays image data, model data, a graphical user interface (GUI) for receiving various operations from an operator, a medical image, or the like. Examples of the display device 66 are a liquid crystal display, an organic electro luminescence (EL) display, or a cathode ray tube display. The input device 65 and the display device 66 may be integrated in the form of a touch panel, for example.

The external I/F 67 is an interface for connecting (communication) with an external device such as the diagnostic medical imaging device 50 or the modeling device 70.

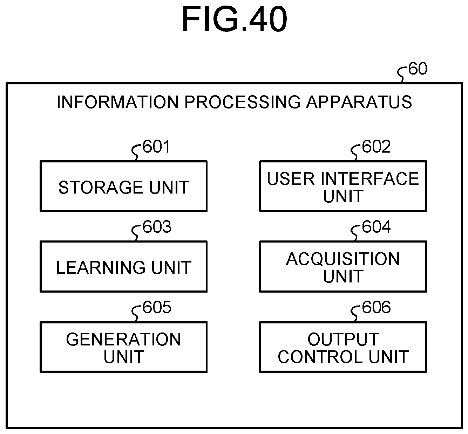

FIG. 40 is a diagram illustrating an example of a function of the information processing apparatus 60 of the sixth embodiment. In the example of FIG. 40, only the functions related to the present embodiment are illustrated, but the functions of the information processing apparatus 60 are not limited to these. As illustrated in FIG. 40, the information processing apparatus 60 includes a storage unit 601, a user interface unit 602, a learning unit 603, an acquisition unit 604, a generation unit 605, and an output control unit 606. The function of the storage unit 601 is implemented by the auxiliary storage device 64 (for example, HDD) illustrated in FIG. 39, for example. The function of the user interface unit 602 is implemented by the input device 65 and the display device 66, for example. Individual functions of the learning unit 603, the acquisition unit 604, the generation unit 605, and the output control unit 606 are implemented by the CPU 61, for example.

Here, the information processing apparatus 60 of the sixth embodiment includes a trained model, making it possible to achieve facilitated designing and stable quality of the orthosis by using the trained model. While the following embodiment describes a case where the information processing apparatus 60 generates a trained model, the present invention is not limited to this. For example, the information processing apparatus 60 can achieve facilitated designing and stable quality of the orthosis by using a trained model generated by an information processing apparatus different from the information processing apparatus 60.

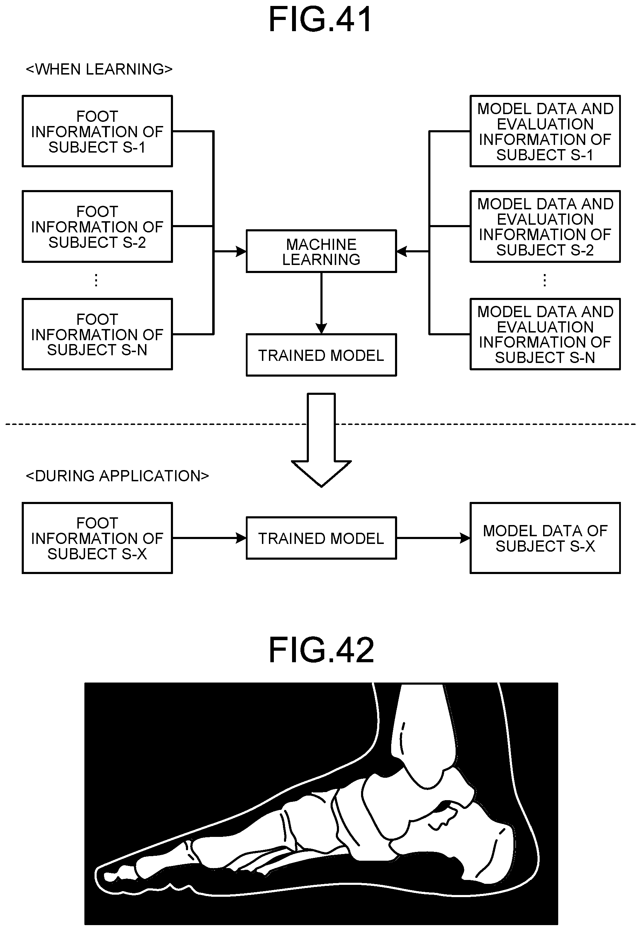

FIG. 41 is a diagram illustrating processing during learning and application performed by the information processing apparatus 60 of the sixth embodiment. As illustrated in the upper part of FIG. 41, at the time of learning, the information processing apparatus 60 performs machine learning using individual foot information, model data, and evaluation information regarding subject S-1, subject S-2,... and subject S-N (hereinafter, abbreviated as "subjects S-1 to S-N"). Here, the foot information of individual subjects S-1 to S-N is information including the outer shape data of the foot of each of the individual subjects S-1 to S-N. In addition, the model data of individual subjects S-1 to S-N is information for designing an insole to be worn on the foot of the individual subjects S-1 to S-N. Furthermore, the evaluation information of the individual subjects S-1 to S-N is information related to evaluation of the insole of the individual subjects S-1 to S-N. By performing machine learning using foot information, model data, and evaluation information of the individual subjects S-1 to S-N, the information processing apparatus 60 builds a trained model capable of outputting model data based on input foot information. This trained model is stored in the storage unit 601 of the information processing apparatus 60, for example.

As illustrated in the lower part of FIG. 41, during application, the information processing apparatus 60 inputs foot information of a subject S-X, which is a target for creating the insole, to the trained model so as to output model data of the subject S-X from the trained model. Subsequently, the information processing apparatus 60 presents the model data of the subject S-X output from the trained model to an operator (designer of the insole). This enables the information processing apparatus 60 to achieve facilitated designing and stable quality of an orthosis.

Although FIG. 41 illustrates a trained model capable of outputting model data based on input of foot information, the embodiment is not limited to this. For example, the information processing apparatus 60 performs machine learning using information including outer shape data of a hand (hand information) and model data for designing a hand orthosis to be worn on the hand. With this configuration, the information processing apparatus 60 can build a trained model capable of outputting model data for designing a hand orthosis based on input of hand information. That is, the information processing apparatus 60 performs machine learning using site information including the outer shape data of a site of the subject and the model data for designing the orthosis to be worn on the site. With this configuration, the information processing apparatus 60 can build a trained model capable of outputting model data of the orthosis worn on the site based on input of the site information.

Returning to the description of FIG. 40, individual functions of the storage unit 601, the user interface unit 602, the learning unit 603, the acquisition unit 604, the generation unit 605, and the output control unit 606 of the information processing apparatus 60 will be described in detail.

The storage unit 601 stores various types of information used for machine learning, a machine learning program for performing machine learning, or the like. In addition, the storage unit 601 stores the trained model built by machine learning. In addition, the storage unit 601 can also store image data (DICOM data) captured by a diagnostic medical imaging device.

The user interface unit 602 has a function of receiving input from the operator and a function of outputting various types of information. For example, the user interface unit 602 receives an input operation performed by the operator via the input device 15, converts the received input operation into an electric signal, and transmits the signal to individual units in the information processing apparatus 60. Furthermore, the user interface unit 602 receives various types of information from individual units in the information processing apparatus 60, stores the received information in the storage unit 101, or displays the received information on the display device 16. Furthermore, the user interface unit 602 transmits the information designated by the operator to an external device via the external I/F 67.

The learning unit 603 generates, for the sites of the plurality of subjects S-1 to S-N, a trained model by performing machine learning using site information including outer shape data of the sites of the individual subjects S-1 to S-N, model data for designing the orthosis to be worn on the sites of the individual subjects S-1 to S-N, and the evaluation information regarding evaluation of the orthosis.

For example, when the user interface unit 602 has received a request for generating the trained model, the generation request is transmitted to the learning unit 603. Having received the request for generating the trained model, the learning unit 603 reads out the foot information of the individual subjects S-1 to S-N, the model data of the insole of individual subjects S-1 to S-N, and the evaluation information of the insole of the individual subjects S-1 to S-N from a storage circuit 204. The learning unit 603 then performs machine learning by using the read information, namely, the foot information of the individual subjects S-1 to S-N, the model data of the insole of the individual subjects S-1 to S-N, and the evaluation information of the insole of the individual subjects S-1 to S-N.

Hereinafter, "foot information", "model data", and "evaluation information" used for machine learning will be described in order.

First, "foot information" will be described. The foot information is information including outer shape data of the foot of each of the individual subjects S-1 to S-N. For example, the outer shape data of the foot is acquired from the image data obtained by imaging the foot of each of the individual subjects S-1 to S-N.

FIG. 42 is a diagram illustrating an example of foot information of the sixth embodiment. FIG. 42 is an example of the foot information of the subject S-1. As illustrated in FIG. 42, the foot information of the subject S-1 includes outer shape data of the foot. Here, the outer shape data of the foot is information representing the three-dimensional surface shape of the foot of the subject S-1. The outer shape data of the foot is obtained from the three-dimensional X-ray CT image data in which the foot of the subject S-1 is imaged, for example. Since the process for acquiring the outer shape data of the foot is similar to the process in the acquisition unit 604 described below, the description thereof is omitted here.

In this manner, the foot information of the individual subjects S-1 to S-N includes the outer shape data of the foot, for example. The foot information of the individual subjects S-1 to S-N is acquired from the image data of the individual subjects S-1 to S-N by the information processing apparatus 60 or another information processing apparatus different from the information processing apparatus 60, and is preliminarily stored in the storage unit 601. Although FIG. 42 describes a case where the site information includes the outer shape data of the site, the present invention is not limited to this. For example, the site information can include various types of feature information acquirable from the X-ray CT image data in addition to the outer shape data.

Next, "model data" will be described. The model data is information for designing an insole to be worn on the foot of individual subjects S-1 to S-N, information that is a basis for modeling the insole with the modeling device 70. The model data is information including outer shape data of the insole and shape parameters that define the physical properties of the insole, for example.

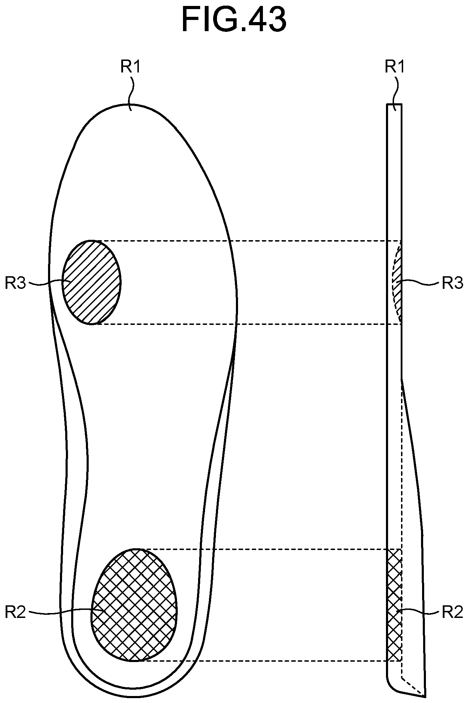

FIG. 43 is a diagram illustrating an example of outer shape data of an insole of the sixth embodiment. The left figure of FIG. 43 illustrates a plan view of an insole for the right foot as viewed from a contact surface side in direct or indirect contact with individual sites of a human body. The right figure in FIG. 43 illustrates a side view of the left figure. The toe side of the insole is illustrated on the upper side of FIG. 43, while the heel side of the insole is illustrated on the lower side of FIG. 43.

As illustrated in FIG. 43, for example, the outer shape data of the insole includes the information of the region R1, the region R2, and the region R3. Of these, the region R1 is a region that constitutes the overall outer shape of the insole. The region R2 is a region where the site near the calcaneus (heel bone) touches. The region R3 is a region coming in contact with the site near the metatarsal joint of the big toe.

Here, the outer shape data of the insole includes information regarding the shape of the region, the size of the region, the thickness of the region, and the position of the region for each of the regions R1, R2, and R3. Furthermore, the outer shape data of the insole includes information indicating the position of the region R2 with respect to the region R1 and information indicating the position of the region R3 with respect to the region R1.

The shape parameter of the insole includes at least one of the number of a plurality of structural columns constituting the unit cell structure, a direction of the structural columns, a thickness of the structural columns, a volume of intersection of the structural columns, a shape of the unit cell structure, or size of the unit cell structure.

Here, the unit cell structure is a structural unit that constitutes each of regions of the insole, having a space of a polygonal prism shape as one unit. The unit cell structure is constituted with a plurality of structural columns, and various physical properties can be achieved by setting various shape parameters.

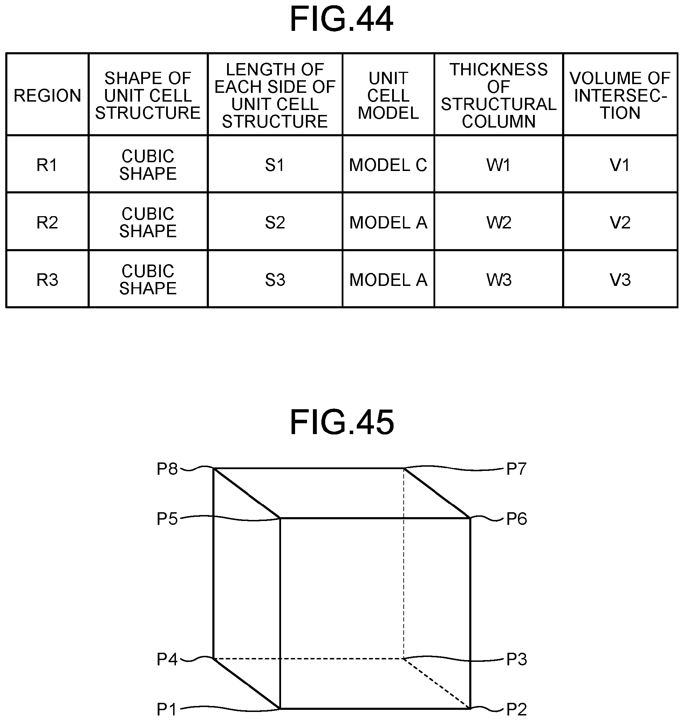

FIG. 44 is a diagram illustrating an example of the shape parameters of the insole of the sixth embodiment. As illustrated in FIG. 44, for example, shape parameters are associated with each other for each of regions of the insole. In FIG. 44, "shape of unit cell structure", "length of each side of unit cell structure", "unit cell model", "thickness of structural column", and "volume of intersection" are an example of shape parameters.

As illustrated in an example of FIG. 44, for example, the parameters defined in the region R1 are the shape of the unit cell structure: "cubic shape", the length of each side of the unit cell structure: "S1", the unit cell model: "model C", the thickness of the structural columns: "W1", and the volume of the intersection: "V1". This indicates that the region R1 is constituted with the unit cell model of model C, the thickness of its structural columns is "W1", and the volume of its intersection is "V1".

Moreover, the parameters defined in the region R2 are the shape of the unit cell structure: "cubic shape", the length of each side of the unit cell structure: "S2", the unit cell model: "model A", the thickness of the structural columns: "W2", and the volume of the intersection: "V2". This indicates that the region R2 is constituted with the unit cell model of model A, the thickness of its structural columns is "W2", and the volume of its intersection is "V2".

Moreover, the parameters defined in the region R3 are the shape of the unit cell structure: "cubic shape", the length of each side of the unit cell structure: "S3", the unit cell model: "model A", the thickness of the structural columns: "W3", and the volume of the intersection: "V3". This indicates that the region R3 is constituted with the unit cell model of model A, the thickness of its structural columns is "W3", and the volume of its intersection is "V3".

In this manner, each of the regions R1 to R3 has a difference in at least one shape parameter, out of the shape of the unit cell structure, the length of each side of the unit cell structure, the number of structural columns, the direction of the structural columns, the thickness of the structural columns, or the volume of the intersection. This gives each of the regions R1 to R3 mutually different physical properties. Furthermore, it is preferable that the unit cell structure of the region R1, the unit cell structure of the region R2, and the unit cell structure of the region R3 define a space having the identical shape and the identical size (here, the length of each side), as one unit.

Hereinafter, various shape parameters, namely, the shape of the unit cell structure, the length of each side of the unit cell structure, the model, the thickness of the structural columns, and the volume of the intersection, will be described with reference to FIGS. 45 to 51.

First, the "shape of the unit cell structure" and the "length of each side of the unit cell structure" in FIG. 44 will be described. The "shape of the unit cell structure" is the shape of the space defined as one unit of the unit cell structure. The "length of each side of the unit cell structure" corresponds to the size of the space defined as one unit of the unit cell structure.

FIG. 45 is a diagram illustrating the shape of the unit cell structure and the length of each side of the unit cell structure according to the sixth embodiment. As illustrated in FIG. 45, the "shape of the unit cell structure" corresponds to a cubic shape formed with six square faces. This cubic space has eight points P1 to P8 corresponding to the vertices. Furthermore, in the example of FIG. 45, since this space has a cubic shape, the lengths of all sides are equal. The length of each side of the space in FIG. 45 is expressed by a distance between the points P1 and P2, for example.

Here, the notation method of lines and faces in the unit cell structure will be described. In the present embodiment, when a line or a face is described, the vertices constituting the line or the face are described in parentheses. For example, the notation "line (P1, P2)" represents a line connecting the point P1 and the point P2. The notation "line (P1, P7)" represents a line (diagonal) connecting the point P1 and the point P7. Furthermore, the notation "face (P1, P2, P3, P4)" represents a face (bottom face) having points P1, P2, P3, and P4 as vertices. The structural columns are described similarly to the line notation method.

The unit cell structure according to the sixth embodiment includes a plurality of structural columns connecting two points out of the plurality of points P1 to P8. That is, the spatial shape of FIG. 45 corresponds to the shape of the unit cell structure. The number and direction (arrangement direction) of the structural columns are defined in advance by a unit cell model, for example.

The contents illustrated in FIG. 45 are merely an example, and the present invention is not limited to the contents in the drawing. For example, the cubic space illustrated in FIG. 45 is just an example, and the spatial shape in which the unit cell structure is one unit may be a polygonal prism shape. In this case, it is preferable that the unit cell structure has a space having any one of a triangular prism shape, a quadrangular prism shape, or a hexagonal prism shape, as one unit. As the triangular prism shape, a regular triangular prism shape is preferable. Furthermore, as the quadrangular prism shape, a rectangular parallelepiped shape is preferable in addition to the above cubic shape. Furthermore, as the hexagonal prism shape, a regular hexagonal prism shape is preferable. Note that it is preferable that the unit cell structure of each of the regions R1 to R3 constituting the orthosis has an identical spatial shape. Furthermore, the structural column is a column such as a polygonal prism shape or a cylindrical shape. Furthermore, the lateral direction of the structural column is a direction orthogonal to an axial direction of the structural column.

Next, the "unit cell model" of FIG. 44 will be described. The unit cell model represents the basic skeleton shape of the unit cell structure.

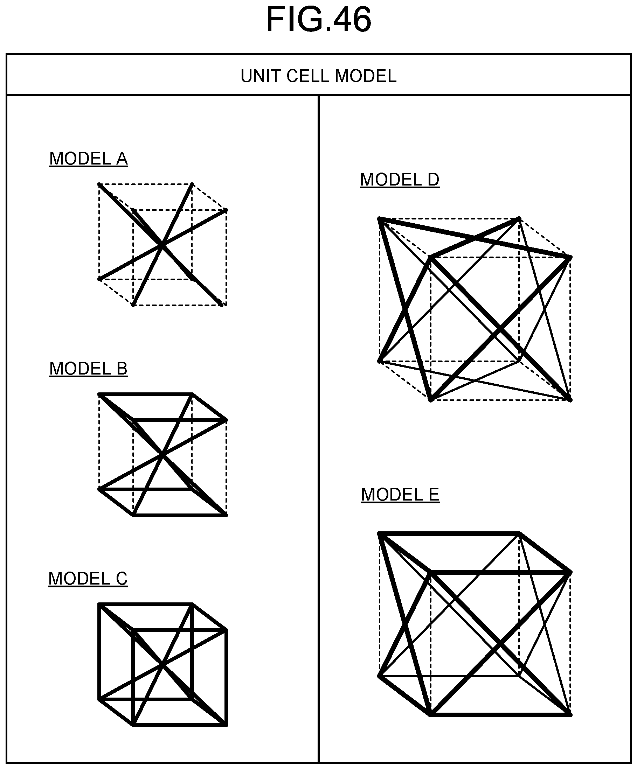

FIG. 46 is a diagram illustrating the unit cell model of the sixth embodiment. In FIG. 46, the dashed lines indicate a cubic space (space in FIG. 45) corresponding to one unit. The solid lines indicate the existence of structural columns. Preferably, the unit cell model illustrated in FIG. 46 is arranged in each of regions of the insole so that its arrangement direction matches the direction of a load applied to the orthosis in the vertical downward direction of FIG. 46. The direction of load applied to the orthosis is a direction in which the load received from each of sites of the human body is applied.

As illustrated in FIG. 46, for example, a unit cell model includes model A, model B, model C, model D, and model E. Here, model A, model B, and model C each have an intersection of structural columns at a center of a cubic space. Furthermore, model D and model E each have an intersection of structural columns at the center of at least one of a plurality of faces constituting the cubic shape.
Model A is a unit cell model with four structural columns arranged along the diagonals of the cubic shape. Specifically, model A has a structural column (P1, P7), a structural column (P2, P8), a structural column (P3, P5), and a structural column (P4, P6).
Model B is a unit cell model including, in addition to the four structural columns similar to model A, eight structural columns arranged along a plurality of sides surrounding the bottom face and the top face constituting the cubic shape. Specifically, model B includes four structural columns included in model A. In addition, model B includes four structural columns that surround the bottom face (P1, P2, P3, P4), namely, a structural column (P1, P2), a structural column (P2, P3), a structural column (P3, P4), and a structural column (P4, P1). In addition, model B has four structural columns that surround the top face (P5, P6, P7, P8), namely, a structural column (P5, P6), a structural column (P6, P7), a structural column (P7, P8), and a structural column (P8, P5) .
Model C is a unit cell model further including four structural columns arranged in the load direction in addition to 12 structural columns similar to model B. Specifically, model C has 12 structural columns included in model B. Model C further includes four structural columns, namely, a structural column (P1, P5), a structural column (P2, P6), a structural column (P3, P7), and a structural column (P4, P8) arranged in the direction of the load applied to the insole.
   That is, the difference between model B and model C is whether the model includes the four structural columns, namely, a structural column (P1, P5), a structural column (P2, P6), a structural column (P3, P7), and a structural column (P4, P8) arranged in the direction of the load.
Model D is a unit cell model with 12 structural columns arranged along the diagonals of each of the six faces. Specifically, model D has two structural columns, namely, a structural column (P1, P3) and a structural column (P2, P4) along the diagonals of the bottom face (P1, P2, P3, P4). Specifically, model D has two structural columns, namely, a structural column (P5, P7) and a structural column (P6, P8) along the diagonals of the top face (P5, P6, P7, P8). Specifically, model D has two structural columns, namely, a structural column (P1, P6) and a structural column (P2, P5) along the diagonals of the side face (P1, P2, P5, P6). Moreover, model D has two structural columns, namely, a structural column (P2, P7) and a structural column (P3, P6) along the diagonals of the side face (P2, P3, P6, P7). In addition, model D has two structural columns, namely, a structural column (P3, P8) and a structural column (P4, P7) along the diagonals of the side face (P3, P4, P7, P8). In addition, model D has two structural columns, namely, a structural column (P1, P8) and a structural column (P4, P5) along the diagonals of the side face (P1, P4, P5, P8).

As compared with model D, model E is a unit cell model further including eight structural columns arranged along a plurality of sides surrounding the bottom face and the top face, without including the structural columns along the diagonals of the bottom face or the top face. Specifically, model E has two structural columns, namely, a structural column (P1, P6) and a structural column (P2, P5) along the diagonals of the side face (P1, P2, P5, P6). In addition, model E has two structural columns, namely, a structural column (P2, P7) and a structural column (P3, P6) along the diagonals of the side face (P2, P3, P6, P7). In addition, model E has two structural columns, namely, a structural column (P3, P8) and a structural column (P4, P7) along the diagonals of the side face (P3, P4, P7, P8). In addition, model E has two structural columns, namely, a structural column (P1, P8) and a structural column (P4, P5) along the diagonals of the side face (P1, P4, P5, P8). In addition, model E has four structural columns surrounding the bottom face (P1, P2, P3, P4), namely, a structural column (P1, P2), a structural column (P2, P3), a structural column (P3, P4), and a structural column (P4, P1). In addition, model E has four structural columns surrounding the top face (P5, P6, P7, P8), namely, a structural column (P5, P6), a structural column (P6, P7), a structural column (P7, P8), and a structural column (P8, P5).

That is, the difference between model D and model E is the presence or absence of structural columns arranged on the bottom face and top face. Specifically, model D has four structural columns, namely, a structural column (P1, P3), a structural column (P2, P4), a structural column (P5, P7), and a structural column (P6, P8), along the diagonals of the bottom face and the top face. In addition, model E has eight structural columns surrounding the bottom face and the top face, namely, a structural column (P1, P2), a structural column (P2, P3), a structural column (P3, P4), a structural column (P4, P1), a structural column (P5, P6), a structural column (P6, P7), a structural column (P7, P8), and a structural column (P8, P5).

In FIG. 6, the structural columns of model D and model E are illustrated by solid lines of two types of thickness. However, this is intended to clarify the illustration, rather than indicating the actual thickness of the structural columns. In other words, the thicker solid line indicates the structural column included in the "faces visible on the front side of the box" when the cubic space is regarded as a box. Furthermore, the thinner solid line indicates a structural column included only in the "faces visible on the back side of the box" when the cubic space is regarded as a box. The "faces visible on the front side of the box" correspond to the top face (P5, P6, P7, P8), the side face (P1, P2, P5, P6), and the side face (P1, P4, P5, P8). The "faces visible on the back side of the box" correspond to the bottom face (P1, P2, P3, P4), the side face (P2, P3, P6, P7), and the side face (P3, P4, P7, P8) .

That is, each of regions of the insole of the present embodiment has a structure containing a plurality of unit cell models repeatedly and continuously arranged. Specifically, each of regions of the insole has at least one layer including a plurality of unit cell structures arranged on an identical plane, with these layers being stacked to form the region. The cross-sectional shape of the structural column may be any shape such as a polygon including a quadrangle, a pentagon or a hexagon, a circle or an ellipse, or the like.

The contents illustrated in FIG. 46 are merely an example, and the present invention is not limited to the contents in the drawing. For example, the model illustrated in FIG. 46 is merely an example, and the unit cell model may have structural columns at any positions. Still, it is preferable that the unit cell structure includes a plurality of structural columns so that any of the structural columns passes through all the vertices constituting the space of the polygonal prism shape.

Furthermore, the unit cell structure is not to limited to a structure having an intersection of structural columns at the center of a cubic space, or a structure having an intersection of structural columns at the center of at least one of a plurality of faces constituting the cubic shape. Still, it would be preferable that the unit cell structure has an intersection where at least two structural columns intersect at different position than the plurality of vertices.

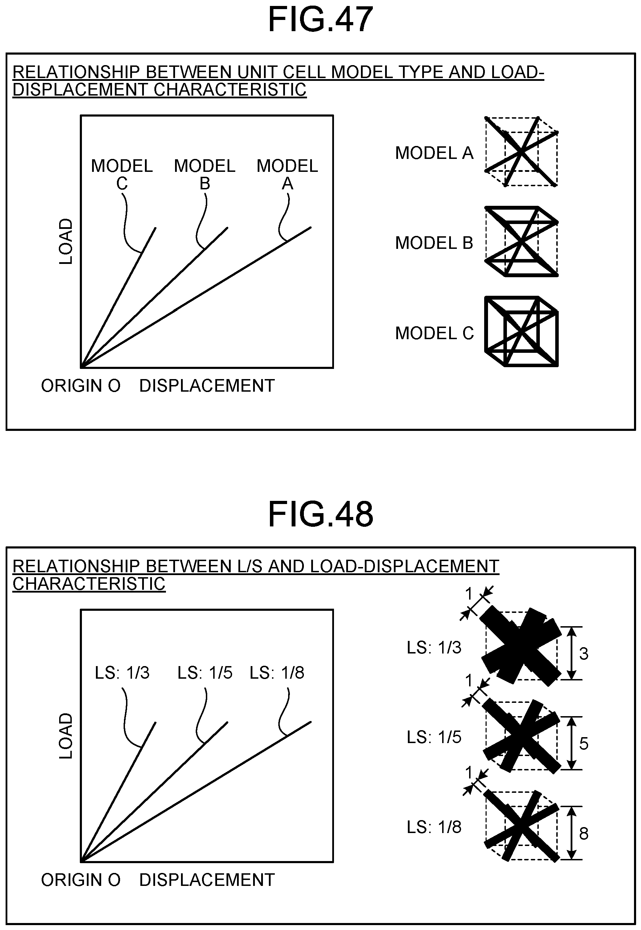

FIG. 47 is a diagram illustrating a relationship between the type of unit cell model and the load-displacement characteristic of the sixth embodiment. Here, the load-displacement characteristic represents a relationship between a load [N] applied to an object and a displacement [mm] of the object due to the load, and is utilized as a physical index of the hardness or adhesion of the object. In the present embodiment, the load-displacement characteristic is measured by applying a load to an object having a unit cell structure. In the graph of load-displacement characteristic, the vertical axis of the graph corresponds to the load [N], and the horizontal axis of the graph corresponds to the displacement [mm]. That is, in the graph of load-displacement characteristic, the harder the object, the steeper the graph, and the softer the object, the gentler the graph. Normally, although the graph is often obtained as a curve, the following figures use a straight line for simplification.

In FIG. 47, the load-displacement characteristic of the object constituted with the unit cell models of the models A, B, and C illustrated in FIG. 46 will be described. In FIG. 47, the thickness of the structural columns of the models A, B, and C and the volume of the intersection are constant.

From the load-displacement characteristic illustrated in FIG. 47, it is observed that the larger the number of structural columns included in each model, the harder the object tends to be. Furthermore, based on the load-displacement characteristic illustrated in FIG. 47, it is observed that the closer the direction of the structural column is to the load direction, the harder the object tends to be. Therefore, constituting the individual regions with different types of unit cell models would make it possible to provide a plurality of regions having different physical properties.

Next, the "thickness of structural columns" in FIG. 44 will be described. The thickness of the structural columns is the thickness of each of the structural columns in the unit cell model, and is represented by the ratio of the thickness (L) of the structural columns to the length (S) of one side of the unit space.

FIG. 48 is a diagram illustrating a relationship between L/S and the load-displacement characteristic of the sixth embodiment. FIG. 48 uses model A of FIG. 46 to explain the load-displacement characteristic of objects having structural columns of different thicknesses. In FIG. 48, the volume of the intersection of each of the objects is constant.

Furthermore, FIG. 48 illustrates as an example of the thickness of the structural column, in which the ratio of the thickness (L) of the structural column and the length (S) of one side of the unit space is different. In the example of FIG. 48, "L/S: 1/3" corresponds to the case where the structural column is the thickest, with "L/S: 1/5" and "L/S: 1/8" corresponding to the case where the structural column is thinner and thinnest, respectively. The thickness of the structural column can be, for example, the length of the longest straight line among the straight lines passing through the center of gravity in the "cross section in the lateral direction" of the structural column.

From the load-displacement characteristic illustrated in FIG. 48, it is observed that the larger the L/S (thicker the column), the harder the object tends to be. In addition, the smaller the L/S (thinner the column), the softer the object tends to be. Therefore, constituting a plurality of regions with structural columns with different thicknesses would make it possible to provide a plurality of regions having different physical properties.

Next, the "volume of the intersection" in FIG. 44 will be described. The volume of the intersection is the volume of the site (intersection) where at least two structural columns intersect in the unit space.

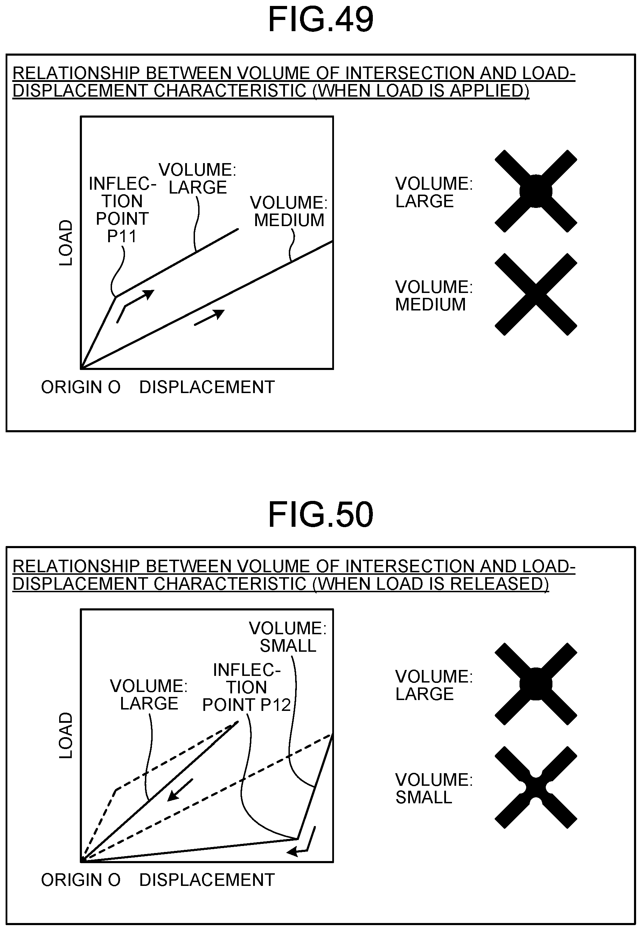

FIG. 49 is a diagram illustrating a relationship between the volume of an intersection and the load-displacement characteristic of the structural columns in the unit cell model generated in the sixth embodiment. FIG. 49 illustrates a relationship between the volume of the intersection and the load-displacement characteristic when a load is applied. FIG. 49 uses model A of FIG. 46 to explain the load-displacement characteristic of objects having intersections with different volumes. In FIG. 49, the thickness (L) of the structural column of each of objects is constant. In the load-displacement characteristic when a load is applied as illustrated in FIG. 49, a point (position) on the graph indicating the displacement corresponding to the load moves in the direction of the arrow in the graph.

In the load-displacement characteristic illustrated in FIG. 49, an inflection point P11 is observed in the graph in a case where the volume of the intersection is large. In other words, an object with a large volume at the intersection behaves like a hard substance between an origin 0 and the inflection point P11 together with application with a load, and behaves like a soft substance after the inflection point P11. The change in physical properties (change in the slope of the graph) in this load-displacement characteristic is felt as adhesion when a person using the orthosis applies a load to the insole. Moreover, an object with a medium volume at the intersection behaves similarly to a soft object. Therefore, constituting a plurality of regions with intersections with different volumes would make it possible to provide a plurality of regions having different physical properties.

FIG. 50 is a diagram illustrating the relationship between the volume of the intersection and the load-displacement characteristic of the sixth embodiment. FIG. 50 illustrates a relationship between the volume of the intersection and the load-displacement characteristic when a load is released. FIG. 50 uses model A of FIG. 46 to explain the load-displacement characteristic of objects having intersections with different volumes. In FIG. 50, the thickness (L) of the structural column of each of objects is constant. In the load-displacement characteristic when a load is released as illustrated in FIG. 50, a point (position) on the graph indicating the displacement corresponding to the load moves in the direction of the arrow in the graph. The dashed graph illustrated in FIG. 50 corresponds to the graph when a load is applied.

In the load-displacement characteristic illustrated in FIG. 50, when the volume of the intersection is large, the shape recovery speed is high and a slope relatively close to linear can be obtained. That is, for an object having a large volume at the intersection, the displacement can make a quick recovery when the load is released. This physical property is felt as adhesion when the person using the orthosis releases the load from the insole. By contrast, when the volume of the intersection is small in the load-displacement characteristic illustrated in FIG. 50, the shape recovery speed is slow with observation of an inflection point P12. In other words, regarding an object with a small volume at the intersection, the amount of change in the displacement is small up to the inflection point P12 together with the release of the load, and the shape recovers to the original shape between the inflection point P12 and the origin 0. Therefore, constituting a plurality of regions with intersections with different volumes would make it possible to provide a plurality of regions having different physical properties.

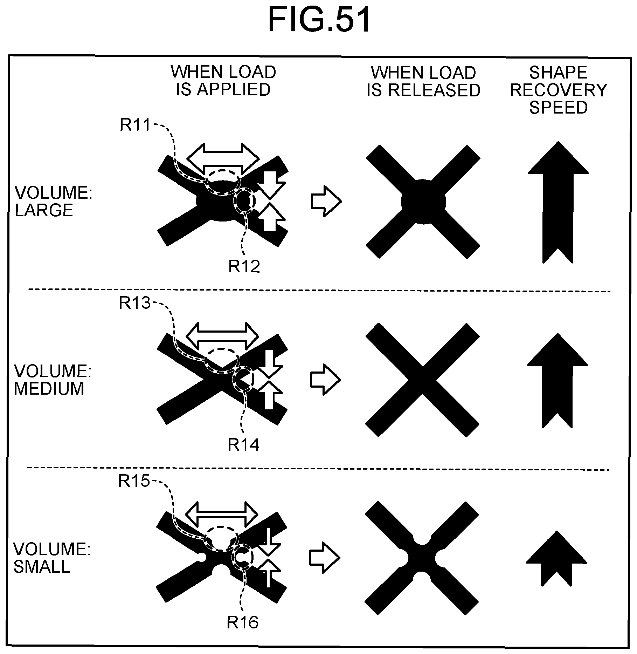

FIG. 51 is a diagram illustrating a difference in shape recovery speed depending on the volume of the intersection of the sixth embodiment. FIG. 51 describes the difference in shape recovery speed according to the volume of the intersection when a load is applied from the top face to the bottom face of the unit cell structure in model A of FIG. 46. FIG. 51 illustrates the change in the shape of the intersection at transition from application of the load to the release of the load, and the shape recovery speed in the change. The upper row of FIG. 51 illustrates a case where the intersection has a large volume, the middle row illustrates a case where the intersection has a medium volume, and the lower row illustrates a case where the intersection has a small volume. The length of the arrow indicating the shape recovery speed corresponds to the degree of the shape recovery speed.

With FIG. 51, the shape of the intersection will be described. When the volume is not adjusted in particular, the intersection has a shape as illustrated in the middle row of FIG. 51 (when the load is released). In the present embodiment, this shape will be described as a state in which the intersection has a medium volume.

When the intersection has a large volume, the intersection has a spherical shape or a polyhedral shape with the intersection as a substantially center, as illustrated in the upper row of FIG. 51 (when the load is released). That is, the intersection has a positively raised shape as compared with a state in which a plurality of structural columns is simply intersected. In this case, the intersection has a cross section larger than the cross section of the structural column in the lateral direction. Specifically, the area of the cross section of the intersection is preferably 1.1 times or more and 20 times or less as compared with the cross section of the structural column in the lateral direction, more preferably be 1.2 times or more and 10 times or less, and still more preferable when the area is 1.5 times or more and 5 times or less, in particular. Here, the area of the cross section of the intersection is based on the surface having the largest area among the surfaces passing through the center of gravity of the intersection.

In contrast, when the intersection has a small volume, the intersection has a shape in which the structural columns in the vicinity of the intersection are thinned, as illustrated in the lower row of FIG. 51 (when the load is released). In this case, the intersection has a cross section smaller than the cross section of the structural column in the lateral direction. Specifically, the area of the cross section of the intersection is preferably 0.05 times or more and 0.9 times or less as compared with the cross section of the structural column in the lateral direction, more preferably be 0.1 times or more and 0.8 times or less, and particularly preferable when the area is 0.2 times or more and 0.7 times or less. Here, the area of the cross section of the intersection is based on the surface having the largest area among the surfaces passing through the center of gravity of the intersection.

Here, as illustrated in FIG. 51, energy for shape recovery is considered to be accumulated at the intersection when a load is applied. For example, when a load is applied from the upper side to the lower side in FIG. 51, the two structural columns on both sides of a region R13 at the intersection are expanded, resulting in accumulation of the restoring force for the recovery of the columns in the region R13. In addition, the two structural columns on both sides of a region R14 at the intersection are narrowed, resulting in accumulation of a repulsive force for their recovery in the region R14.

When the intersection has a large volume, the restoring force and the repulsive force are accumulated as a large amount of energy. Therefore, the restoring force of a region R11 is greater than the restoring force of the region R13, and the repulsive force of a region R12 is greater than the repulsive force of the region R14. Consequently, it is considered that the greater the volume of the intersection, the higher the shape recovery speed.

When the intersection has a small volume, the restoring force and the repulsive force are accumulated as a small amount of energy. Therefore, the restoring force of a region R15 is less than the restoring force of the region R13, and the repulsive force of a region R16 is less than the repulsive force of the region R14. Consequently, it is considered that the smaller the volume of the intersection, the lower the shape recovery speed.

In this manner, the model data of the individual subjects S-1 to S-N includes the outer shape data of the insole and the shape parameters of the insole, for example. The model data of the individual subjects S-1 to S-N is the information used as a basis when the insoles of individual subjects S-1 to S-N are modeled by the modeling device 70. That is, the model data of individual subjects S-1 to S-N is generated in advance by a manufacturer of the insoles of the individual subjects S-1 to S-N and is preliminarily stored in the storage unit 601.

The contents illustrated in FIGS. 45 to 51 are merely an example, and the present invention is not limited to the contents in the drawings. Furthermore, although FIG. 51 uses three levels for illustrating the volume of the intersection, the present invention is not limited to the three levels of volumes and can manufacture the intersection so as to have a desired volume.

Next, "evaluation information" will be described. The evaluation information is information related to evaluation of the insole of the individual subjects S-1 to S-N. For example, the evaluation information includes at least one of wearability evaluation information regarding the wearability of the insole by the individual subjects S-1 to S-N or symptom evaluation information regarding the symptoms of the individual subjects S-1 to S-N.

The wearability evaluation information is information evaluated by each of the subjects S-1 to S-N based on evaluation items such as ease of wearing the insole, tightness when wearing the insole, supportiveness, breathability, time required for wearing, and design. For example, the wearability evaluation information is expressed by a numerical value (score) in three levels of "1" to "3". Higher numerical value indicates higher evaluation, for example.

The symptom evaluation information is information evaluated based on evaluation items such as the degree of posture retention when the insole is worn, the change in posture before and after the use of the insole, and the improvement status of skeletal deformity or the like before and after the use of the insole. For example, the symptom evaluation information is obtained by evaluation by the individual subjects S-1 to S-N, person(s) (family, etc.) around the individual subjects S-1 to S-N, or medical professionals such as doctors or physiotherapists who evaluate the symptoms and treatment conditions of the individual subjects S-1 to S-N. For example, the symptom evaluation information is expressed by a numerical value (integer) in three levels of "1" to "3". Higher numerical value indicates higher evaluation, for example.

In this manner, the evaluation information includes at least one of the wearability evaluation information or the symptom evaluation information. The evaluation information is collected by a builder of the trained model and is stored in advance in the storage unit 601. For example, a builder of a trained model collects evaluation information by conducting a questionnaire survey or the like on a subject, a family member, a medical person, or the like. The above description is merely an example, and is not limited to the above example. For example, the evaluation items for obtaining evaluation information can be arbitrarily set by the builder of the trained model.

Back to the description of FIG. 40. The learning unit 603 reads out the foot information of the individual subjects S-1 to S-N, the model data of the insole of the individual subjects S-1 to S-N, and the evaluation information of the insole of the individual subjects S-1 to S-N, from the storage unit 601. The learning unit 603 then performs machine learning by inputting the read information, namely, the foot information of the individual subjects S-1 to S-N, the model data of the insole of the individual subjects S-1 to S-N, and the evaluation information of the insole of the individual subjects S-1 to S-N, as training data, into a machine learning engine.

Here, the machine learning in the learning unit 603 can be implemented by using a known machine learning engine. For example, machine learning engines can be implemented by application of various algorithms including deep learning, neural networks, logistic regression analysis, non-linear discriminant analysis, a support vector machine (SVM), Random Forest, Naive Bayes, or the like.

Moreover, in this machine learning, the model data of the insole is used as labeled training data. The foot information corresponds to the information to become input data at the time of application of the trained model. Furthermore, the evaluation information is information for weighting the model data which is the labeled training data.

For example, the learning unit 603 builds a correlation matrix between the foot information for each foot information of the subjects S-1 to S-N. The learning unit 603 determines boundary planes (hyperplanes) in upper k eigenvector spaces that have a large contribution to the model data by principal component analysis using a correlation matrix. At this time, a weight determined based on the evaluation information of individual subjects S-1 to S-N is given to the model data of the individual subjects S-1 to S-N.

In this way, the learning unit 603 performs machine learning to generate a trained model that outputs the optimum model data for the input of foot information. The learning unit 603 stores the generated trained model in the storage unit 601.

While the above has described machine learning in the case of weighting the model data of individual subjects S-1 to S-N using evaluation information, the embodiment is not limited to this. For example, the learning unit 603 can perform machine learning without using evaluation information. In this case, the learning unit 603 performs machine learning by inputting the foot information of individual subjects S-1 to S-N and the model data of the insole of the individual subjects S-1 to S-N into the machine learning engine as training data.

Furthermore, although the above description is the case where the foot information and model data of a plurality of subjects S-1 to S-N are used as training data regardless of the disease or symptom, the embodiment is not limited to this. For example, the learning unit 603 may narrow down the training data depending on a specific disease or symptom. For example, the learning unit 603 performs machine learning using the foot information of the subject with hallux valgus and the model data of the insole manufactured to treat or improve the hallux valgus of the subject as training data. With this learning, the learning unit 603 can build a trained model specialized for hallux valgus.

The acquisition unit 604 acquires site information including the outer shape data of the site of the subject S-X based on the image data obtained by imaging the site of the subject S-X. For example, the acquisition unit 604 acquires the foot information by using the three-dimensional X-ray CT image data in which the foot of the subject S-X being the target for creating the insole is captured, as the image data.

For example, when the user interface unit 602 has received a model data generation request, the generation request is transmitted to the acquisition unit 604. Here, the model data generation request includes, for example, identification information (patient ID, or the like) for identifying the subject S-X being the target for creating a new insole. Having received the model data generation request, the acquisition unit 604 reads out the image data of the subject S-X (three-dimensional X-ray CT image data) identified by the patient ID from the various types of information stored in the storage unit 601.

Subsequently, the acquisition unit 604 performs known image processing such as edge detection processing on the three-dimensional X-ray CT image data to extract the contour (body surface) of the foot. With this operation, the acquisition unit 604 acquires outer shape data of the foot representing the three-dimensional surface shape of the foot of the subject from the image data.

In this manner, the acquisition unit 604 acquires the foot information including the outer shape data (refer to FIG. 5). Although the above description is a case where the site information includes the outer shape data, the present invention is not limited to this. For example, the site information can include various types of feature information acquirable from the X-ray CT image data by using segmentation processing, pattern matching processing, or the like, in addition to the outer shape data. Furthermore, although the above description is a case where the image data is three-dimensional X-ray CT image data, the present invention is not limited to this. For example, the image data captured by a 3D scanner is also applicable. For example, a 3D scanner captures 3D scan data as image data. The 3D scan data includes foot outer shape data as foot information.

The generation unit 605 inputs site information of the subject S-X into the trained model to generate model data for designing the orthosis to be worn on the site of the subject S-X. Note that the trained model is a model trained, for the sites of the plurality of subjects S-1 to S-N, by using site information including the outer shape data of the sites of the individual subjects S-1 to S-N, model data for designing the orthosis to be worn on the sites of the individual subjects S-1 to S-N, and the evaluation information regarding evaluation of the orthosis.

For example, the generation unit 605 reads out the trained model generated by the learning unit 603 from the storage unit 601. The generation unit 605 then inputs the site information of the subject S-X acquired by the acquisition unit 604 into the read trained model to allow the trained model to output model data for designing an orthosis to be worn on the site of the subject S-X. Subsequently, the generation unit 605 transmits the model data output from the trained model to the output control unit 606.

The model data output from the trained model includes the same type of information as the model data used for building the trained model. That is, in a case where the model data used for machine learning includes shape parameters of the shape of the unit cell structure, the length of each side of the unit cell structure, the model, the thickness of the structural columns, and the volume of the intersection, the model data output from the trained model also includes the same type of shape parameters.

In addition, the generation unit 605 receives an operation of correcting the model data on an image in which the model data and the site information are simultaneously displayed, and corrects the model data in accordance with the received operation.

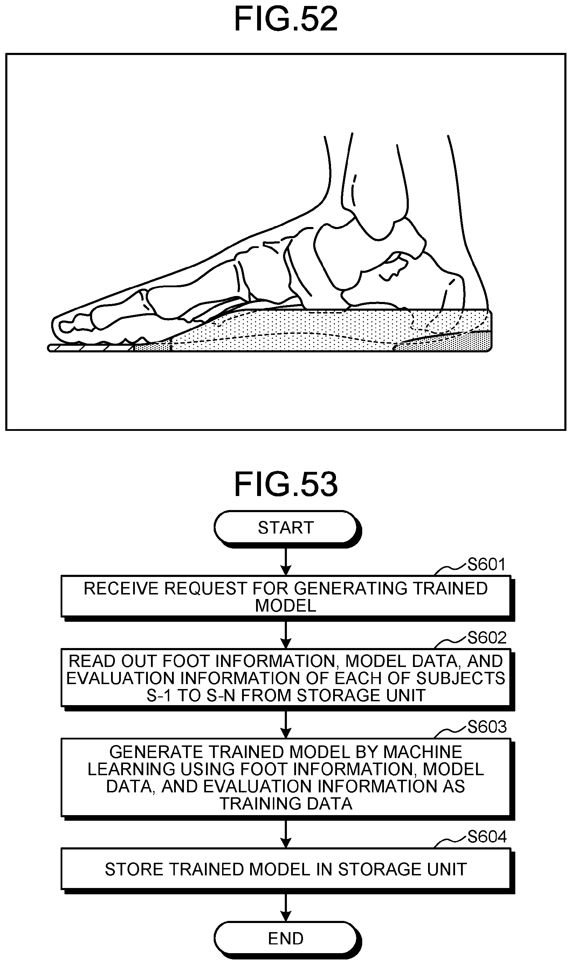

FIG. 52 is a diagram illustrating a correction process of model data according to the sixth embodiment.

As illustrated in FIG. 52, the display device 66 displays model data and foot information. Here, the position of the outer shape data of the insole and the position of the outer shape data of the foot are displayed in association with each other.

For example, with reference to the model data and the foot information displayed on the display device 66, the operator performs an operation of correcting the model data by using the input device 65. Having received the operation of correcting the model data from the operator, the user interface unit 602 notifies the generation unit 605 of the received operation. The generation unit 605 makes correction to the model data according to the operation received by the user interface unit 602. With this procedure, the operator can correct the outer shape data of the insole while confirming the outer shape data of the actual foot on the screen.

The output control unit 606 outputs the model data generated by the generation unit 605. For example, the output control unit 104 stores the model data of the subject S-X generated by the generation unit 605 in the storage unit 101. Furthermore, the output control unit 606 transmits the model data of the subject S-X to the modeling device 70.

In addition, the output control unit 606 displays the model data of the subject S-X on the display device 16. For example, the output control unit 606 controls to simultaneously display the model data and the site information of the subject S-X. With this configuration, the operator can perform an operation of correcting the model data while referring to the model data and the foot information displayed simultaneously on the display device 66.

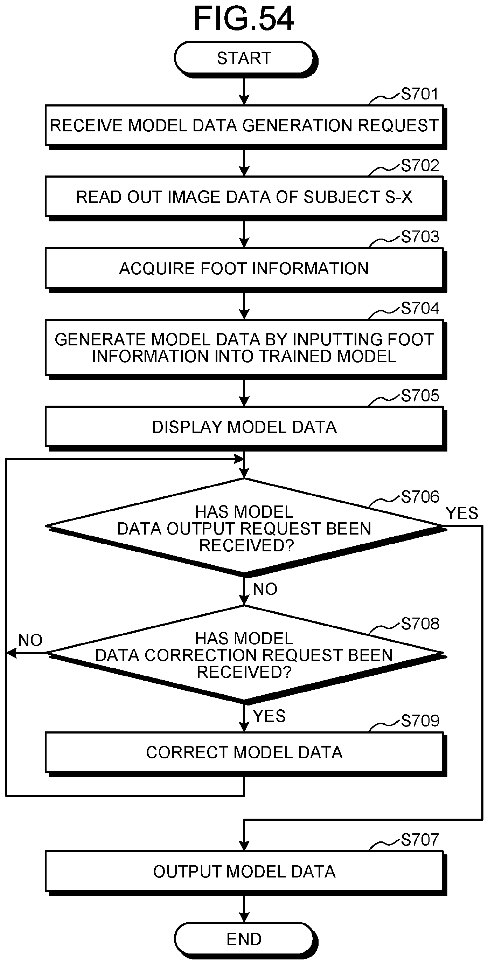

FIGS. 53 and 54 are flowcharts illustrating an operation example of the information processing apparatus 60 according to the sixth embodiment. FIG. 53 illustrates the process of the information processing apparatus 60 during learning. FIG. 54 illustrates the process of the information processing apparatus 60 during the application of the trained model. Since the specific process of each of steps is as described above, detailed description thereof will be omitted as appropriate.

As illustrated in FIG. 53, the user interface unit 602 receives a trained model generation request (step S601). The user interface unit 602 transmits the received generation request to the learning unit 603.

The learning unit 603 reads out the foot information, model data, and evaluation information of each of the subjects S-1 to S-N from the storage unit 601 (step S602). The learning unit 603 generates a trained model by machine learning using the foot information, the model data, and the evaluation information as training data (step S603). The learning unit 603 then stores the trained model in the storage unit 601.

As illustrated in FIG. 54, the user interface unit 602 receives a model data generation request (step S701). The user interface unit 602 transmits the received model data generation request to the acquisition unit 604. The acquisition unit 604 reads out image data of the subject S-X (step S702). The acquisition unit 604 then acquires the foot information from the read image data (step S703).

Subsequently, the generation unit 605 generates model data by inputting the foot information into the trained model (step S704). Subsequently, the output control unit 606 displays the generated model data (step S705) .

Next, the user interface unit 602 determines whether the model data output request has been received (step S706). When the model data output request has been received (step S706, Yes), the output control unit 606 outputs the model data (step S707) and ends the process.

In contrast, when the model data output request has not been received (step S706, No), the user interface unit 602 determines whether the model data correction request has been received (step S708). When the model data correction request has been received (step S708, Yes), the generation unit 605 corrects the model data in response to the model data correction request (step S709).

In contrast, when the model data correction request has not been received (step S708, No), or after step S709, the user interface unit 602 returns to step S706 and determines whether the model data output request has been received. When the model data output request has been received in step S706 after step S709 (step S706, Yes), the output control unit 606 outputs corrected model data (step S707), and ends the process.

The processing procedures illustrated in FIGS. 53 and 54 are merely an example, and are not limited to the contents illustrated in the drawings. For example, other processing procedures can be added (inserted) or the order of individual processes can be exchanged as long as there is no contradiction in the processing content. Furthermore, each of processing procedures does not necessarily have to be executed.

As described above, the information processing apparatus 60 of the sixth embodiment acquires first site information including outer shape data of a site of a first subject based on the image data obtained by imaging the site of the first subject. Furthermore, the information processing apparatus 60 generates first model data for designing a first orthosis to be worn on a site of the first subject by inputting the first site information into a trained model that has been trained, for sites of a plurality of second subjects, by using second site information including outer shape data of the site of each of the second subjects, second model data for designing a second orthosis to be worn on the site of each of the second subjects, and evaluation information related to evaluation of the second orthosis. In addition, the information processing apparatus 60 outputs the first model data. According to this, the information processing apparatus 60 of the sixth embodiment can achieve facilitated designing and stable quality of an orthosis.

For example, since the information processing apparatus 60 generates model data by inputting foot information to the trained model, it is possible to reduce the time and cost required for designing the orthosis. Furthermore, since the information processing apparatus 60 generates model data based on the trained model, it is possible to reduce the varying quality.

While the present embodiment has described a case where the process during learning and the process during application are executed in one information processing apparatus 60, each of these processes can be executed in individual information processing apparatuses.

For example, the information processing apparatus 60 responsible for processing during learning includes at least the learning unit 603. In this case, the trained model generated by the learning unit 603 is used by another apparatus (another information processing apparatus) different from the information processing apparatus 60. Therefore, the trained model generated by the information processing apparatus 60 is handed to the different apparatus and stored in the internal storage unit of the different apparatus. Information may be exchanged between the information processing apparatus 60 and the different apparatus via a network or a recording medium.

Furthermore, for example, the information processing apparatus 60 that is responsible for processing during application includes at least the functions of the acquisition unit 604, the generation unit 605, and the output control unit 606. In this case, the information processing apparatus 60 generates model data using a trained model built by another apparatus (another information processing apparatus) different from the information processing apparatus 60. The trained model is handed over from another apparatus and stored in advance in the storage unit 601.

### (Other embodiments related to sixth embodiment)

In addition to the above-described embodiments, various types of different embodiments may be implemented.

### (Orthosis manufacturing method)

The modeling device 70 of the sixth embodiment can manufacture an orthosis by the following manufacturing method.

For example, the modeling device 70 can model (mold) an orthosis by using various additive manufacturing technologies (three-dimensional modeling technology) such as a material extrusion method, a vat photopolymerization method, and a selective laser sintering method.

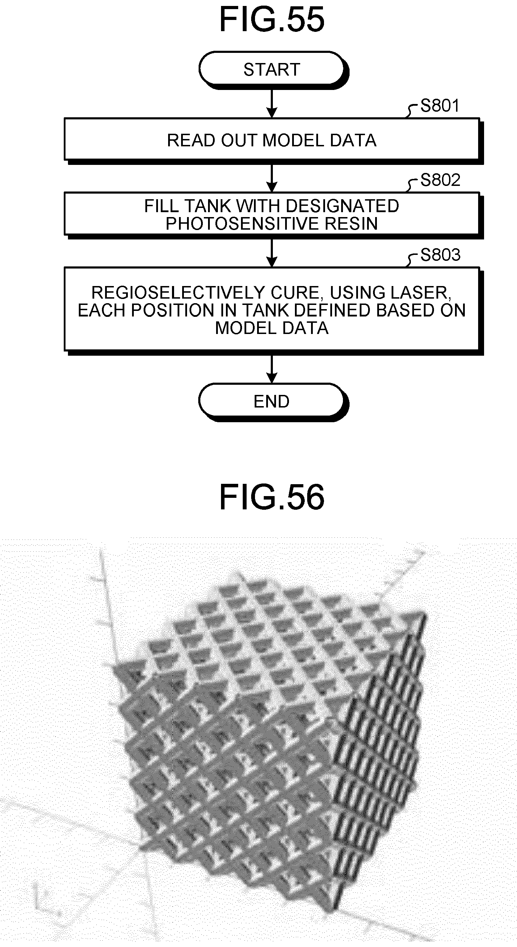

FIG. 55 is a flowchart illustrating an example of a manufacturing method of the embodiment. The manufacturing method illustrated in FIG. 55 is executed by the modeling device 70 capable of executing a three-dimensional modeling technology by a vat photopolymerization method. The modeling device 70 (modeling unit 31) includes a vat (tank) for filling the photosensitive resin, which is the material of the orthosis, and a laser for regioselectively curing the photosensitive resin in the vat by a photopolymerization reaction. Note that known modeling devices can be flexibly applied as the modeling device 70.

As illustrated in FIG. 55, the modeling unit 31 reads out model data (step S801). This model data is information that forms the basis for modeling with the modeling device 70. The model data is created in advance by a manufacturer, for example, and preliminarily stored in a storage device included in the modeling device. The storage device corresponds to, for example, a hard disc drive (HDD), a solid state drive (SSD), or the like.

Subsequently, the modeling unit 31 fills the tank with designated photosensitive resin (step S802). Here, the photosensitive resin may be designated in advance by the model data, or may be designated by an operator each time the modeling method is executed.

Subsequently, by using a laser, the modeling unit 31 regioselectively cures individual positions in the tank defined based on the model data (step S803). For example, the modeling unit 31 sequentially emits laser toward the positions in the tank corresponding to the positions where the structural columns exist in the model data. That is, the modeling unit 31 sequentially cures the positions in the tank corresponding to the unit cell structure of each of regions of the orthosis. With this procedure, the modeling unit 31 can manufacture an orthosis having appropriate physical properties.

The material of the orthosis can be any material as long as it is a material that can be used for modeling technology. For example, any photosensitive resin that can be modeled by additive manufacturing technology can be appropriately selected.

### (Orthosis manufacturing method using system 2)

As described above, the system 2 of the embodiment includes the information processing apparatus 60 and the modeling device 70. That is, the system 2 can manufacture the orthosis by the functions of the information processing apparatus 60 and the modeling device 70.

That is, in the system 2, the information processing apparatus 60 acquires first site information including outer shape data of the site of a first subject based on the image data obtained by imaging the site of the first subject. In addition, the information processing apparatus 60 generates first model data for designing a first orthosis to be worn on a site of the first subject by inputting the first site information into a trained model that has been trained, for sites of a plurality of second subjects, by using second site information including outer shape data of the site of each of the second subjects, second model data for designing a second orthosis to be worn on the site of each of the second subjects, and evaluation information related to evaluation of the second orthosis. In the system 2, the modeling device 70 models the first orthosis based on the first model data.

For example, in the system 2, the information processing apparatus 60 generates model data by the processes of steps S701 to S709 illustrated in FIG. 54.

The information processing apparatus 60 transmits the model data to the modeling device 70. For example, the output control unit 606 of the information processing apparatus 60 converts the model data into a data format that can be used by the modeling device 70, and transmits the converted model data to the modeling device 70.

Subsequently, in the system 2, the modeling device 70 models the orthosis based on the model data received from the information processing apparatus 60 by using the processes of steps S801 to S803 illustrated in FIG. 55.

According to this, the system 2 can achieve facilitated designing and stable quality of an orthosis. The individual processing units included in the information processing apparatus 60 and the modeling device 70 may be provided in any of the devices. For example, when the modeling device 70 includes the generation unit 605, the modeling device 70 may generate model data using the trained model. In this case, the output control unit 606 of the information processing apparatus 60 will transmit the site information acquired by the acquisition unit 604 to the modeling device 70.

### (Load direction)

Although the above embodiment is a case where the load direction corresponds to the vertical downward direction in the drawing as illustrated in FIG. 51, for example, the embodiment is not limited to this. For example, the direction of the unit cell structure in the orthosis can be appropriately set by the manufacturer. For example, in the case of a hand-worn orthosis, the normal direction at the contact point between the hand and the orthosis may be defined as the load direction. In this case, the unit cell structure of FIG. 46 is arranged in the orthosis so that the vertical downward direction in the drawing corresponds to the normal direction at the contact point with the human body.

According to the embodiment described above, it is possible to provide an information processing apparatus, a system, an information processing method, and a program capable of achieving facilitated designing and stable quality of an orthosis.

The above-described embodiments can be combined with the above modifications in any manner, and the above modifications may be combined in any manner.

The programs executed by the information processing apparatus 60 of the above-described embodiments may be recorded as files in an installable format or an executable format in computer readable recording medium such as a CD-ROM, flexible disk (FD), CD-R, DVD, or universal serial bus (USB) and provided. Alternatively, the programs may be provided or distributed via a network such as the Internet. Furthermore, various programs may be provided by being incorporated in advance in a non-volatile storage medium such as ROM.

### [Examples]

Hereinafter, the present invention will be described in more detail based on examples, but the present invention is not limited to these.

### (Mechanical properties of Architected Material)

As an example, the mechanical properties of Architected Material will be described. The following will sequentially describe fabrication and evaluation of a lattice cube structure, indentation behavior of the lattice cube structure, a comparison with an existing 3D printer structure, and a comparison with an existing insole material. In the following description, an object created by combining unit cell structures is referred to as a "structure".

### (Fabrication and evaluation of lattice cube structure)

As an Architected Material, a lattice cube structure having a periodic structure of 5 × 5 × 5 (a cubic structure formed by combining lattice structures, which will be simply referred to as a "lattice cube") was designed by using OpenSCAD. For the unit cell structure, the structural pattern of the cubic system illustrated in FIG. 3 (body-centered cubic model: models A to C, face-centered cubic: models D and E) and the simple cubic model F were used. Model F is a structural pattern having structural columns on individual sides of the cubic space illustrated in FIG. 2.

The designed lattice cube structure (FIG. 56) is modeled with a CLIP modeler M2 (manufactured by Carbon) using a photocurable urethane elastomer EPU 40 (manufactured by Carbon) based on a Standard Triangulated Language (STL) structure. For each of lattice cube samples, the load-displacement curve during indentation was evaluated with a precision universal testing machine (INSTRON 5967). Note that FIG. 56 is a diagram illustrating a lattice cube designed by OpenSCAD.

### (Indentation behavior of lattice cube structure)

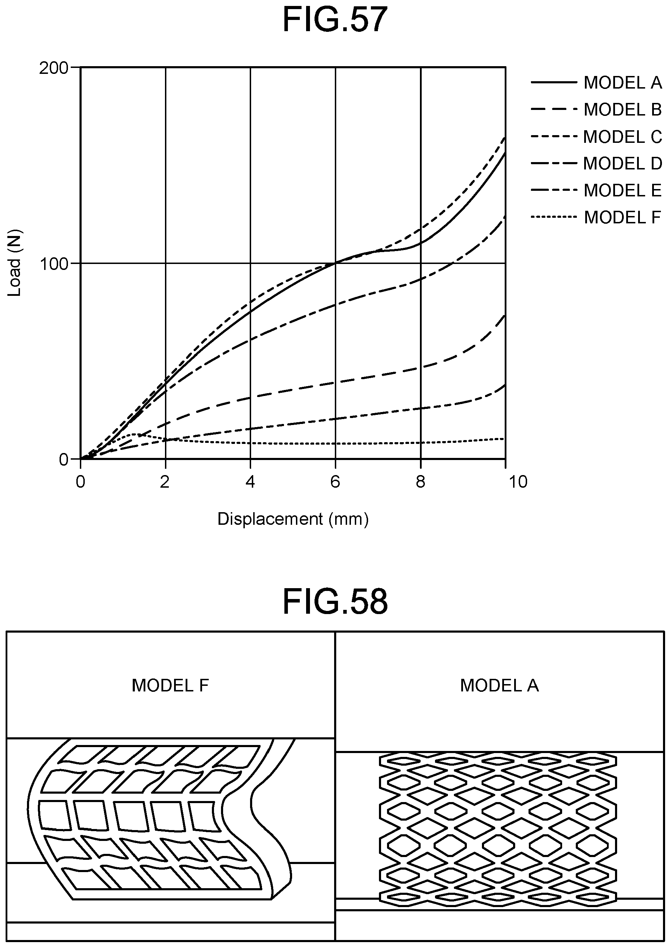

It was confirmed that, by changing the structure of the unit cell, the outline of the load-displacement curve will change significantly as illustrated in FIG. 57. Only in the case of model F, there is observed bending of the load-displacement curve considered to be caused by the buckling of the cube structure as observed at the displacement of 1 mm (5% of the cube size) (left figure in FIG. 58). In the case of models A to E, it was confirmed that deformation as initial deformation was deformation which is due to the folding of the structure, and in which the apparent Poisson's ratio became 0 until the upper and lower unit cells of compression come in contact with each other (right figure in FIG. 58). In addition, all the lattice cubes tested demonstrated the behavior of elastic work that restored to the original structure when unloading after 50% compression. Note that FIG. 57 is a diagram illustrating a load-displacement curve of a lattice cube with a different unit cell. FIG. 58 is a diagram illustrating a behavior during cube compression. In FIG. 58, the left figure illustrates the compression behavior of the model F, and the right figure illustrates the compression behavior of the model A.

### (Comparison with existing 3D printer structure)

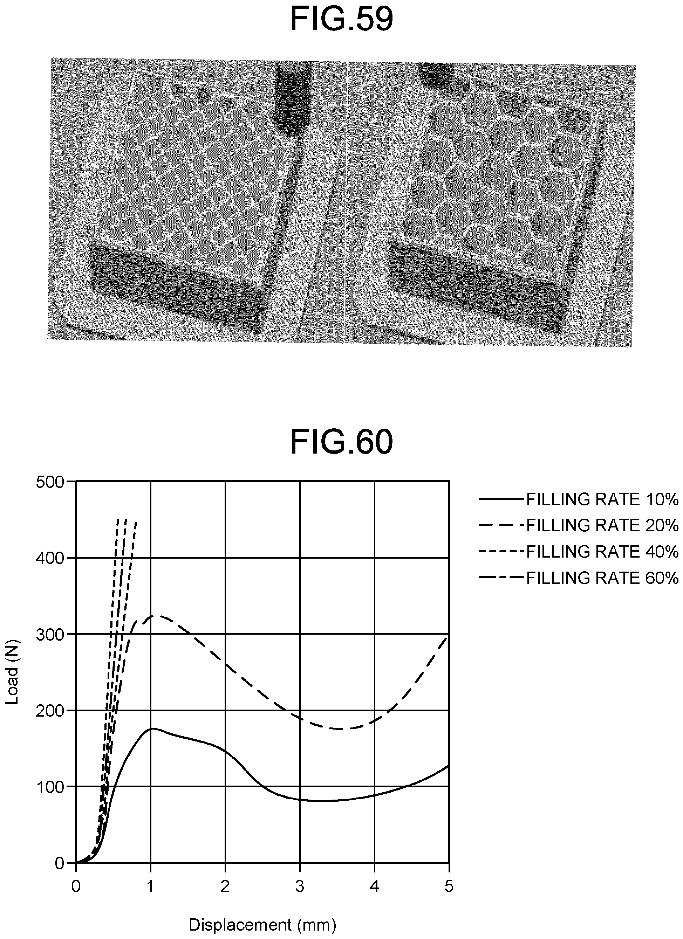

As a comparative example, the modeled structure using an existing 3D printer was evaluated. There is a case where a flexible structure is realized by controlling the fill density (Non Patent Literature 1: J. Li, H. Tanaka: The flexibility controlling study for 3D printed splint, Nanosensors, Biosensors, Info-Tech Sensors and 3D Systems, 101671A, (2017)). Using the filament FABRIAL (registered trademark) -R (manufactured by JSR Corporation) manufactured using a thermoplastic elastomer material, a cube shape of the same size as the lattice cube modeled in FIG. 56 was designed by the fused deposition modeling (FDM) method, which is a general-purpose method for 3D printers. A sample was modeled by using the slicer settings to change the internal filling pattern (FIG. 59) and filling rate (increasing the set value increases the line-space ratio (L/S) in the modeling plane and makes a pattern with higher density), and the load-displacement curve during indentation in the modeled sample was evaluated with a precision universal testing machine (INSTRON 5967). Note that FIG. 59 is a diagram illustrating filling patterns by the slicer when modeling a cube by the FDM method. The left figure of FIG. 59 illustrates rectilinear shape, and the right figure illustrates a honeycomb shape.

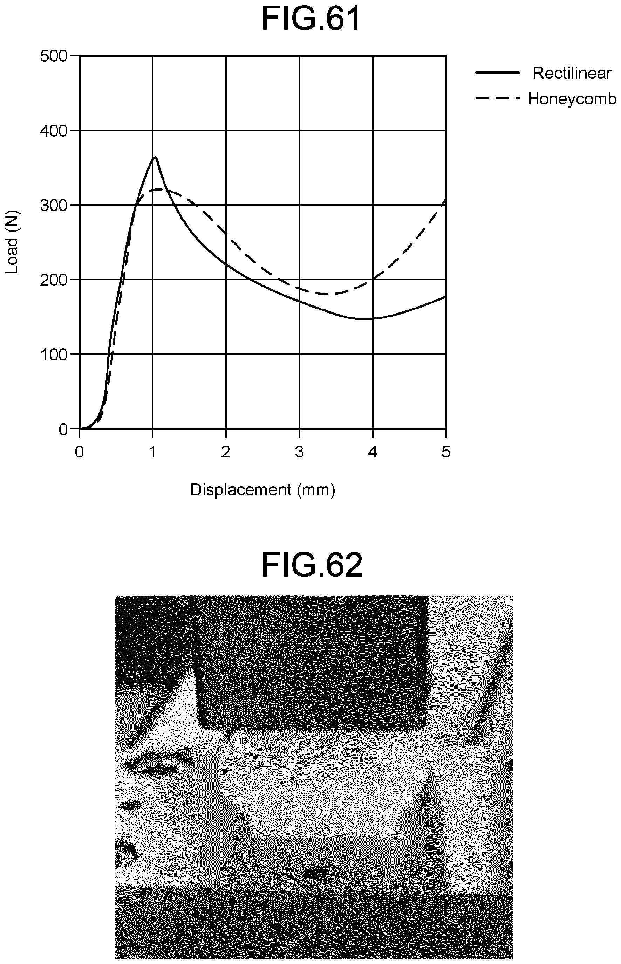

As seen in FIGS. 60 and 61, the slope of the initial load-displacement curve would not change even with the change in the filling rate or filling pattern. This indicates that there is no change in the initial indentation stress. Furthermore, when the filling rate is low, the load-displacement curve presumed to be derived from buckling is bent similarly to the model F of FIG. 57. As illustrated in FIG. 62, all FDM modeling samples demonstrate compression behaviors in which the apparent Poisson's ratio is positive during compression. In observation of the sample after compression and unloading, while there was no peeling of the stacked interface, cube shape yielding had occurred, and restoration of the shape could not be confirmed. These results are considered to be caused by the FDM modeling in which shape forming is performed by stacking walls, lead to buckling deformation and yielding because there is no escape for the force during compression. Note that FIG. 60 is a diagram illustrating a load-displacement curve of a cube in which the filling rate is varied with the filling pattern as a rectilinear shape. FIG. 61 is a diagram illustrating a load-displacement curve of a cube in which the filling rate is fixed at 20% and the filling pattern is changed. FIG. 62 is a diagram illustrating a compression behavior of an FDM modeling sample.

The Architected Material using EPU 40 evaluated in FIG. 57 and FIG. 58 has a configuration that maintains the structure by columns instead of walls, in which due to the structure of the lattice, that is, the folding of the structure as in model A of FIG. 59, it is possible to express a wider variety of physical properties.

### (Comparison with existing insole materials)

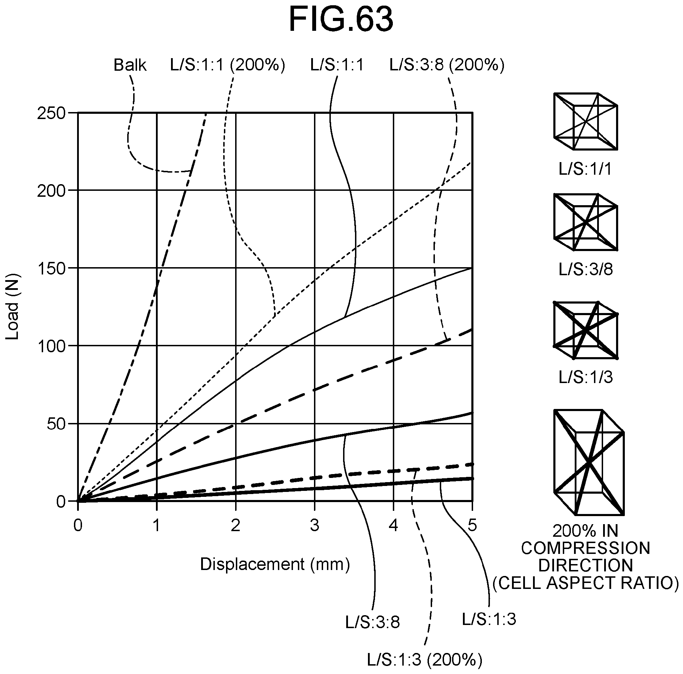

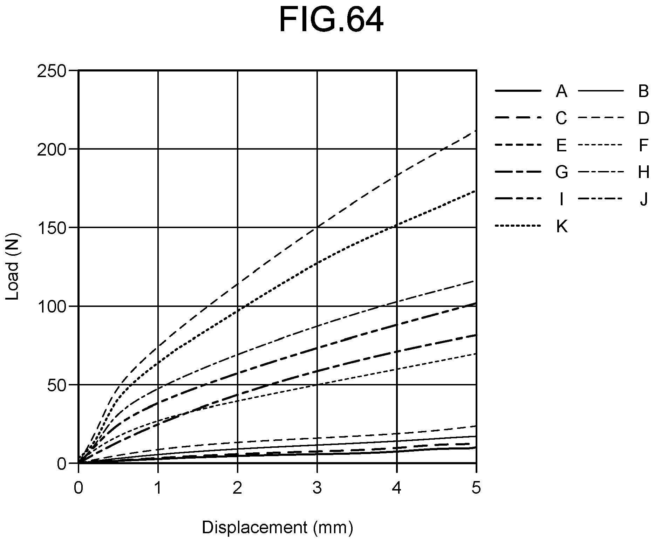

On the other hand, as illustrated in FIG. 63, it was observed that, when the unit cell is fixed (model A) and the column thickness (L/S) and cell dimensional ratio are changed, the slope of the load-displacement curve can be controlled. In addition, it was confirmed that this control range covered a sufficient range as compared with FIG. 64, which evaluated the load-displacement curve when the existing orthosis material was compressed. From these, it was conformed that the Architected Material with lattice structure (unit cell structure) formed with EPU 40 can freely control the buckling and compression behavior and the load-displacement curve compared to the bulk material, and thus effective for controlling not only the overall shape design but also the physical properties. Note that FIG. 63 is a diagram illustrating a load-displacement curve of the lattice cube when the unit cell is fixed and the column thickness (L/S) and the cell dimensional ratio are changed. FIG. 64 is a diagram illustrating a load-displacement curve of an existing medical insole material.

### Reference Signs List

- 1, 2: SYSTEM

- 10, 40, 60: INFORMATION PROCESSING APPARATUS
- 11, 61: CPU
- 12, 62: ROM
- 13, 63: RAM
- 14, 64: AUXILIARY STORAGE DEVICE
- 15, 65: INPUT DEVICE
- 16, 66: DISPLAY DEVICE
- 17, 67: EXTERNAL I/F
- 20, 70: MODELING DEVICE
- 21, 71: MODELING UNIT
- 30, 50: DIAGNOSTIC MEDICAL IMAGING DEVICE
- 100: INSOLE
- 101, 401: STORAGE UNIT
- 102, 402: RECEPTION UNIT
- 103, 403: GENERATION UNIT
- 104, 404: OUTPUT CONTROL UNIT
- 405: ACQUISITION UNIT
- 601: STORAGE UNIT
- 602: USER INTERFACE UNIT
- 603: LEARNING UNIT
- 604: ACQUISITION UNIT
- 605: GENERATION UNIT
- 606: OUTPUT CONTROL UNIT

## Claims

1. An orthosis comprising:
a first region constituted with a first unit cell structure being a unit cell structure having a space of a polygonal prism shape as one unit and having a plurality of structural columns connecting two points out of a plurality of vertices forming the polygonal prism shape; and
a second region constituted with a second unit cell structure different from the first unit cell structure,
wherein the first unit cell structure and the second unit cell structure have at least one structural column connecting a certain vertex among the plurality of vertices to a vertex different from a vertex on a side including the certain vertex.

2. The orthosis according to claim 1,
wherein each of the first unit cell structure and the second unit cell structure
includes an intersection where at least two the structural columns intersect at a different position from the plurality of vertices,
defines a space having an identical shape and an identical size, as one unit, and
has mutually different shape parameters that define physical properties.

3. The orthosis according to claim 2,
wherein the shape parameters include at least one of a shape of the unit cell structure, a length of each side of the unit cell structure, the number of structural columns, a direction of the structural columns, a thickness of the structural columns, or a volume of the intersection.

4. The orthosis according to claim 3,
wherein the intersection has a cross section larger than a cross section of the structural column in a lateral direction.

5. The orthosis according to any one of claims 2 to 4,
wherein the intersection has a spherical shape or a polyhedral shape.

6. The orthosis according to any one of claims 2 to 4,
wherein at least one of the first unit cell structure or the second unit cell structure has the intersection at a center of the space.

7. The orthosis according to claim 6,
wherein at least one of the first unit cell structure or the second unit cell structure has at least two structural columns arranged along a diagonal of the polygonal prism shape and intersecting each other at the intersection.

8. The orthosis according to claim 7,
wherein the polygonal prism shape is a quadrangular prism shape, and
at least one of the first unit cell structure or the second unit cell structure is arranged along a diagonal of the quadrangular prism shape, and has four structural columns intersecting each other at the intersection.

9. The orthosis according to any one of claims 2 to 4,
wherein at least one of the first unit cell structure or the second unit cell structure has the intersection at a center of at least one face out of a plurality of faces constituting the polygonal prism shape.

10. The orthosis according to claim 9,
wherein at least one of the first unit cell structure or the second unit cell structure has at least two structural columns arranged along a diagonal of at least one face out of a bottom face, a top face, or a side face constituting the polygonal prism shape and intersecting each other at the intersection.

11. The orthosis according to any one of claims 1 to 4,
wherein at least one of the first unit cell structure or the second unit cell structure has a plurality of structural columns each arranged along each of a plurality of sides surrounding at least one face out of the bottom face or the top face constituting the polygonal prism shape.

12. The orthosis according to any one of claims 1 to 4,
wherein at least one of the first unit cell structure or the second unit cell structure includes a plurality of structural columns arranged along a direction of load applied to the orthosis.

13. The orthosis according to any one of claims 1 to 4,
wherein each of the first unit cell structure and the second unit cell structure is a unit cell structure in which a space having any one of a triangular prism shape, a quadrangular prism shape, and a hexagonal prism shape is defined as one unit.

14. The orthosis according to any one of claims 1 to 4,
wherein the first region is a structure having a plurality of the first unit cell structures repeatedly and continuously arranged, and
the second region is a structure having a plurality of the second unit cell structures repeatedly and continuously arranged.

15. The orthosis according to any one of claims 1 to 4,
wherein the first region includes at least one layer having a plurality of the first unit cell structures arranged on an identical plane, and
the second region includes at least one layer having a plurality of the second unit cell structures arranged on an identical plane.

16. The orthosis according to any one of claims 1 to 4,
wherein the first region and the second region have different physical properties from each other.

17. The orthosis according to any one of claims 1 to 4,
wherein the first region and the second region are formed of an identical material.

18. The orthosis according to any one of claims 1 to 4, the orthosis being an insole.

19. An information processing apparatus comprising:
a reception unit that receives input related to physical property information represented by a relationship between a change in load and a change in a displacement amount;
a generation unit that
determines shape parameters that define physical properties of an orthosis based on the physical property information received by the reception unit, and
generates model data for designing the orthosis including the determined shape parameters; and
an output control unit that outputs the model data generated by the generation unit.

20. The information processing apparatus according to claim 19,
wherein the generation unit determines, as the shape parameter, a parameter including at least one of the number of a plurality of structural columns constituting a unit cell structure with a polygonal prism shaped space defined as one unit, a direction of the structural columns, a thickness of the structural columns, a volume of an intersection of the structural columns, a shape of the unit cell structure, or size of the unit cell structure.

21. The information processing apparatus according to claim 20,
wherein the generation unit determines the number of structural columns and the direction of the structural columns by determining a unit cell model representing an arrangement pattern of the plurality of structural columns constituting the unit cell structure.

22. The information processing apparatus according to any one of claims 19 to 21,
wherein the reception unit receives input of the physical property information for each of a plurality of regions of the orthosis, and
the generation unit determines the shape parameters that define the physical properties of the orthosis for each of the plurality of regions.

23. The information processing apparatus according to any one of claims 19 to 21,
wherein the output control unit displays a graph of physical property information represented by the relationship between the change in load and the change in the displacement amount, for the model data.

24. The information processing apparatus according to any one of claims 19 to 21, further comprising
an acquisition unit that acquires site information including outer shape data of at least a part of a body of a subject based on image data,
wherein the generation unit generates the model data based on the site information.

25. The information processing apparatus according to claim 24,
wherein the output control unit controls to simultaneously display the model data and the site information,
the reception unit receives an operation of correcting the model data, on an image in which the model data and the site information are simultaneously displayed, and
the generation unit corrects the model data in accordance with the operation received by the reception unit.

26. An information processing apparatus comprising:
an acquisition unit that acquires first site information including outer shape data of a site of a first subject based on image data obtained by imaging the site of the first subject;
a generation unit that generates first model data for designing a first orthosis to be worn on the site of the first subject by inputting the first site information into a trained model that has been trained, for sites of a plurality of second subjects, by using second site information including outer shape data of the site of each of the second subjects, second model data for designing a second orthosis to be worn on the site of each of the second subjects, and evaluation information related to evaluation of the second orthosis; and
an output control unit that outputs the first model data.

27. The information processing apparatus according to claim 26,
wherein the generation unit generates the first model data including shape parameters that define the physical properties of the first orthosis.

28. The information processing apparatus according to claim 27,
wherein the generation unit generates the first model data that includes, as the shape parameter, at least one of the number of a plurality of structural columns constituting a unit cell structure with a polygonal prism shaped space defined as one unit, a direction of the structural columns, a thickness of the structural columns, a volume of an intersection of the structural columns, a shape of the unit cell structure, or size of the unit cell structure.

29. The information processing apparatus according to any one of claims 26 to 28,
wherein the acquisition unit acquires the first site information by using three-dimensional X-ray CT image data in which the site of the first subject is imaged, as the image data.

30. The information processing apparatus according to any one of claims 26 to 28,
wherein the output control unit controls to simultaneously display the first model data and the first site information, and
the generation unit receives an operation of correcting the first model data, on an image in which the first model data and the first site information are simultaneously displayed, and corrects the first model data in accordance with the received operation.

31. The information processing apparatus according to any one of claims 26 to 28, further comprising
a learning unit that generates the trained model by performing machine learning using the second site information, the second model data, and the evaluation information, for the sites of the plurality of second subjects.

32. The information processing apparatus according to claim 31,
wherein the learning unit performs the machine learning by using at least one of wearability evaluation information regarding wearability of the second orthosis obtained by the second subject or symptom evaluation information regarding a symptom of the second subject, as the evaluation information.

33. A system equipped with at least an information processing apparatus and a modeling device,
the system comprising:
an acquisition unit that acquires first site information including outer shape data of a site of a first subject based on image data obtained by imaging the site of the first subject;
a generation unit that generates first model data for designing a first orthosis to be worn on the site of the first subject by inputting the first site information into a trained model that has been trained, for sites of a plurality of second subjects, by using second site information including outer shape data of the site of each of the second subjects, second model data for designing a second orthosis to be worn on the site of each of the second subjects, and evaluation information related to evaluation of the second orthosis; and
a modeling unit that models the first orthosis based on the first model data.

34. An orthosis manufacturing method comprising:
an acquisition step of acquiring first site information including outer shape data of a site of a first subject based on image data obtained by imaging the site of the first subject;
a generation step of generating first model data for designing a first orthosis to be worn on the site of the first subject by inputting the first site information into a trained model that has been trained, for sites of a plurality of second subjects, by using second site information including outer shape data of the site of each of the second subjects, second model data for designing a second orthosis to be worn on the site of each of the second subjects, and evaluation information related to evaluation of the second orthosis; and
a modeling step of modeling the first orthosis based on the first model data.

35. An information processing method comprising:
an acquisition step of acquiring first site information including outer shape data of a site of a first subject based on image data obtained by imaging the site of the first subject;
a generation step of generating first model data for designing a first orthosis to be worn on the site of the first subject by inputting the first site information into a trained model that has been trained, for sites of a plurality of second subjects, by using second site information including outer shape data of the site of each of the second subjects, second model data for designing a second orthosis to be worn on the site of each of the second subjects, and evaluation information related to evaluation of the second orthosis; and
an output control step of outputting the first model data.

36. A program for causing a computer to execute processes comprising:
acquiring first site information including outer shape data of a site of a first subject based on image data obtained by imaging the site of the first subject;
generating first model data for designing a first orthosis to be worn on the site of the first subject by inputting the first site information into a trained model that has been trained, for sites of a plurality of second subjects, by using second site information including outer shape data of the site of each of the second subjects, second model data for designing a second orthosis to be worn on the site of each of the second subjects, and evaluation information related to evaluation of the second orthosis; and
outputting the first model data.

37. An information processing apparatus comprising:
a learning unit that generates a trained model, for sites of a plurality of subjects, by performing machine learning using site information including outer shape data of the site of each of the subjects, model data for designing an orthosis to be worn on the site of each of the subjects, and evaluation information regarding evaluation of the orthosis; and
an output control unit that outputs the trained model.

38. An information processing method comprising:
a learning step of generating a trained model, for sites of a plurality of subjects, by performing machine learning using site information including outer shape data of the site of each of the subjects, model data for designing an orthosis to be worn on the site of each of the subjects, and evaluation information regarding evaluation of the orthosis; and
an output control step of outputting the trained model.

39. A program for causing a computer to execute processes comprising:
generating a trained model, for sites of a plurality of subjects, by performing machine learning using site information including outer shape data of the site of each of the subjects, model data for designing an orthosis to be worn on the site of each of the subjects, and evaluation information regarding evaluation of the orthosis; and
outputting the trained model.
